(19) Europäisches Patentamt
European Patent Office
Office européen des brevets

(11) EP 1 967 517 A1

(12) EUROPEAN PATENT APPLICATION
published in accordance with Art. 158(3) EPC

(43) Date of publication:
**10.09.2008 Bulletin 2008/37**

(21) Application number: **06835174.1**

(22) Date of filing: **25.12.2006**

(51) Int Cl.:
***C07D 277/84*** *(2006.01)* ***A61K 31/4245*** *(2006.01)*
***A61K 31/4439*** *(2006.01)* ***A61K 31/5377*** *(2006.01)*
***A61K 51/00*** *(2006.01)* ***A61P 25/14*** *(2006.01)*
***A61P 25/16*** *(2006.01)* ***A61P 25/28*** *(2006.01)*
***C07D 401/06*** *(2006.01)* ***C07D 405/06*** *(2006.01)*
***C07D 413/04*** *(2006.01)* ***C07D 417/06*** *(2006.01)*

(86) International application number:
**PCT/JP2006/325804**

(87) International publication number:
**WO 2007/074786 (05.07.2007 Gazette 2007/27)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **26.12.2005 JP 2005371821**

(71) Applicant: **TOHOKU UNIVERSITY**
**Aoba-ku**
**Sendai-shi, Miyagi 980-8577 (JP)**

(72) Inventors:
- **KUDO, Yukitsuka**
**Miyagi 980-8577 (JP)**
- **ARAI, Hiroyuki**
**Miyagi 980-8577 (JP)**
- **OKAMURA, Nobuyuki**
**Miyagi 980-8577 (JP)**
- **MARUYAMA, Masahiro**
**Miyagi 980-8577 (JP)**
- **FURUMOTO, Syozo**
**Miyagi 980-8577 (JP)**
- **DOH-URA, Katsumi**
**Miyagi 980-8577 (JP)**

(74) Representative: **Zwicker, Jörk et al**
**Dr. Volker Vossius**
**Patent- und Rechtsanwaltskanzlei**
**Geibelstrasse 6**
**81679 München (DE)**

(54) **PROBE FOR DIAGNOSIS OF CONFORMATIONAL DISEASE**

(57) The invention provides a probe compound useful for early diagnosis of conformation disease, a composition and a kit comprising it for diagnosis for conformation disease, and a medical composition for treatment and/or prevention of conformation disease.

EP 1 967 517 A1

## Description

TECHNICAL FIELD

The present invention relates to a diagnostic probe for conformation disease, in particular to an imaging diagnostic probe, and in detail, to a probe labeled with a positron emitter, and to a composition for imaging diagnosis that comprises the probe. Further, the invention relates to a medical composition, for example, for detection/staining of amyloid β protein and neurofibrillary tangle in a brain material, and for example, for detection/staining of senile plaque in the brain of Alzheimer disease patients, and for prevention and/or treatment of conformation disease. The invention also relates to a composition for diagnosis of conformation disease that comprises the above-mentioned probe compound.

BACKGROUND ART

Disorders with deposition of a β sheet structured protein that is intrinsic to conformation disease include various diseases characterized by deposition of insoluble fibrillary protein in various organs and tissues of a body. These diseases include Alzheimer disease, prion disease, Lewy body disease, Parkinson disease, Huntington disease, spinobulbar muscular atrophy, dentatorubral-pallidoluysian atrophy, spinocerebellar ataxia, Machado-Joseph disease, amyophic lateral sclerosis (ALS), Down syndrome, Pick disease, FTDP-17 (frontotemporal dementia and parkinsonism linked to chromosome 17), LNTD (limbic neurofibrillary tangle dementia), sudanophilic leukodystrophy, amyloidosis, etc.

Of those, Alzheimer disease (AD) is at present considered as one of most incurable diseases, and accurate early diagnosis is desired for it. Alzheimer disease is a disease characterized by progressive dementia occurring essentially in the presenile stage to the senile stage. From the pathological viewpoint, the disease is characterized by entire cerebral involution, extreme denaturation and omission of neurons and appearance of neurofibrillary tangle and senile plaque. It is known that the most significant risk factor of dementia such as typically Alzheimer disease is aging. Accordingly, the increase in the number of the case patients with the increase in the senile population is remarkable especially in Japan, America and European countries that are in aging society, and the medical cost for the disease has brought about a crisis of the medical system in these countries.

In our country, the number of Alzheimer disease patients is estimated at about 1,000,000, and with the increase in the senile population in future, the number of the patients will surely increase. The cost for one Alzheimer disease patient inclusive of care expense will be from 1,000,000 yen to 3,000,000 yen/year, and therefore, our country would have already paid a social economic cost of from 1,000,000,000,000 yen to 3,000,000,000,000 yen. Medical treatment of Alzheimer disease before the actual development of the symptom of the disease or in the stage thereof as early as possible could bring about a great medical economic effect, and it is now a global common sense.

At present, various methods are known for diagnosis of Alzheimer disease. In our country, generally employed is a method of quantitatively detecting the reduction in the cognitive function of an individual that may have suffered from Alzheimer disease, such as a Hasegawa method, ADAS, MMSE or the like; but rarely and secondarily employed is an imaging diagnostic method (e.g., MRI, CT). However, these diagnostic methods are unsatisfactory for deciding the disease, and the definite diagnosis requires biopsy of the brain during the lifetime and histopathologic examination of the brain after death. Despite of energetic studies made for it, no one could make any significant progress in diagnosis of Alzheimer disease. As a result of many studies, it has become known that neurodegeneration characteristic of Alzheimer disease may begin much before the development of the first clinical symptom of the disease (in a long case, it is before about 40 years). In addition, it is known that, when the family or the clinicians around the patient of the disease have noticed the first clinical symptom of the disease, then the intracerebral pathologic feature of the patient has already advanced to an irreparable state. In consideration of the progressive characteristic of the disease symptom and of the significant increase in the number of the disease patients, the necessity and the meaning of accurate early stage diagnosis of Alzheimer disease is extremely great.

The histopathologic feature of Alzheimer disease is characterized by two typical cardinal signs. They are senile plaque and neurofibrillary tangle. The essential constitutive component of the former is a β sheet structured amyloid β (Aβ) protein; and that of the latter is a hyperphosphorylated tau protein. The definite diagnosis of Alzheimer disease is based on the expression of these pathologic characteristics in a patient's brain.

Amyloid β protein is characteristic of conformation disease that includes Alzheimer disease, and the two have close relation to each other. Accordingly, detection of a β sheet structured amyloid β protein as a marker in a body, especially in a brain is one important method for diagnosis of conformation disease, especially Alzheimer disease. Searches for substances capable of specifically binding to intracorporeal, especially intracerebral amyloid β protein to stain it have heretofore been made for the purpose of diagnosis of a disease with amyloid deposition such as typically Alzheimer disease. As such substances, known are Congo red (see Non-Patent Reference 1), Thioflavin S (see Non-Patent Reference 2), Thioflavin T (Non-Patent Reference 3) and Crysamine G and its derivatives (see Patent Reference 1 and Patent Reference 2). However, these have a lot of problems in point of their binding specificity to amyloid β protein,

blood-brain barrier permeability, solubility and toxicity. We, the present inventors have found out various compounds characterized by high specificity to amyloid β protein, great blood-brain barrier permeability and solubility and less toxicity (see Patent Reference 3, Patent Reference 4, Patent Reference 5, Patent Reference 6 and Patent Reference 7).

A disease is known, which is caused by an intracerebral protein itself having a β sheet structure. It is considered that, in Alzheimer disease, amyloid β protein and tau protein may have a β sheet structure and the proteins themselves may be a cause of the disease or a part of the cause of the disease. Yankner, et al. reported for the first time that, when amyloid β protein is made to have a β sheet structure, then it exhibits neuron toxicity (see Non-Patent Reference 4). After that, many replication studies for it have been made, and have confirmed that the β sheet structured amyloid β protein has neuron toxicity. In that manner, the β sheet structured amyloid β protein and tau protein have neuron toxicity, and therefore, it may be suggested that a compound capable of inhibiting the cytotoxicity could be a remedial drug for a disease, of which the cause or a part of the cause is the β sheet structured protein itself, or that is, conformation disease such as Alzheimer disease. At present, however, the development of such a remedial drug could not bring about a sufficient result.

Accordingly, the necessity is increasing for a compound having high specificity to amyloid β protein for diagnosis of conformation disease such as typically Alzheimer disease, for a staining agent specific to amyloid β protein, and for treatment and prevention of conformation disease.

Another histopathologic cardinal sign of Alzheimer disease comprises neurofibrillary tangle and its essential constitutive component, hyperphosphorylated tau protein, but in general, it is considered that these may be expressed later than amyloid β protein. However, it is considered that neurofibrillary tangle may well correlate to the degree of dementia as compared with amyloid β protein (see Non-Patent Reference 5 and Non-Patent Reference 6).

Apart from Alzheimer disease, disorders characterized by the cardinal sign of intracerebral deposition tau protein (tauopathy) are Pick disease and progressive supranuclear palsy (PSP). Conformation disease also includes these diseases.

To that effect, tau protein is characteristic of the disease with deposition of tau protein that includes Alzheimer disease, and it has close relation to the disease. Accordingly, the detection of intracorporeal, especially intracerebral β sheet structured tau protein as a marker is one important method for diagnosis of diseases with tau deposition, especially Alzheimer disease.

A method for quantitatively determining the tau level in a body, especially in a cerebrospinal fluid for the purpose of diagnosis of tau deposition-associated diseases such as typically Alzheimer disease has been reported by a few groups (see Non-Patent Reference 7 and Non-Patent Reference 8). However, any probe for in-vivo noninvasive quantitative determination of tau is not known at all in the world.

Accordingly, a necessity is increasing for a compound having high specificity to neurofibrillary tangle, for diagnosis and treatment of a disease of which the cause or a part of the cause is neurofibrillary tangle such as typically Alzheimer disease or for staining neurofibrillary tangle.

Heretofore, compounds have been reported which have high specificity to amyloid β protein and neurofibrillary tangle (see Patent Reference 8, Patent Reference 9, Patent Reference 10, Patent Reference 11). When these compounds are used in-vivo, especially in human patients' bodies, it is desirable that the compounds have little or no mutagenicity. Accordingly, a search is necessary for compounds having little or no mutagenicity and capable of being used as a probe for diagnosis of conformation disease.

Patent Reference 1: PCT/US96/05918
Patent Reference 2: PCT/US98/07889
Patent Reference 3: Japanese Patent Application 2000-080082
Patent Reference 4: Japanese Patent Application 2000-080083
Patent Reference 5: Japanese Patent Application 2001-076075
Patent Reference 6: PCT/JP01/02204
Patent Reference 7: PCT/JP01/02205
Patent Reference 8: PCT/JP03/07183
Patent Reference 9: PCT/JP03/15269
Patent Reference 10: PCT/JP03/15229
Patent Reference 11: PCT/JP2004/01546
Non-Patent Reference 1: Puchtler et al., Journal of Histochemistry and Cytochemistry, Vol. 10, p. 35, 1962 Non-Patent Reference 2: Puchtler et al., Journal of Histochemistry and Cytochemistry, Vol. 77, p. 431, 1983 Non-Patent Reference 3: Le Vine, Protein Science, Vol. 2, pp. 404-410, 1993
Non-Patent Reference 4: Yankner et al., Science, Vol. 245, pp. 417-420, 1989
Non-Patent Reference 5: Braak H. and Braak E., Acta Neuropathol., Vol. 82, pp. 239-259, 1991
Non-Patent Reference 6: Wischik et al., Neurobiology of Alzheimer's Disease, pp. 103-206, Oxford University Press, Oxford, 2001

Non-Patent Reference 7: Ishiguro et al., Neurosci. Lett., Vol. 270, pp. 81-84, 1999
Non-Patent Reference 8: Itoh et al., Ann. Neurol., Vol. 50, pp. 150-156, 2001

DISCLOSURE OF THE INVENTION

PROBLEMS THAT THE INVENTION IS TO SOLVE

In consideration of the above-mentioned situation, the present invention provides a substance capable of being used as a diagnostic probe for conformation disease, which has high specificity to amyloid β protein and/or neurofibrillary tangle, has high brain permeability and has little or no mutagenicity. The invention also provides a labeled such substance capable of being used as an imaging diagnostic probe for conformation disease, and a composition and a kit for imaging diagnosis comprising the probe. The invention further provides a method for detection and/or staining of amyloid β protein and neurofibrillary tangle in a brain material, a kit for it, and a medical composition for prevention and/or treatment of conformation disease. The invention also provides a compound useful for early stage diagnosis of conformation disease, and a composition for imaging diagnosis comprising it.

MEANS FOR SOLVING THE PROBLEMS

We, the present inventors have assiduously studied for the purpose of solving the above-mentioned problems, and, as a result, have found that compounds of formula (I) to formula (VI), or salts or solvates thereof in the present description are usable for a diagnostic probe for conformation disease, as having high specificity to amyloid P protein and/or neurofibrillary tangle, having high brain permeability and having little or no mutagenicity, and have completed the present invention. In particular, the compounds of the invention having a morpholine ring at the terminal have little mutagenicity or have no mutagenicity as shown in Examples. One characteristic feature of the invention is that the invention includes a group of such compounds having little or no mutagenicity. Accordingly, the compounds of the invention are extremely highly safe. Since the compounds of the invention stain amyloid β protein specifically and sharply, they may enable accurate early stage diagnosis of especially Alzheimer disease and Down syndrome. Further, the compounds of the invention have high brain permeability, or that is, high blood-brain barrier permeability. Having these characteristics, using the compound of the invention enables in-vivo noninvasive early stage diagnosis especially in human patients.

EFFECT OF THE INVENTION

The invention provides compounds having high specificity to amyloid β protein and/or neurofibrillary tangle, having high blood-brain barrier permeability, having little or no mutagenicity and having extremely high safety. Accordingly, using the compound of the invention enables diagnosis, treatment and/or prevention of conformation disease. In addition, the invention enables imaging diagnosis of conformation disease, especially imaging diagnosis with PET. Accordingly, the invention enables accurate early stage diagnosis, and effective treatment and prevention of conformation disease.

BRIEF DESCRIPTION OF THE DRAWINGS

[Fig. 1] Fig. 1 is a fluorescence microscopy image (ex-vivo) in intravenous administration of THK-097 (0.2 mg/kg) to a Tg mouse (Tg2576) with amyloid β protein deposition.
[Fig. 2] Fig. 2 is a fluorescence microscopy image (ex-vivo) in intravenous administration of THK-525 (4 mg/kg) to a Tg mouse (Tg2576) with amyloid β protein deposition. The white space arrow indicates amyloid β protein.
[Fig. 3] Fig. 3 is a fluorescence microscopy image (ex-vivo) in intravenous administration of THK-727 (4 mg/kg) to a Tg mouse (APPswe2576/Tau JPL3) with amyloid β protein deposition. The white space arrow indicates amyloid β protein.
[Fig. 4] Fig. 4 is a fluorescence microscopy image in intravenous administration of THK-702 (4 mg/kg) to a Tg mouse (Tg2576) with amyloid β protein deposition (upper panel); and an anti-amyloid (Aβ) antibody-stained image of the same section (lower panel).
[Fig. 5] Fig. 5 shows enlarged microscope images of Fig. 4. A, B and C correspond to A, B and C of Fig. 4, respectively. The white and black arrows indicate amyloid β protein.
[Fig. 6] Fig. 6 is a THK-097 (left panel)-stained image and an anti-amyloid β (Aβ) antibody-stained image (right panel: adjacent section to that of left panel) in a brain section of an Alzheimer disease patient. The white space

arrow indicates amyloid β protein.

[Fig. 7] Fig. 7 is a THK-184-stained image in a brain section of an Alzheimer disease patient. The white space arrow indicates amyloid β protein.

[Fig. 8] Fig. 8 is a THK-185-stained image in a brain section of an Alzheimer disease patient. The white space arrow indicates amyloid β protein; and the white space arrow head indicates neurofibrillary tangle.

[Fig. 9] Fig. 9 is a THK-203-stained image in a brain section of an Alzheimer disease patient. The white space arrow indicates amyloid P protein.

[Fig. 10] Fig. 10 is a THK-207-stained image in a brain section of an Alzheimer disease patient. The white space arrow indicates amyloid β protein.

[Fig. 11] Fig. 11 is a THK-248-stained image in a brain section of an Alzheimer disease patient. The white space arrow indicates amyloid β protein.

[Fig. 12] Fig. 12 is a THK-254-stained image in a brain section of an Alzheimer disease patient. The white space arrow head indicates neurofibrillary tangle.

[Fig. 13] Fig. 13 is a THK-258-stained image in a brain section of an Alzheimer disease patient. The white space arrow indicates amyloid β protein; and the white space arrow head indicates neurofibrillary tangle.

[Fig. 14] Fig. 14 is a THK-262-stained image in a brain section of an Alzheimer disease patient. The white space arrow indicates amyloid β protein.

[Fig. 15] Fig. 15 is a THK-276-stained image in a brain section of an Alzheimer disease patient. The white space arrow indicates amyloid β protein.

[Fig. 16] Fig. 16 is a THK-281-stained image in a brain section of an Alzheimer disease patient. The white space arrow indicates amyloid β protein.

[Fig. 17] Fig. 17 is a THK-308-stained image in a brain section of an Alzheimer disease patient. The white space arrow indicates amyloid β protein.

[Fig. 18] Fig. 18 is a THK-317-stained image in a brain section of an Alzheimer disease patient. The white space arrow indicates amyloid β protein; and the white space arrow head indicates neurofibrillary tangle.

[Fig. 19] Fig. 19 is a THK-383-stained image in a brain section of an Alzheimer disease patient. The white space arrow indicates amyloid β protein.

[Fig. 20] Fig. 20 is a THK-385-stained image in a brain section of an Alzheimer disease patient. The white space arrow indicates amyloid β protein.

[Fig. 21] Fig. 21 is a THK-386-stained image in a brain section of an Alzheimer disease patient. The white space arrow indicates amyloid β protein; and the white space arrow head indicates neurofibrillary tangle.

[Fig. 22] Fig. 22 is a THK-525 (left panel)-stained image and an anti-amyloid β (Aβ) antibody-stained image (right panel: adjacent section to that of left panel) in a brain section of an Alzheimer disease patient. The white space arrow indicates amyloid β protein.

[Fig. 23] Fig. 23 is a THK-556-stained image in a brain section of an Alzheimer disease patient. The white space arrow indicates amyloid β protein.

[Fig. 24] Fig. 24 is a THK-558-stained image in a brain section of an Alzheimer disease patient. The white space arrow indicates amyloid β protein.

[Fig. 25] Fig. 25 is a THK-559-stained image in a brain section of an Alzheimer disease patient.. The white space arrow indicates amyloid P protein.

[Fig. 26] Fig. 26 is a THK-561-stained image in a brain section of an Alzheimer disease patient. The white space arrow indicates amyloid P protein.

[Fig. 27] Fig. 27 is a THK-562-stained image in a brain section of an Alzheimer disease patient. The white space arrow indicates amyloid β protein.

[Fig. 28] Fig. 28 is a THK-563-stained image in a brain section of an Alzheimer disease patient. The white space arrow indicates amyloid β protein.

[Fig. 29] Fig. 29 is a THK-565 (left panel)-stained image and an anti-amyloid β (Aβ) antibody-stained image (right panel: adjacent section to that of left panel) in a brain section of an Alzheimer disease patient. The white space arrow indicates amyloid β protein.

[Fig. 30] Fig. 30 is a THK-585-stained image in a brain section of an Alzheimer disease patient. The white space arrow indicates amyloid β protein.

[Fig. 31] Fig. 31 is a THK-702-stained image in a brain section of an Alzheimer disease patient. The white space arrow indicates amyloid β protein.

[Fig. 32]. Fig. 32 is a THK-708-stained image in a brain section of an Alzheimer disease patient. The white space arrow indicates amyloid β protein.

[Fig. 33] Fig. 33 is a THK-727-stained image in a brain section of an Alzheimer disease patient. The white space arrow indicates amyloid P protein; and the white space arrow head indicates neurofibrillary tangle.

[Fig. 34] Fig. 34 is a THK-752-stained image in a brain section of an Alzheimer disease patient. The white space

arrow indicates amyloid β protein. [Fig. 35] Fig. 35 is a THK-761-stained image in a brain section of an Alzheimer disease patient. The white space arrow indicates amyloid P protein.

[Fig. 36] Fig. 36 is a THK-763-stained image in a brain section of an Alzheimer disease patient. The white space arrow indicates amyloid β protein.

[Fig. 37] Fig. 37 is a THK-766-stained image in a brain section of an Alzheimer disease patient. The white space arrow indicates amyloid β protein.

BEST MODE FOR CARRYING OUT THE INVENTION

The compounds of the invention are compounds of formulae (I) to (VI) described hereinunder, salts or solvates thereof. Unless otherwise specifically indicated, "the compounds of the invention" as referred to in this description include compounds of formulae (I) to (VI) and salts and solvates thereof. The compounds of the invention can be obtained according to methods known by those skilled in the art.

In this description, for example, "$C_{1-4}$ alkyl" means an alkyl group having from 1 to 4 carbon atoms. The number of carbon atoms of the other alkyl group is expressed in the same manner, and its meaning shall be interpreted according to the above-mentioned case. For example, "$C_{1-4}$ alkyl" or "alkyl having from 1 to 4 carbon atoms" includes methyl, ethyl, propyl, butyl and their structural isomers. In this description, "halogen" includes fluorine, chlorine, bromine and iodine. In this description, "amyloid beta protein", "amyloid β protein", "Aβ protein", "amyloid beta", "amyloid β" and "Aβ" all have the same meaning.

When the compound of the invention has a double bond between two rings therein, then, the compound include both cis and trans isomers.

The first embodiment of the compounds of the invention is a compound of a formula (I):

[Formula 1]

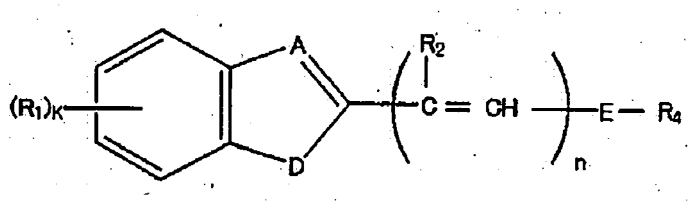

[wherein A represents CH or N;
D represents S, NH, N-$C_{1-3}$ alkyl, O or $CH_2$;
$R_1$ each independently represents hydrogen, halogen, OH, COOH, $SO_3H$, $NO_2$, SH, $NR^aR^b$, $C_{1-6}$ alkyl, O-$C_{1-6}$ alkyl, CN, C=O, O-$(CH_2)_1$-OTs, or O-$(CH_2)_m$-$CHR^cR^d$, or

[Formula 2]

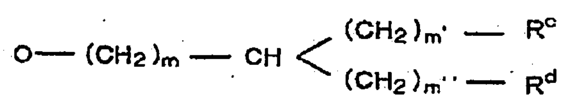
;

adjacent $R_1$'s, taken together, may form a phenyl ring;
$R_2$ represents hydrogen, $C_{1-4}$ alkyl, $C_{1-4}$ alkyl-halogen, OH or CN;
$R_4$ represents hydrogen, halogen, OH, COOH, $SO_3H$, $NO_2$, SH, $NR^5R^6$, $C_{1-6}$ alkyl, O-$C_{1-6}$ alkyl, CN, C=O, pyrrolidine ring, pyrrole, pyrazole, imidazole, triazole, or

[Formula 3]

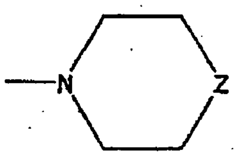,
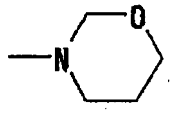,
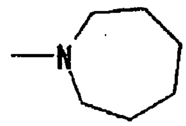
or
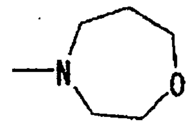

$R^a$ and $R^b$ each independently represent hydrogen or $C_{1-4}$ alkyl;
$R^c$ and $R^d$ each independently represent hydrogen, halogen, OH, COOH, $SO_3H$, $NO_2$, SH, $NR^aR^b$, $C_{1-6}$ alkyl, O-$C_{1-6}$ alkyl, CN, C=O or OTs;.
$R^5$ and $R^6$ each independently represent hydrogen or $C_{1-4}$ alkyl;
k indicates an integer of from 1 to 4;
1 indicates an integer of from 1 to 4;
m indicates an integer of from 0 to 3;
m' indicates an integer of from 0 to 3;
m" indicates an integer of from 0 to 3;
n indicates an integer of from 1 to 4;
E represents a benzene ring or

[Formula 4]

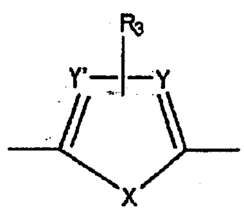
;

X represents CH, S, NH, $NC_{1-3}$ alkyl or O;
Y represents CH or N;
Y' represents CH or N;
Z represents O, S, $CH_2$ or N-$R^e$;
$R_3$ is present when both or one of Y and Y' is CH, representing hydrogen, $C_{1-4}$ alkyl, OH or halogen;
$R^e$ represents hydrogen or $C_{1-4}$ alkyl;
the above alkyl may be substituted with halogen;
the configuration around the double bond that bonds two ring parts may be any of cis-form or trans-form], or a salt or a solvate thereof.

The second embodiment is a compound of a formula (II) :

[Formula 5]

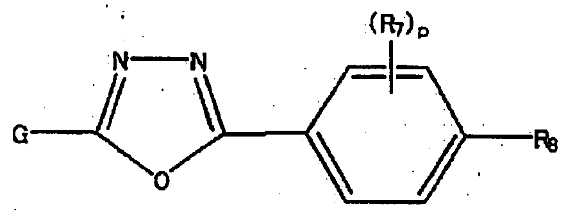

[wherein G represents furan, thiophen, pyrrole, pyridine, benzofuran, benzothiophen, benzoxazole, benzothiazole, benzimidazole or indol ring;
the ring may be substituted with halogen, OH, COOH, $SO_3H$, $NO_2$, SH, $NR^fR^g$, $C_{1-6}$ alkyl, O-$C_{1-6}$ alkyl, CN or C=O;
$R_7$ represents hydrogen, halogen, OH, COOH, $SO_3H$, $NO_2$, SH, $NR^fR^g$, $C_{1-6}$ alkyl, O-$C_{1-6}$ alkyl,- CN or C=O;
$R_8$ represents hydrogen, halogen, OH, COOH, $SO_3H$, $NO_2$, SH, $NR^fR^g$, $C_{1-6}$ alkyl, O-$C_{1-6}$ alkyl, CN, C=O, pyrrolidine ring, or

[formula 6]

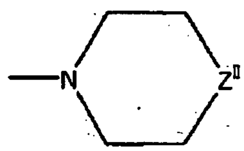

;

$R^f$ and $R^g$ each independently represent hydrogen or $C_{1-4}$ alkyl;
p indicates an integer of from 1 to 4;
$Z^{II}$ represents O, $CH_2$, N-$R^{e'}$ ;
$R^{e'}$ represents hydrogen or $C_{1-4}$ alkyl;
the alkyl may be substituted with halogen], or a salt or a solvate thereof.

The third embodiment is a compound of a formula (III) :

[Formula 7]

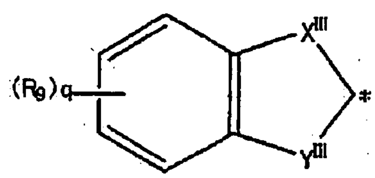

[wherein $X^{III}$ and $Y^{III}$ each independently represent $CH_2$ or C=O;
$R^9$ represents hydrogen, halogen, OH, COOH, $SO_3H$, $NO_2$, SH, $NR^hR^i$, $C_{1-6}$ alkyl, O-$C_{1-6}$ alkyl, CN or C=O;
$R^h$ and $R^i$ each independently represent hydrogen or $C_{1-4}$ alkyl;
at *, the following part bonds to the formula:

[Formula 8]

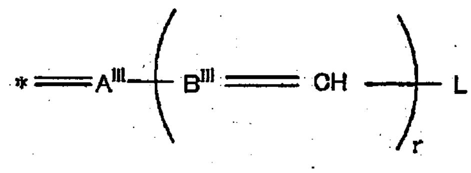

[Formula 9]

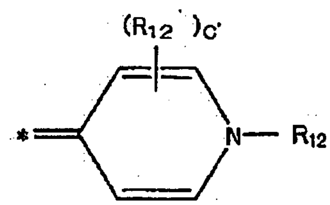

or

[Formula 10]

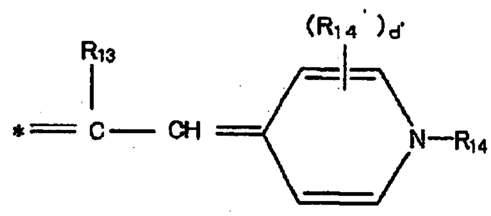

;

L represents

[Formula 11]

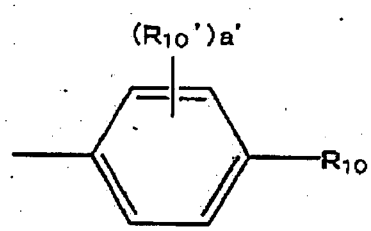

or

[Formula 12]

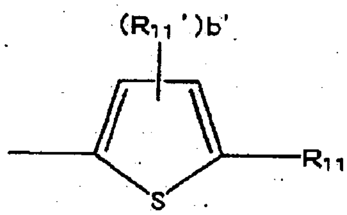

;

$A^{III}$ and $B^{III}$ each independently represent CH or N;
$R_{10}$, $R_{11}$. $R_{12}$ and $R_{14}$ each independently represent hydrogen, halogen, OH, COOH, $S0_3H$, $NO_2$, SH, $NR^IR^{II}$, $C_{1-6}$ alkyl, O-$C_{1-6}$ alkyl, CN, C=O, pyrrolidine ring, or

[Formula 13]

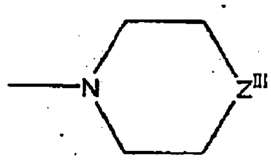

;

$R_{13}$ represents hydrogen, halogen or $C_{1-4}$ alkyl;
$R_{10}$' , $R_{11}$', $R_{12}$' and $R_{14}$' each independently represent hydrogen, halogen or $C_{1-4}$ alkyl;
r indicates an integer of from 0 to 2;
a' indicates an integer of from 1 to 4;
b' indicates an integer of 1 or 2;
c' indicates an integer of from 1 to 4;
d' indicates an integer of from 1 to 4;
$Z^{III}$ represents O, $CH_2$ or N-$R^{e"}$ ;
$R^{e"}$ represents hydrogen or $C_{1-4}$ alkyl;
the alkyl may be substituted with halogen;
the configuration around the double bond that bonds two ring parts may be, if possible, any of cis-form or trans-form], or a salt or a solvate thereof.

The fourth embodiment is a compound of a formula (IV):

[Formula 14]

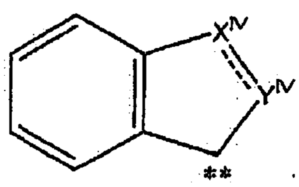

[wherein $X^{IV}$ represents N or $NR^{IV}$;
$NR^{IV}$ represents hydrogen or $C_{1-4}$ alkyl;
$Y^{IV}$ represents CH or C=O;
the dotted line means an optionally-existing single bond; at **, the following part bonds to the formula:

[Formula 15]

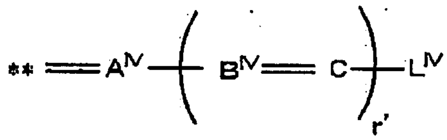

[Formula 16]

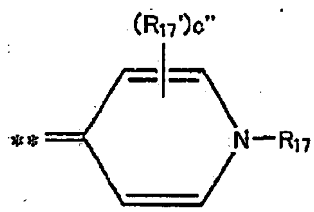

or

[Formula 17]

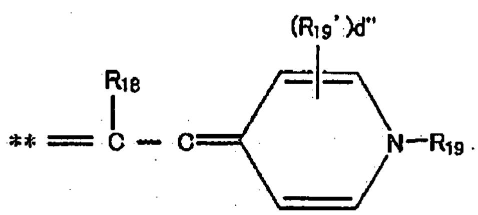

;

$L^{IV}$ represents

[Formula 18]

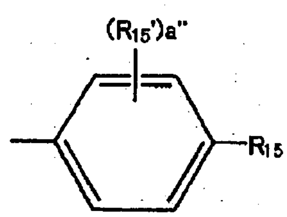

or

[Formula 19]

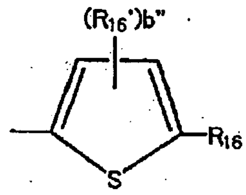

;

$A^{IV}$ and $B^{IV}$ each independently represent CH, $CCH_3$ or N;
$R_{15}$, $R_{16}$, $R_{17}$ and $R_{19}$ each independently represent hydrogen, halogen, OH, COOH, $SO_3H$, $NO_2$ SH, $NR^jR^k$, $C_{1-6}$ alkyl, O-$C_{1-6}$ alkyl, CN, C=O, pyrrolidine ring or

[Formula 20]

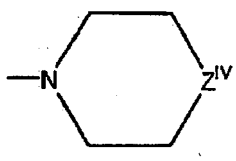

;

$R^J$ and $R^k$ each independently represent hydrogen or $C_{1-4}$ alkyl;
$R_{18}$ represents hydrogen, halogen or $C_{1-4}$ alkyl;
$R_{15}$' , $R_{16}$', $R_{17}$' and $R_{19}$' each independently represent hydrogen, halogen or $C_{1-4}$ alkyl;
r' indicates an integer of from 0 to 2;
a" indicates an integer of from 1 to 4;
b" indicates an integer of 1 or 2;
c" indicates an integer of from 1 to 4;
d" indicates an integer of from 1 to 4;
$Z^{IV}$ represents O, $CH_2$ or N-$R^{e'''}$;
$R^{e'''}$ represents hydrogen or $C_{1-4}$ alkyl;
the alkyl may be substituted with halogen;
the configuration around the double bond that bonds two ring parts may be, if possible, any of cis-form or trans-' form], or a salt or a solvate thereof.

The fifth embodiment is a compound of a formula (V) :

[Formula 21]

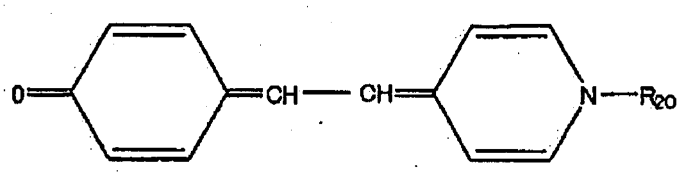

[wherein $R_{20}$ represents hydrogen, $C_{1-4}$ alkyl, pyrrolidine ring or

[Formula 22]

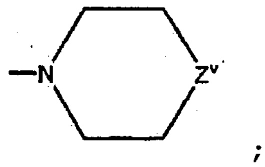

$Z^V$ represents O, $CH_2$ or N-$R^{eV}$;
$R^{eV}$ represents hydrogen or $C_{1-4}$ alkyl], or a salt or a solvate thereof.

The sixth embodiment is a compound of a formula (VI):

[Formula 23]

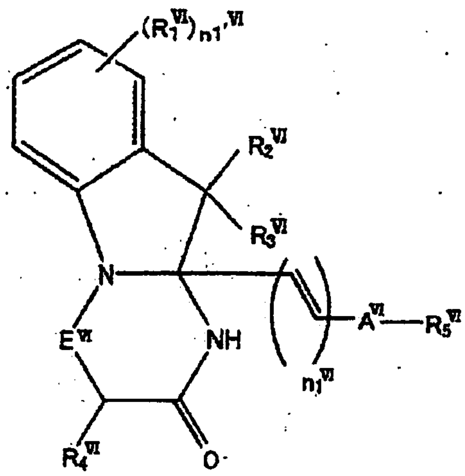

[wherein $R_1^{VI}$ represents hydrogen, $C_{1-6}$ alkyl, halogen or $C_{1-6}$ alkyl-halogen;
$R_2^{VI}$ and $R_3^{VI}$ each independently represent hydrogen or $C_{1-6}$ alkyl;
$R_4^{VI}$ represents hydrogen or $C_{1-6}$ alkyl;
$E^{VI}$ represents $CH_2$, or is absent;
$A^{VI}$ represents a 5-membered or 6-membered ring, having the following structure:

[Formula 24]

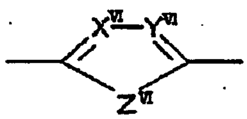

or

[Formula 25]

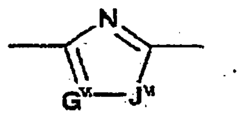

or

[Formula 26]

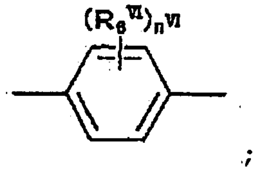

$X^{VI}$ and $Y^{VI}$ each independently represent N or CH;
$Z^{VI}$ represents O, S, $CH_2$ or N-$C_pH_{2p+1}$;
$G^{VI}$ represents N or CH;
$J^{VI}$ represents O, S, $CH_2$ or N-$C_qH_{2q+1}$;
$R_5^{VI}$ represents hydrogen, $C_{1-6}$ alkyl, pyrrole, pyrazole, imidazole, triazole or $NR_I^{VI}R_{II}^{VI}$;
$R_6^{VI}$ represents hydrogen, $C_{1-6}$ alkyl or halogen;
$R_I^{VI}$ and $R_{II}^{VI}$ each independently represent hydrogen or $C_{1-6}$ alkyl, or taken together, they form a pyrrolidine ring, a morpholine ring, a piperidine ring, or a piperazine ring in which the hydrogen atom may be substituted with $C_{1-3}$ alkyl;
$n_1{}^{VI}$ indicates an integer of from 1 to 4;
$n_{1'}{}^{VI}$ indicates an integer of from 1 to 4;
$n^{VI}$ indicates an integer of from 1 to 4;
$p^{VI}$ indicates an integer of from 1 to 4;
$q^{VI}$ indicates an integer of from 1 to 4;
in the above formula, the configuration around the double bond that bonds two ring parts is a trans-form, but this configuration may be any of cis-form or trans-form], or a salt or a solvate thereof.

Examples of the compounds of the invention, or salts or solvates thereof are shown in the following Table 1.

[Table 1-1]

| | Structure | |
|---|---|---|
| THK-097 | 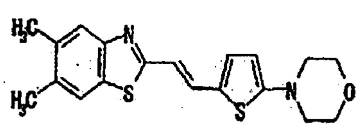 | 5,6-dimethyl-2-[2-(5-morpholin-4-yl-thiophen-2-yl) vinyl]-benzothiazole |
| THK-101 | 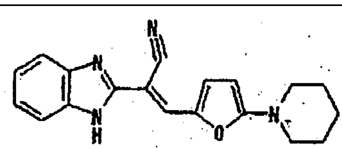 | 2-(1H-benzimidazol-2-yl)-3-(5-piperidin-1-yl-furan-2-yl)-acrylonitrile |
| THK-203 | 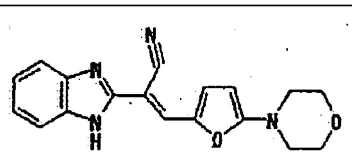 | 2-(1H-benzimidazol-2-yl)-3-(5-morpholin-4-yl-furan-2-yl)-acrylonitrile |

(continued)

| | Structure | |
|---|---|---|
| THK-207 | 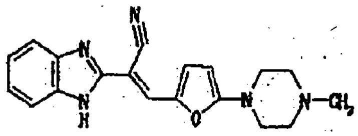 | 2-(1H-benzimidazol-2-yl)-3-[5-(4-methyl-piperazin-1-yl)-furan-2-yl]-acrylonitrile |
| THK-525 | 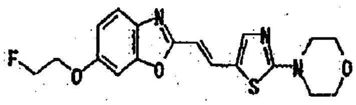 | 6-(2-fluoro-ethoxy)-2-[2-(2-morpholin-4-yl-thiazol-5-yl)-vinyl]-benzoxazole |
| THK-575 | 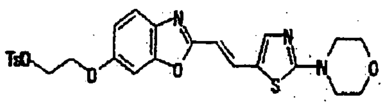 | Toluene-4-sulfonic acid 2-[2-[2-(2-morpholin-4-yl-thiazol-5-yl)-vinyl-benzoxazol-6-yloxy]-ethyl toluene-4-sulfonate |
| THK-683 | 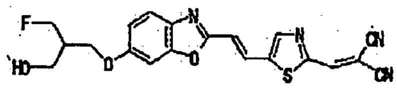 • 0.2$H_2O$ | (E) -2-[2[(2,2-dicyanoethenylthiazol-5-yl)-ethenyl]-6-[(2-fluoromethyl-3-hydroxy)-propoxy]benzoxazole |

[Table 1-2]

| | Structure | |
|---|---|---|
| THK-702 | 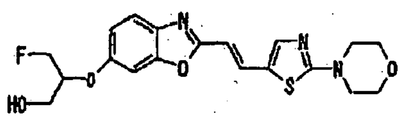 | (E) -2- [2- (2-morpholinothiazol-5-yl)ethenyl]-6-[(1-fluoromethyl-2-hydroxy)ethoxy]-benzoxazole |
| THK-703 | 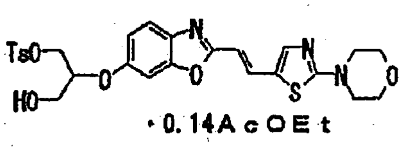 • 0.14AcOEt | (E)-6-[(2-hydroxy-1-tosyloxymethyl)-ethoxy]-2-[2-(2-morpholinothiazol-5-yl)-ethenyl]-benzoxazole |
| THK-707 | 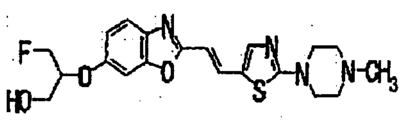 | (E)-6-[(1-fluoromethyl-2-hydroxy)ethoxy]-2-[2-[2-(4-methylpiperazin-1-yl)thiazol-5-yl]ethenyl]benzoxazole |
| THK-708 | 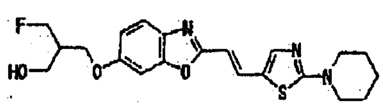 | (E)-6-[(2-fluoromethyl-3-hydroxy)propoxy]-2-[2-[2-(pyrrolidin-1-yl)thiazol-5-yl]ethenyl]-benzoxazole |
| THK-710 | 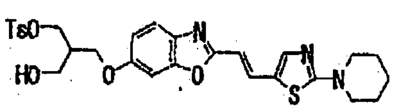 • 0.19AcOEt | (E)-6-[(3-hydroxy-2-tosyloxymethyl)propoxy]-2-[2-(2-piperidinothiazol-5-yl)ethenyl]-benzoxazole |

(continued)

| | Structure | |
|---|---|---|
| THK-711 | 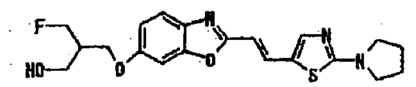 | (E)-6-[(2-fluoromethyl-3-hydroxy) propoxy] -2- [2- (2-piperidinothiazol-5-yl)ethenyl]-benzoxazole |
| THK-712 | 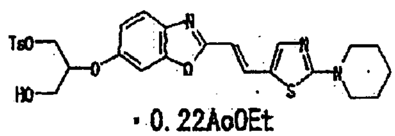 · 0.22AcOEt | (E)-6-[(2-hydroxy-1-tosyloxymethyl)ethoxy]-2-[2-(2-piperidinothiazol-5-yl)ethenyl]-benzoxazole |
| THK-713 | 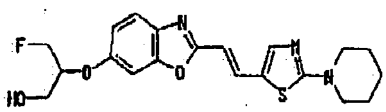 | (E)-6-[(1-fluoromethyl-2-hydroxy)ethoxy]-2-[2-(2-piperidinoxazol-5-yl)ethenyl]-benzoxazole |

[Table 1-3]

| | Structure | |
|---|---|---|
| THK-726 | 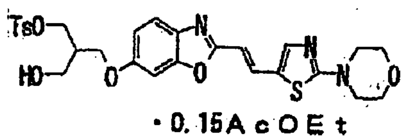 · 0.15AcOEt | (E)-6-[(3-hydroxy-2-tosyloxymethyl)propoxy]-2-[2-(2-morpholinothiazol-5-yl)ethenyl]-benzoxazole |
| THK-727 | 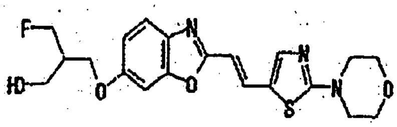 | 2-fluoromethyl-3-[2-[2-(2-morpholin-4-yl-thiazol-5-yl)-vinyl]-benzoxazol-6-yloxy]-propan-1-ol |
| THK-751 | 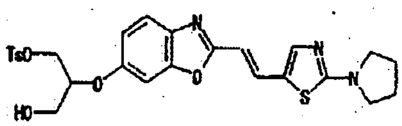 | (E)-6-[(1-hydroxymethyl-2-tosyloxy)ethoxy]-2-[2-[2-(pyrrolidin-1-yl)thiazol-5-yl]ethenyl]benzoxazole |
| THK-752 | 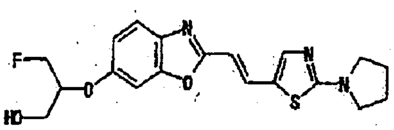 | (E)-6-[(1-fluoromethyl-2-hydroxy) ethoxy] -2-[2-[2-(pyrrolidin-1-yl)-thiazol-5-yl] ethenyl] benzoxazole |
| THK-757 | 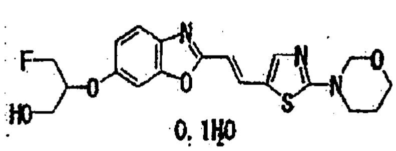 $0.1H_2O$ | (E)-6-[(1-fluoromethyl-2-hydroxy) ethoxy]-2-[2-[2-([1,3] oxadinan-3-yl)-thiazol-5-yl]ethenyl]benzoxazole |

(continued)

| | Structure | |
|---|---|---|
| THK-760 | 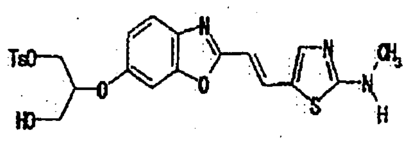 | (E)-6-[(2-hydroxy-1-tosyloxymethyl)ethoxy]-2-[2-[2-methylaminothiazol-5-yl]ethenyl]-benzoxazole |
| THK-761 | 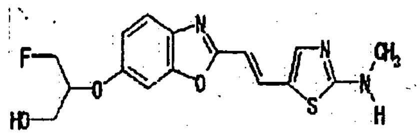 | (E)-6-[(1-fluoromethyl-2-hydroxy)ethoxy]-2-[2-[2-methylaminothiazol-5-yl]ethenyl]-benzoxazole |

[Table 1-4]

| | Structure | |
|---|---|---|
| THK-762 | 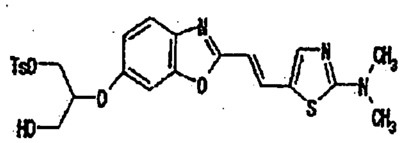 | (E)-6-[(2-hydroxy-1-tolyloxymethyl)ethoxy]-2-[2-[2-dimethylamino-thiazol-5-yl]ethenyl]benzoxazole |
| THK-763 | 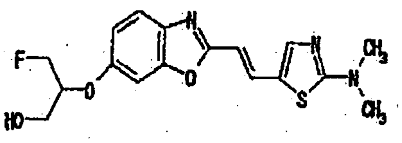 | (E)-6-[(1-fluoromethyl-2-hydroxy)ethoxy]-2-[2-[2-dimethylamino-thiazol-5-yl]ethenyl]benzoxazole |
| THK-765 | 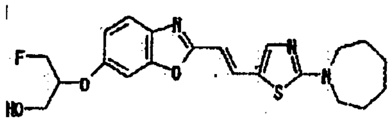 | (E)-6-[(1-fluoromethyl-2-hydroxy) ethoxy] -2-[2-[2-(homopiperidin-1-yl)thiazol-5-yl]ethenyl] benzoxazole |
| THK-766 | 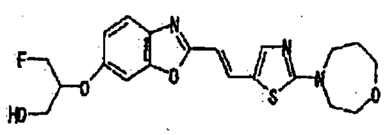 | (E)-6-[(1-fluoromethyl-2-hydroxy) ethoxy] -2- [2- (2-homomorpholinothiazol-5-yl)ethenyl]benzoxazole |
| THK-767 | 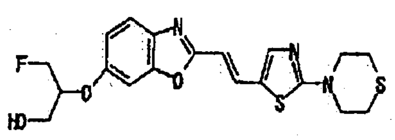 | (E)-6-[(1-fluoromethyl-2-hydroxy) ethoxy] -2- [2- (2-thiomorpholinothiazol-5-yl)ethenyl]benzoxazole |
| THK-775 | 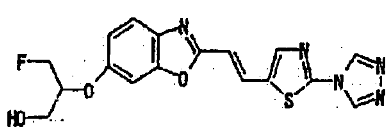 | (E)-6-[(1-fluoromethyl-2-hydroxy) ethoxy] -2- [2 [2-(1,2,4-triazol-4-yl)-thiazol-5-yl) ethenyl] benzoxazole |
| THK-281 | 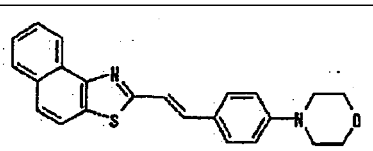 | 2-[2-(4-morpholin-4-yl-phenyl)-vinyl]naphthol[1,2-d] thiazole |

[Table 1-5]

| | Structure | |
|---|---|---|
| THK-255 | 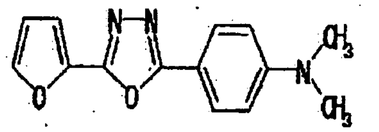 | [4-(5-furan-2-yl-[1,3,4]oxadiazol-2-yl)-phenyl]-dimethyl-amine |
| THK-256 | 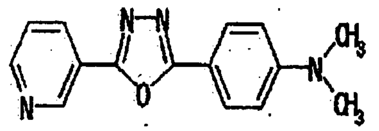 | dimethyl-[4-(5-pyridin-3-yl-[1,3,4]oxadiazol-2-yl)-phenyl]-amine |
| THK-257 | 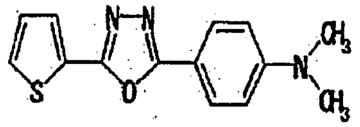 | dimethyl-[4-(5-thiophen-2-yl-[1,3,4]oxadiazol-2-yl)-phenyl]-amine |
| THK-258 | 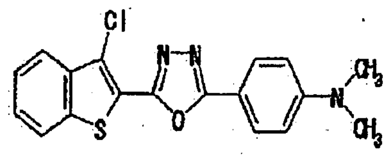 | [4-[5-(3-chloro-benzo[b]thiophen-2-yl)-[1,3,4]oxadiazol-2-yl]-phenyl]-dimethylamine |
| THK-262 | 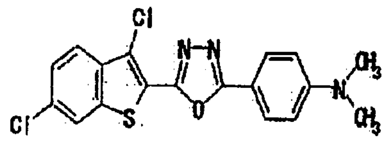 | [4-[5-(3,6-dichloro-benzo[b]thiophen-2-yl)-[1,3,4] oxadiazol-2-yl]-phenyl]-dimethylamine |
| THK-383 | 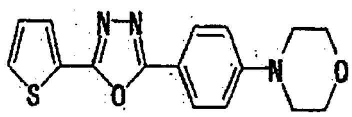 | 4-[4-(5-thiophen-2-yl-[1,3,4]oxadiazol-2-yl)-phenyl]-morpholine |
| THK-384 | 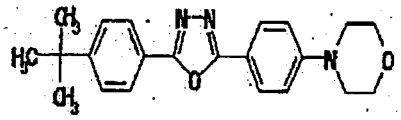 | 4-[4-[5-(4-tert-butyl-phenyl)-[1,3,4]oxadiazol-2-yl]-phenyl]-morpholine |
| THK-385 | 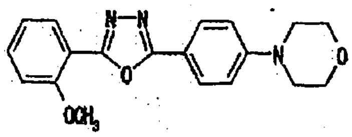 | 4-[4-(5-o-tolyl-[1,3,4]oxazin-2-yl)-phenyl]-morpholine |
| THK-386 | 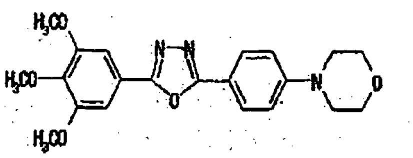 | 4-{4-[5-(3,4,5-trimethyl-phenyl)-[1,3,4]oxadiazol-2-yl]-phenyl}morpholine |
| THK-387 | 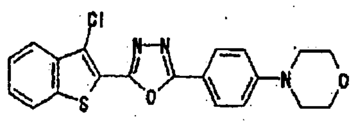 | 4-[4-[5-(3-chloro-benzo[b]thiophen-2-yl)-[1,3,4]oxadiazol-2-yl]-phenyl]-morpholine |

[Table 1-6]

| | Structure | |
|---|---|---|
| THK-156 | 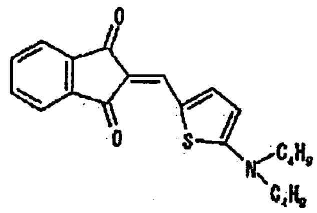 | 2-(5-dibutylamino-thiophen-2-ylmethylene)-indane-1,3-dione |
| THK-184 | 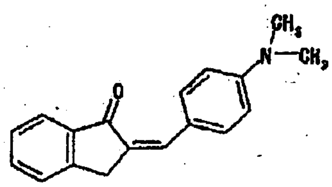 | 2-(4-dibutylamino-benzylidene)-indan-1-one |
| THK-248 | | 2-[3-(4-dimethylamino-phenyl)-allylidene]-indane-1,3-dione |
| THK-253 | 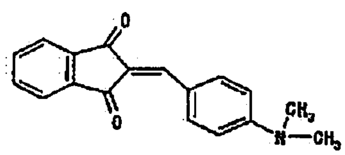 | 2-(4-dimethylamino-benzylidene)-indane-1,3-dione |
| THK-287 | 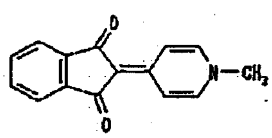 | 2-(1-methyl-1H-pyridin-4-ylidene)-indane-1,3-dione |
| THK-532 | 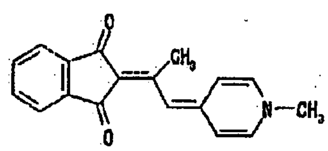 | 2-[1-methyl-2-(1-methyl-1H-pyridin-4-ylidene)-ethylidene]-indane-1,3-dione |

[Table 1-7]

| | Structure | |
|---|---|---|
| THK-185 | 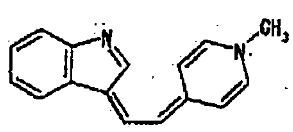 | 3-[2-(1-methyl-1H-pyridin-4-ylidene)-ethylidene]-3H-indole |
| THK-186 | 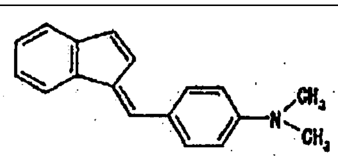 | (4-indene-1-ylidenemethyl-phenyl)-dimethyl-amine |

(continued)

| | Structure | |
|---|---|---|
| THK-209 | 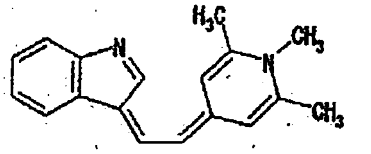 | 3-[2-(1,2,6-trimethyl-1H-pyridin-4-ylidene)-ethylidene]-3H-indole |
| THK-254 | 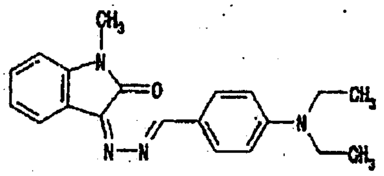 | 3-[(4-dimethylamino-benzylidene)-hydrazono]-1-methyl-1,3-dihydroindol-2-one |
| THK-276 | 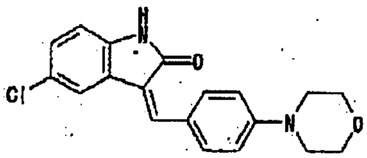 | 5-chloro-3-(4-morpholin-4-yl-benzylidene)-1,3-dihydroindol-2-one |
| THK-277 | 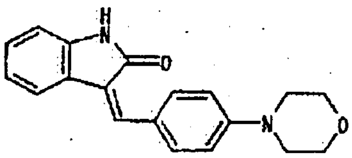 | 3-(4-morpholin-4-yl-benzylidene)-1,3-dihydroindol-2-one |
| THK-308 | 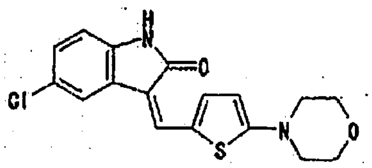 | 5-chloro-3-(5-morpholin-4-yl-thiophen-2-ylmethylene)-1,3-dihydroindol-2-one |

[Table 1-8]

| | Structure | |
|---|---|---|
| THK-317 | 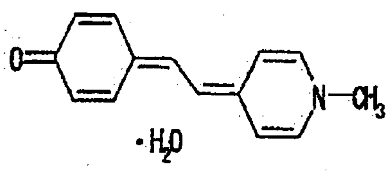 | 4-[2-(1-methyl-1H-pyridin-4-ylidene)-ethylidene]-cyclohexa-2,5-dienone |

[Table 1-9]

| | Structure | |
|---|---|---|
| THK-330 | 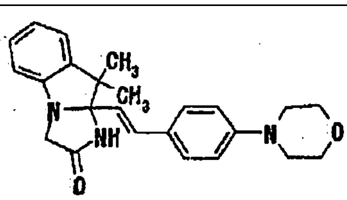 | 9,9-dimethyl-9a-[2-(4-morpholin-4-yl-phenyl)-vinyl-9,9a-dihydro-1H-imidazol[1,2-a]indol-2-one |

(continued)

| | Structure | |
|---|---|---|
| THK-336 | 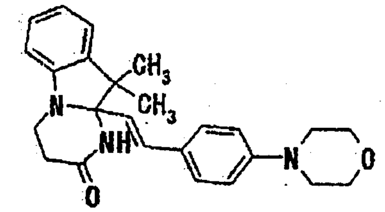 | 10,10-dimethyl-10a-2-(4-morpholin.4-yl-phenyl)-vinyl]-3,4,10,10a-tetrahydro-1H-pyrimidol[1,2-a]indol-2-one |
| THK-533 | 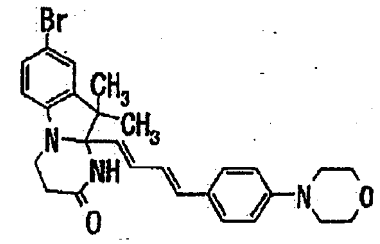 | 8-bromo-10,10-dimethyl-10a-[4-(4-morpholin-4-yl-phenyl)-buta-1,3-dienyl]-3,4,10,10a-tetrahydro-1H-pyrimido [1,2-a]indol-2-one |
| THK-556 | 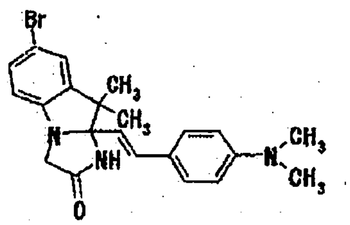 | 7-bromo-9a-[2-(4-dimethylamino-phenyl)-vinyl]-9,9-dimethyl-9,9a-dihydro-1H-imidazo[1,2- a]indol-2-one |
| THK-558 | 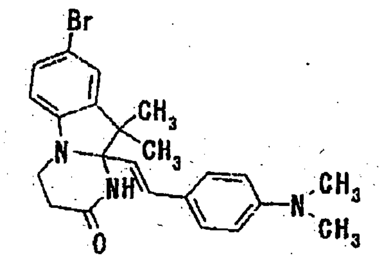 | 8-bromo-10a-[2-(4-dimethylamino-phenyl)-vinyl]-10,10-dimethyl 1-3,4,10,10a-tetrahydro-1H-pyrimido[1,2-a]indol-2-one |

[Table 1-10]

| | Structure | |
|---|---|---|
| THK-559 | 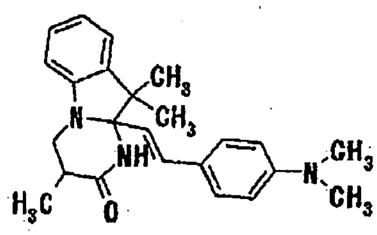 | 10a-[2-(4-dimethylamino-phenyl)-vinyl]-3,10,10-trimethyl-3,4,10,10a-tetrahydro-1H-pyrimido[1,2-a]indol-2-one |
| THK-560 | 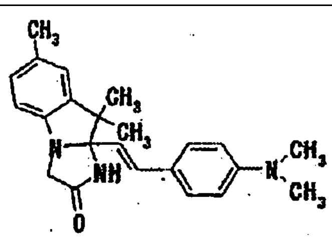 | 9a-[2-(4-dimethylamino-phenyl)-vinyl]-7,9,9-trimethyl-9,9a-dihydro-1H-imidazo[1,2-a]indol-2-one |

(continued)

| | Structure | |
|---|---|---|
| THK-561 | 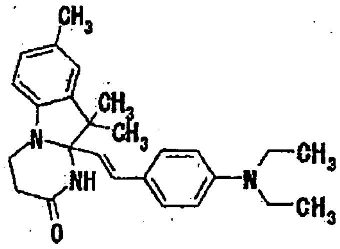 | 10a-[2-(4-dimethylamino-phenyl)-vinyl]-8,10,10-trimethyl-3,4,10,10a-tetrahydro-1H-pyrimido[1,2-a]indol-2-one |
| THK-562 | 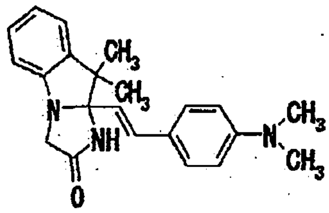 | 9a-2-(4-dimethylamino-phenyl)-vinyl]-9,9-dimethyl-9,9a-dihydro-1H-imidazo[1,2-a]indol-2-one |
| THK-563 | 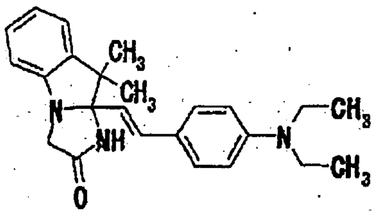 | 9a-[2-(4-dimethylamino-phenyl)-vinyl]-9,9-dimethyl-9,9a-dihydro-1H-imidazo[1,2-a]indol-1-2-one |

[Table 1-11]

| | Structure | |
|---|---|---|
| THK-564 | 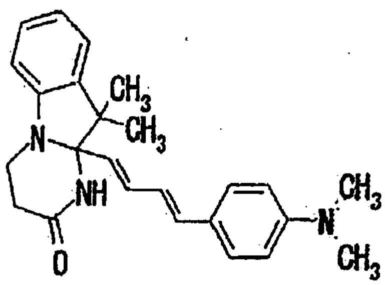 | 10a-[4-(4-dimethylamino-phenyl)-buta-1,3-dienyl]-10,10-dimethyl 1-3,4,10,10a-tetrahydro-1H-pyrimido[1,2-a] indol-2-one |
| THK-565 | 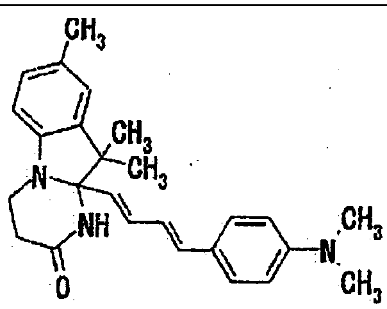 | 10a-[4-(4-dimethylamino-phenyl)-buta-1,3-dienyl]-8,10,10-trimethyl-3,4,10,10a-tetrahydro-1H-pyrimido[1,2-a]indol-2-one |

(continued)

| | Structure | |
|---|---|---|
| THK-573 | 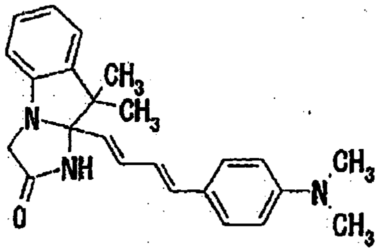 | 9a-[4-(4-dimethylamino-phenyl)-buta-1,3-dienyl]-9,9-dimethyl 1-9,9a-dihydro-1H-imidazo[1,2-a]indol-2-one |
| THK-579 | 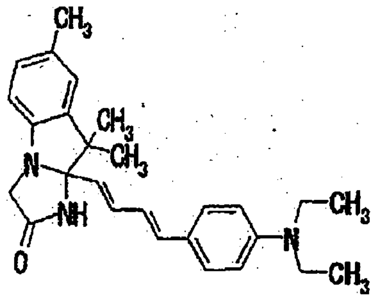 | 9a-[4-(4-dimethylamino-phenyl)-buta-1,3-dienyl]-7,9,9-trimethyl-9,9a-dihydro-1H-imidazo[1,2-a]indol-2-one |
| THK-585 | 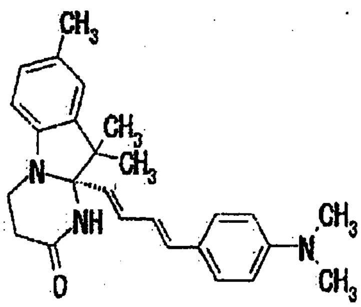 | 10a-[4-(4-dimethylamino-phenyl)-buta-1,3-dienyl]-8,10,10-trimethyl-3,4,10,10a-tetrahydro-1H-pyrimido[1,2-a]indol-2-one |

[Table 1-12]

| | Structure | |
|---|---|---|
| THK-611 | 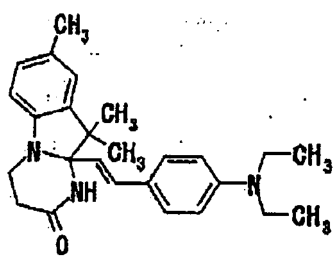 | 10a-[2-(4-diethylamino-phenyl)-vinyl]-8,10,10-trimethyl-3,4,10,10a-tetrahydro-1H-pyrimido[1,2-a]indol-2-one |
| THK-613 | 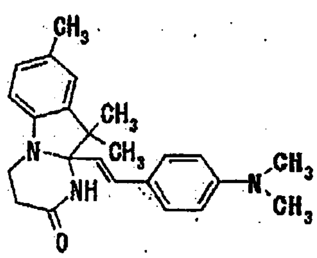 | 10a-[2-(4-dimethylamino-phenyl)-vinyl]-8,10,10-trimethyl-3,4,10,10a-tetrahydro-1H-pyrimido[1,2-a]indol-2-one |

(continued)

| | Structure | |
|---|---|---|
| THK-614 | 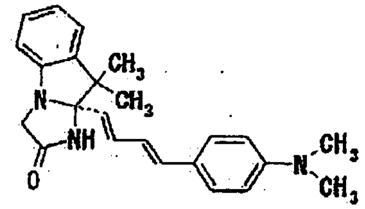 | 9a-[4-(4-dimethylamino-phenyl)-buta-1,3-dienyl]-9,9-dimethyl-9,9a-dihydro-1H-imidazo[1,2-a]indol-2-one |
| THK-651 | 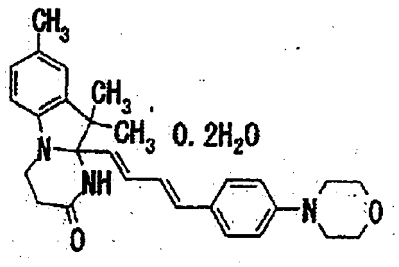 | 10a-[4-[4-(morpholino)phenyl]-1,3-butadienyl]-3,4,10,10a-tetrahydro-8,10,10-, trimethylpyrimido[1,2-a]indol-2(1H)-one |
| THK-652 | 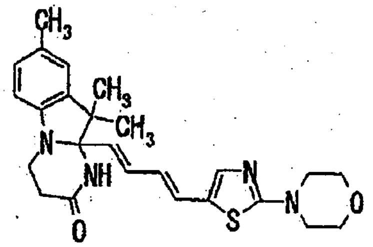 | 10a-[4-[4-(morpholino)thiazol-5-yl]-1,3-butadienyl]-3,4,10,10a-tetrahydro-8,10,10-trimethylpyrimido[1,2-a]indol-2(1H)-one |

[Table 1-13]

| | Structure | |
|---|---|---|
| THK-653 | 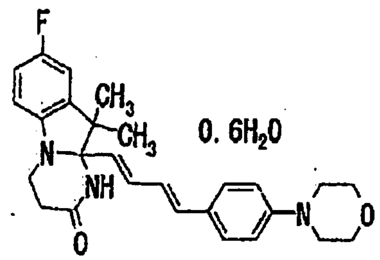 | 8-fluoro-10,10-dimethyl-10a-[4-[4-(2-morpholino)phenyl]-1,3-butadienyl]-3,4,10,10a-tetrahydro-pyrimido[1,2-a]indol-2(1H)-one |
| THK-655 | 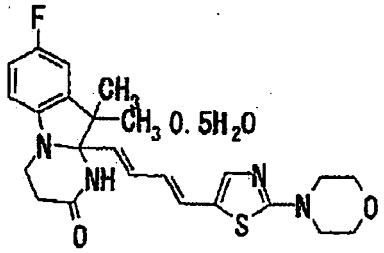 | 8-fluoro-10,10-dimethyl-10a-[4-[4-(2-morpholino)thiazol-5-yl]-1,3-butadienyl]-3,4,10,10a-tetrahydro-pyrimido [1,2-a] indol-2 (1H) -one |

(continued)

| | Structure | |
|---|---|---|
| THK-700 | 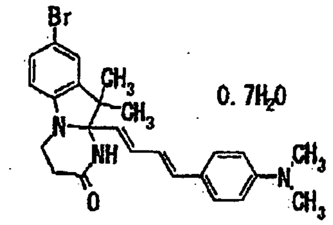 | 8-bromo-10,10-dimethyl-10a-[4-[4-(dimethylamino)phenyl]-1,3-butadienyl]-3,4,10,10a-tetrahydro-pyrimido[1,2-a]indol-2(1H)-one |
| THK-701 | 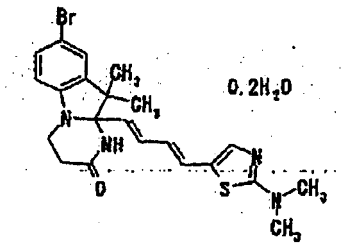 | 8-bromo-10,10-dimethyl-10a-[4-[2-(dimethylamino)thiazol-5-yl]-1,3-butadienyl] -3,4,10,10a-tetrahydro-pyrimido[1,2-a] indol-2(1H)-one |
| THK-705 | 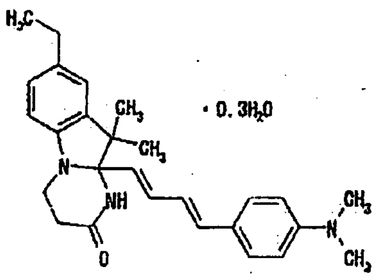 | 8-ethyl-10,10-dimethyl-10a-[4-[4-(dimethylamino)phenyl]-1,3-butadienyl]-3,4,10,10a-tetrahydro-pyrimido[1,2-a]indol-2(1H)-one |

[Table 1-14]

| | Structure | |
|---|---|---|
| THK-715 | 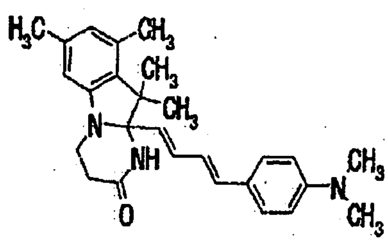 | 7,9,10,10-tetramethyl-10a-[4-[4-(dimethylamino)phenyl]-1,3-butadienyl]-3,4,10,10a-tetrahydro-pyrimido[1,2-a] indol-2(1H)-one |
| THK-716 | 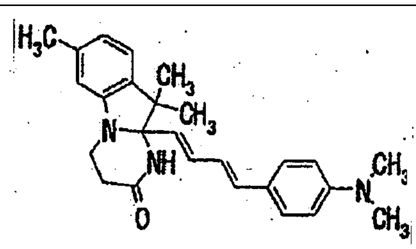 | 7-methyl-10,10-dimethyl-10a-[4-[4-(dimethylamino) phenyl]-1,3-butadienyl]-3,4,10,10a-tetrahydro-pyrimido [1,2-a]indol-2(1H)-one |

(continued)

| | Structure | |
|---|---|---|
| THK-717 | 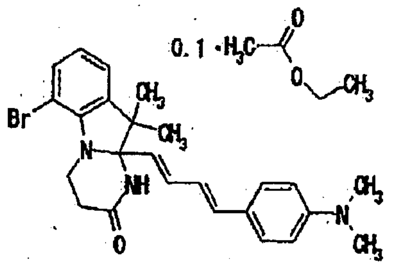 | 6-bromo-10,10-dimethyl-10a-[4-[4-(dimethylamino) phenyl]-1,3-butadienyl]-3,4,10,10a-tetrahydro-pyrimido [1,2-a]indol-2(1H)-one |

Of the compounds of the invention, or salts or solvates thereof, preferred are those of formula (I), or salts or solvates thereof. Of the compounds of the invention, salts or solvates thereof, more preferred are those having a morpholine ring at their terminal. The compounds having a morpholine ring at their terminal have little or no mutagenicity, as shown in Examples. Further, these compounds, salts or solvates thereof have high specificity to Aβ, have low toxicity and have high brain permeability, also as in Examples. Accordingly, the compounds of the invention can be used a safe probe for imaging diagnosis of conformation disease.

Accordingly, the invention provides the following:

(1) A compound of formula (I), or a salt or a solvate thereof;
(2) The compound of (1), or the salt or the solvate thereof, which is selected from a group consisting of THK-097, THK-203, THK-207, THK-281, THK-525, THK-702, THK-708, THK-727, THK-752, THK-761, .THK-763 and THK-766;
(3) The compound of (1), or the salt or the solvate thereof, wherein $R^4$ is a morpholine ring;
(4) The compound of (3), the salt-or the solvate thereof, which is selected from a group consisting of THK-097, THK-203, THK-525, THK-575, THK-702, THK-703, THK-726 and THK-727;
(5) The compound of (1), or the salt or the solvate thereof, which is selected from a group consisting of THK-683, THK-707, THK-708, THK-711, THK-713, THK-752, THK-761, THK-763;
(6) The compound of any of (1) to (5), or the salt or the solvate thereof, which is labeled;
(7) The compound of (6), or the salt or the solvate thereof, which is radioactive-labeled;
(8) The compound of (7), or the salt or the solvate thereof, which is labeled with a positron emitter;
(9) A diagnostic composition for conformation disease, which comprises a compound any of (1) to (8), or the salt or the solvate thereof;
(10) A medical composition for treatment and/or prevention of conformation disease, which comprises a compound of any of (1) to (8), or the salt or the solvate thereof;
(11) A diagnostic kit for conformation disease, which comprises, as the indispensable constitutive component thereof, a compound of any of (1) to (8), or the salt or the solvate thereof;
(12) A composition or kit for detecting or staining a β sheet structured protein or neurofibrillary tangle, which comprises, as the indispensable constitutive component thereof, a compound of any of (1) to (8), or the salt or the solvate thereof;
(13) The composition of (9) or the kit of (11) or (12), which is for imaging diagnosis;
(14) A method for treatment and/or prevention of conformation disease of a subject, which comprises administering a compound of any of (1) to (8), or the salt or the solvate thereof to the subject;
(15) A method for diagnosis of conformation disease of a subject, which comprises administering a compound of any of (1) to (8), or the salt or the solvate thereof to the subject;
(16) Use of the compound of any of (1) to (8), or the salt or the solvate thereof, for producing a composition or kit for diagnosis of conformation disease of a subject;
(17) Use of the compound of any of (1) to (8), or the salt or the solvate thereof, for producing a medical composition for treatment and/or prevention of conformation disease of a subject;
(18) A method for detecting or staining a β sheet structured protein or neurofibrillary tangle in a sample, which comprises staining the sample with a compound of any of (1) to (8), or the salt or the solvate thereof;
(19) Use of the compound of any of (1) to (8), or the salt or the solvate thereof, for producing a composition or kit for detecting or staining a β sheet structured protein or neurofibrillary tangle in a sample;
(20) The composition or kit of (12), the method of (18) or the use of (19), wherein the compound is THK-727.

The compounds of the invention are described in more detail. Preferred substituents in the compounds of formula (I) are mentioned below.

Preferably, D is O, S or NH.
Preferably, $R_1$ is hydrogen, methyl, ethyl, $C_{1-6}$ alkyl, O-$C_{1-6}$ alkyl, O-$(CH_2)_1$-$OT_S$, or O-$(CH_2)_m$-$CH_2R^cR^d$, the alkyl group may be substituted with halogen or OH; and also preferably, taken together, adjacent two $R_1$'s form a benzene ring.
Preferably, $R_2$ is hydrogen or CN.
Preferably, $R_4$ is a pyrrolidine ring, a morpholine ring, a piperidine ring, or a piperazine ring in which the nitrogen atom may be substituted with $C_{1-3}$ alkyl,
Preferably, $R^a$ and $R^b$ each are hydrogen or methyl.
Preferably, $R^c$ and $R^d$ each are hydrogen, methyl, halogen, OH or OTs.
Preferably, $R_5$ and $R_6$ each are hydrogen or methyl.
When $R_1$ is not hydrogen, k is preferably 1 or 2.
Preferably, 1 is 1 or 2.
Preferably m is from 0 to 2.
Preferably, n is 1.
Preferably, X is S or O.
Preferably, Y is CH or N.
Preferably, Y' is CH or N.
Preferably, Z is O, CH, or N-$R^e$.
Preferably, $R_3$ is hydrogen.
Preferably $R^e$ is hydrogen or methyl.
Also preferably, E is a benzene ring.
The alkyl may be substituted with fluorine, chlorine or bromine.
The configuration around the double bond that bonds two ring parts may be any of cis-form or trans-form.

Of the compounds of formula (I), those where $R_4$ is a morpholine ring are more preferred, as they have little or no mutagenicity. The compounds of formula (I) wherein $R_4$ is a morpholine ring include THK-097, THK-203, THK-525, THK-575, THK-702, THK-703, THK-726 and THK-727. Of the compounds of formula (I), THK-683, THK-707, THK-708, THK-711, THK-713, THK-752, THK-761 and THK-763 are also more preferred, as they have little or no mutagenicity. Of the compounds of the invention, more preferred, as having little or no mutagenicity, are those of which the relative activity in the absence of S9mix is minus and the relative activity in the presence of S9mix is minus or on an at most $10^4$ order level, when tested according to the method described in the section of mutagenicity test in Examples given in this description. The compounds of formula (I) having high specificity to amyloid β include THK-097, THK-203, THK-207, THK-281, THK-525, THK-702, THK-708, THK-727, THK-752, THK-761, THK-763, THK-766. The compounds of formula (I) having high specificity to amyloid β and having little or no mutagenicity include THK-097, THK-203, THK-525, THK-702, THK-70.8, THK-727, THK-752, THK-761, THK-763.

Preferred substituents in the compounds of formula (II) are mentioned below.
Preferably, G is furan, thiophen, pyrrole, pyridine, benzofuran, benzothiophene or indole ring.
The ring may be substituted with $C_{1-6}$ alkyl or O-$C_{1-6}$ alkyl.
Preferably, $R_7$ is hydrogen.
Preferably, $R_8$ is hydrogen or $NR^fR^g$ or

[Formula 27]

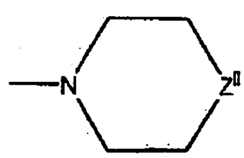

Preferably, $Z^{II}$ is O.
Preferably, $R^f$ and $R^g$ are independently hydrogen or methyl.
p is an integer of from 1 to 4.
The alkyl may be substituted with halogen.

Of the compounds of formula (II), those where $R_8$ is a morpholine ring are more preferred, as they have little or no mutagenicity. The compounds of formula (II) of the type include THK-383, THK-384, THK-385, THK-386, THK-387. The compounds of formula (II) having high specificity to amyloid β are THK-258, THK-262, THK-383, THK-385, THK-386. Accordingly, the compounds of formula (II) having high specificity to amyloid β and having little or no mutagenicity are THK-383, THK-385, THK-386.

Preferred substituents in the compounds of formula (III) are mentioned below.
Preferably, one or both of $X^{III}$ and $Y^{III}$ are C=O.
Preferably, $R^9$ is hydrogen.
At *, the following part bonds to the formula:

[formula 28]

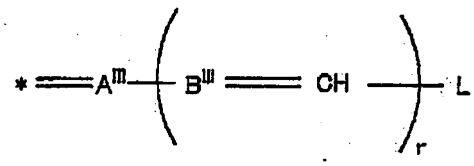

[Formula 29]

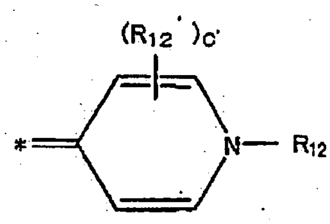

or

[Formula 30]

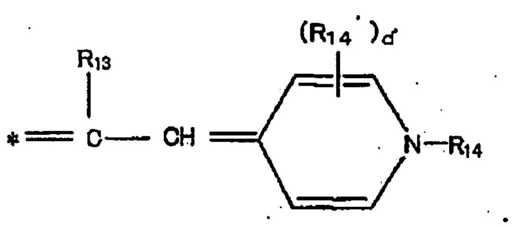

L is

[Formula 31]

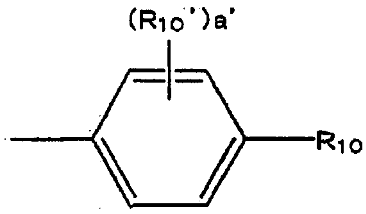

or

[Formula 32]

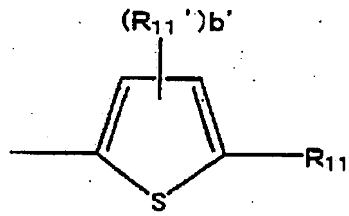

Preferably, $A^{III}$ and $B^{III}$ are CH.

Preferably, $R_{10}$, $R_{11}$, $R_{12}$ and $R_{14}$ are independently hydrogen, $C_{1-6}$ alkyl, or morpholine ring.

Preferably, $R_{13}$ is hydrogen or methyl.

Preferably, $R_{10}'$ , $R_{11}'$, $R_{12}'$ and $R_{14}'$ are independently hydrogen or $C_{1-4}$ alkyl.

Preferably, r is an integer of 0 or 1.

When the substituent is not hydrogen, preferred a', b', c' and d' are independently an integer of 1 or 2.

The alkyl may be substituted with halogen.

If possible, the configuration around the double bond that bonds two ring parts may be any of cis-form or trans-form.

Of the compounds of formula (III), those where $R_{10}$, $R_{11}$, $R_{12}$ or $R_{14}$ is a morpholine are more preferred as they have little or no mutagenicity. The compounds of formula (III) having high specificity to amyloid β are THK-184 and THK-248.

Preferred substituents in the compounds of formula (IV) are mentioned below.

Preferably, $X^{IV}$ is N, NH or $NCH_3$.

Preferably, $Y^{IV}$ is CH or C=O.

The dotted line means an optionally-existing single bond.

At **, the following part bonds to the formula:

[Formula 33]

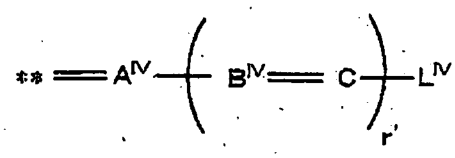

[Formula 34]

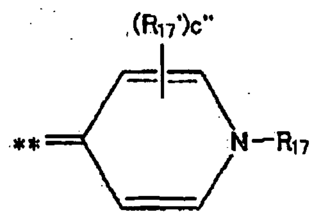

or

[Formula 35]

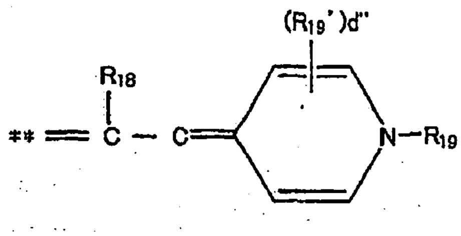

$L^{IV}$ is

[Formula 36]

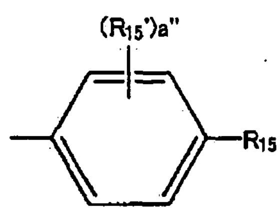

or

[Formula 37]

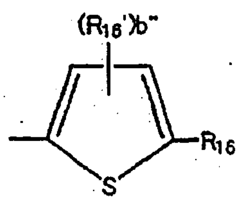

Preferably, $A^{IV}$ and $B^{IV}$ are independently CH or N.
$R_{15}$, $R_{16}$, $R_{17}$ and $R_{19}$ each are hydrogen, $NH_2$, $N(CH_3)_2$ or morpholine ring.
Preferably, $R_{18}$ is hydrogen.
Preferably, $R_{15}'$, $R_{16}'$, $R_{17}'$ and $R_{19}'$ each are hydrogen or $C_{1-4}$ alkali.
Preferably, r' is 1.

a" is an integer of from 1 to 4.
b" is an integer of 1 or 2.
c" is an integer of from 1 to 4.
d" is an integer of from 1 to 4.
$Z^{IV}$ is O, $CH_2$ or N-Re'".
$R^{e'''}$ is hydrogen or $C_{1-4}$ alkyl.
The above alkyl may be substituted with halogen.
If possible, the configuration around the double bond that bonds two ring parts may be any of cis-form or trans-form.

Of the compounds of formula (IV), those where $R_{15}$, $R_{16}$, $R_{17}$ or $R_{19}$ is a morpholine ring are more preferred as they have little or no mutagenicity. The compounds of formula (IV) of the type are THK-276, THK-277 and THK-308. The compounds of formula (IV) having high specificity to amyloid β are THK-185, THK-254, THK-276, THK-308. Accordingly, the compounds of formula (IV) having high specificity to amyloid β and having little or no mutagenicity are THK-276 and THK-308.

Preferred substituents of the compounds of formula (V) are mentioned below.
Preferably, $R_{20}$ is hydrogen, methyl or morpholine ring. The compounds where $R_{20}$ is a morphline ring are more preferred, as having little or no mutagenicity. The compounds of formula (V) having hgih specificity to amyloid β are THK-317, etc.

Preferred substituents of the compounds of formula (VI) are mentioned below.
Preferably, $R_1^{VI}$ is hydrogen, $C_{1-3}$ alkyl, fluorine, chlorine or bromine.
Preferably, $R_2^{IV}$ and $R_3^{VI}$ are methyl.
Preferably, $R_4^{IV}$ is hydrogen.
Preferably, $E^{VI}$ is $CH_2$ or is absent.
$A^{VI}$ is a 5-membered or 6-membered ring, having the following structure:

[Formula 38]

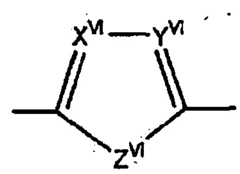

or

[Formula 39]

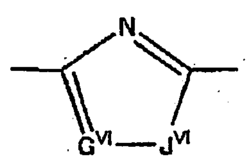

or

[Formula 40]

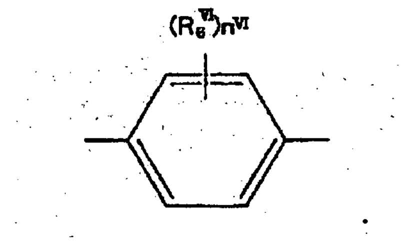

$X^{VI}$ and $Y^{VI}$ are independently N or CH.
Preferably, $Z^{VI}$ is S.
$G^{VI}$ is N or CH.
Preferably, $J^{VI}$ is O, S or $CH_2$.
Preferably, $R_5^{VI}$ is hydrogen, $N(CH_3)_2$ or morpholine ring.
Preferably, all $R_6^{VI}$'S are hydrogen.
Preferably, $n_6^{VI}$ is an integer of 1 or 2.
The configuration around the double bond that bonds two ring parts may be any of cis-form or trans-form.

Of the compounds of formula (VI), those where $R_5{}^{VI}$ is a morpholine ring are more preferred, as having little or no mutagenicity. The compounds of formula (VI) of the type include THK-330, THK-336, THK-533, THK-651, THK-652, THK-653, THK-655. The compounds of formula (VI) having high specificity to amyloid βare THK-556, THK-558, THK-559, THK-561, THK-562, THK-563, THK-565, THK-585.

The invention also encompasses salts of the compounds of the invention. The nitrogen atom or a functional group in the compounds of the invention may from a salt. When the compound has a carboxyl group or a sulfonic acid group, it may form a salt at the group with a metal. Examples of the salts are salts with an alkali metal such as lithium, sodium, potassium; salts with an alkaline earth metal such as magnesium, calcium, barium. When the compound of the invention has a hydroxyl group, the hydrogen may be replaced by a metal such as sodium or potassium, and the invention also encompasses the compounds of the type. When the compound of the invention may form a complex with a metal salt (for example, complex with a metal salt such as magnesium chloride or iron chloride), then such complexes are also within the scope of the salts of the compounds of the invention as referred to in this description. When a salt of the compound of the invention is used in a body of a subject, then the salt is preferably a medically-acceptable salt. The medically-acceptable salt of the compound of the invention includes, for example, a salt with a halide ion such as chloride, bromide, iodide; and a salt with a metal such as sodium, potassium, calcium. The invention encompasses such salts.

The invention also encompasses solvates of the compounds of the invention. The solvates include hydrates, methanol-solvates, ethanol-solvates, ammonia-solvates. When a solvate of the compound of the invention is used in the body of a subject, then the solvate is preferably a medically-acceptable solvate. The medically-acceptable solvate includes hydrates, ethanol-solvates. In this description, the wording, "compounds of the invention" is meant to include the compounds of the invention, as well as salts and solvates thereof.

The invention further provides compounds that are usable as precursors for production of the compounds of the invention. Anyone skilled in the art could easily plan and produce such precursors from the structure of the intended compounds of the invention. Such precursors may also be obtained by modifying commercially-available compounds.

Not labeled, the compound of the invention may be used as a probe for diagnosis of conformation disease. For example, the compound of the invention may be brought into contact with a biopsy sample to confirm the presence or absence of a part of the sample stained with the compound. However, it is general to use a labeled compound of the invention for a diagnostic probe for conformation disease. For the labeling, usable are fluorescent substances, affinity substances, enzyme substrates, radionuclides. For imaging diagnosis of conformation disease, generally used is a radionuclide-labeled probe. According to methods well' known in this technical filed, the compounds of the invention may be labeled with various radionuclides. For example, $^{3}H$, $^{14}C$, $^{35}S$ and $^{131}I$ are radionuclides heretofore used in the art, and there are known many cases of their in-vitro application. General requirements for imaging diagnostic probes and detection methods with them are that they enable *in-vivo* diagnosis, they give little damage to patients (especially, they are noninvasive), their detection sensitivity is high, and they have a suitable half-life period (the time to be taken for preparing labeled probes and the time for diagnosis with them are adequate). Recently, therefore, positron emission tomography (PET) with γ-ray having high detection sensitivity and substance permeability, or single photon emission computed tomography (SPECT) with γ-ray emitter has become used. Of those, PET is preferred since it may readily give informations excellent in resolution and quantification ability, through coincidence spectrometry to detect two γ-rays

that radiate in the completely opposite directions from a positron emitter, with a pair of detectors. For SPECT, the compounds of the invention may be labeled with γ-ray emitter such as $^{99m}Tc$, $^{111}In$,$^{67}Ga$,$^{201}Tl$, $^{123}I$, $^{133}Xe$. $^{99m}TC$ and $^{123}I$ are well used in SPECT. For PET, the compounds of the invention may be labeled with positron emitter such as $^{11}C$ $^{13}N$ $^{15}O$, $^{18}F$, $^{62}Cu$, $^{68}Ga$, $^{76}Br$. Of those positron emitters, $^{11}C$, $^{13}N$, $^{15}O$ and $^{18}F$ are preferred because of easy labelability with them; and $^{18}F$ is more preferred. Regarding the labeling position of the compounds of the invention with a radionuclide such as positron emitter or γ-ray emitter, the compounds may be labeled with it at any position in formula (I). As the case may be, the hydrogen on the ring of the compound may be substituted with a radionuclide such as positron emitter or γ-ray emitter. The compounds of the invention may be labeled at any position thereof, but are preferably labeled at the alkyl group and/or on the phenyl ring in the compounds. The invention encompasses the labeled compounds of the invention. For example, when the compounds of the invention is labeled with $^{18}F$, any side chain thereof may be labeled with $^{18}F$, or the hydrogen on the ring thereof may be substituted with $^{18}F$. In addition, for example, the hydrogen in any alkyl substituent in the compounds may be substituted with $^{18}F$, etc.

In general, the nuclides may be produced by devices that are referred to as cyclotron or generator. Those skilled in the art could select the production method and device suitable to the nuclide to be produced. With the thus-produced nuclide, the compounds of the invention may be labeled.

Methods for producing compounds labeled with such radionuclides are well known in this technical field. Typical methods are a chemical synthesis method, an isotope transfer method and a biosynthesis method. The chemical synthesis method has been used widely from the past, and this does not substantially differ from an ordinary chemical synthesis method except that a radioactive starting substance is used in the former. According to this method, various nuclides are introduced into compounds. The isotope transfer method comprises transferring $^{3}H$, $^{35}S$ or $^{125}I$ in a simple-structured compound into a compound having a complicated structure, thereby giving a compound having a complicated structure and labeled with the nuclide. The biosynthesis method comprises giving a $^{14}C$ or $^{35}S$-labeled compound to cells such as those of microorganisms, thereby producing a metabolite that has the nuclide.

Regarding the labeling position, the synthetic scheme may be planned in accordance with the object, like in ordinary synthesis, whereby the labeling substance may be introduced into the desired position. Those skilled in the art well know the planning.

On the other hand, for example, when a positron emitter having a relatively short half life, such as $^{11}C$, $^{13}N$, $^{15}O$ or $^{18}F$ is used, then a desired nuclide may be obtained in a (ultra)small-size cyclotron installed in an institution such as hospital, thereafter a desired compound may be labeled with it at the desired position thereof according to the above-mentioned method, and it may be directly used for diagnosis, examination or treatment.

According to the methods known to those skilled in the art, the compounds of the invention may be labeled with a desired nuclide by introducing the nuclide to them at the desired position thereof.

The labeled compound of the invention may be administered to a subject either locally or systemically. The administration route includes subcutaneous, intraabdominal, intravenous, intra-arterial or intraspinal injection or infusion; and it may be selected depending on the factors such as the type of the disease, the nuclide used, the compound used, the condition of the subject and the examination site. After the probe of the invention has been administered and after a sufficient period of time has passed for binding the compound to amyloid β protein and disintegrating it, the examination site may be inspected according to means of PET or SPECT. These means may be suitably selected depending on the factors such as the type of the disease, the nuclide used, the compound used, the condition of the subject and the examination site.

The dose of the radionuclide-labeled compound of the invention varies depending on the type of the disease, the nuclide used, the compound used, the age of the subject, the physical condition, the sex, the degree of the disease and the examination site thereof. In particular, special attention should be paid to the dose equivalent to be applied to the subject. For example, the radioactivity of the compound of the invention labeled with a positron emitter such as $^{11}C$, $^{13}N$, $^{15}O$ or $^{18}F$ is within a scope of generally from 3.7 megabecquerels to 3.7 gigabecquerels, preferably from 18 megabecquerels to 740 megabecquerels.

The compounds of the invention, or salts or solvates thereof are suitable for a treatment method, a diagnosis method, a remedial composition, a diagnostic composition or a diagnostic kit for conformation disease mentioned below, for use in producing the composition and kit, and for other use. Preferred are the compounds or salts or solvates thereof mentioned hereinabove for exemplification of the compounds of formulae (I) to (VI); and more preferred are those within the scope of the compounds of formula (I) or salts or solvates thereof. Of the compounds of the invention, those having a morpholine ring, especially those having a morpholine at the terminal thereof are suitable to administration to humans, as they have little or no mutagenicity.

The invention provides a composition for imaging diagnosis of conformation disease, which comprises the compound of the invention. The composition of the invention comprises the compound of the invention and a medically-acceptable carrier. Preferably, the compound of the invention in the composition is labeled. There are known various labeling methods as mentioned above, but for in-vivo imaging diagnosis application, the compound is preferably labeled with a radionuclide (especially for PET, positron emitter such as $^{11}C$, $^{13}N$, $^{15}O$, $^{16}F$). Regarding its form, the composition

of the invention is preferably an injectable or infusible one in view of its object. Accordingly, the medically-acceptable carrier is preferably liquid, for example, a water-based solvent such as potassium phosphate buffer, physiological saline water, Ringer solution, distilled water, or a non-aqueous solvent such as polyethylene glycol, vegetable oil and fat, ethanol, glycerin, dimethyl sulfoxide, propylene glycol, to which, however, the invention should not be limited. The blend ratio of the carrier and the compound of the invention may be suitably determined depending on the application site and the detection means, and in general, it may be from 100,000/1 to 2/1, preferably from 10,000/1 to 10/1. The composition of the invention may contain any known microbicide (e.g., antibiotic), local anesthetic (e.g., procaine hydrochloride, dibucaine hydrochloride), buffer (e.g., tris-HCl buffer, Hepes buffer), osmoregulator (e.g., glucose, sorbitol, sodium chloride).

The invention further provides a kit for imaging diagnosis of conformation disease, which comprises the compound of the invention as the indispensable constitutive ingredient thereof. In general, the kit comprises the components such as a compound of the invention, a solvent for dissolving it, a buffer, an osmoregulator, a microbicide and a local anesthetic, which are individually packaged or are partly combined and packaged together, and are packed in one container. The compound of the invention may be unlabeled or labeled. When the compound is unlabeled, it may be labeled before use according to the ordinary method described in the above. If desired, the compound of the invention may be provided as a solid such as a freeze-dried powder; or it may be provided as a solution prepared by dissolving it in a suitable solvent. The solvent may be the same as that for the carrier for the composition of the invention mentioned hereinabove. The other components such as buffer, osmoregulator, microbicide and local anesthetic may also be the same as those for use in the composition of the invention mentioned in the above. Various types of containers may be suitably selected and used. The containers may have a shape suitable for labeling of the compounds of the invention, or may be formed of a light-shielding material depending on the properties of the compounds. For example, the containers may have a shape of vials or syringes capable of facilitating administration to patients. The kit may comprise appliances necessary for diagnosis, such as syringe, fusion set, as well as appliances for use in PET or SPECT apparatus. In general, an instruction booklet is attached to the kit.

Since the compounds of the invention may specifically bind to amyloid β protein, they may be used for detection and quantification of amyloid β protein in a sample by bringing the compound into in-vitro contact with a sample, while unlabeled or after labeled. For example, the compounds of the invention may be used for amyloid β protein staining of a sample in microscopy, for colorimetry of amyloid β protein in a sample, or for quantification of amyloid β protein with a scinitillation counter. Preparing the sample for microscopy and staining it with the compound of the invention may be attained by any ordinary method known to those skilled in the art.

As so mentioned hereinabove, the compounds of the invention have high specificity to amyloid P protein. Accordingy, the compounds of the invention are useful, for example, for studies of amyloid β protein deposition-related disorders or for diagnosis thereof while alive or after death. For example, the compounds may be useful as a staining agent for seline plaque in the brain of an Alzheimer disease patient. Staining a sample, such as a brain section with the compound of the invention may be attained by any ordinary method known to those skilled in the art.

Of the compounds of the invention, those having a morpholine ring as a terminal group thereof have little or no mutagenicity, as so mentioned hereinabove. Accordingly, the compounds of the invention of the type are not only an extremely safe diagnostic probe for conformation disease but also they may be highly safe when used as a remedial agent or a preventive agent to be described hereinunder.

Accordingly, the invention relates to a composition for staining amyloid β protein in a sample, which comprises the compound of the invention, or medically-acceptable salt or solvate thereof, and to a kit for staining amyloid β protein in a sample, which comprises, as the indispensable constitutive component thereof, the compound of the invention, or medically-acceptable salt or solvate thereof. Further, the invention relates to a method for staining amyloid β protein in a sample, which comprises using the compound of the invention, or medically-acceptable salt or solvate thereof. The sample suitable to staining is a brain section.

As mentioned above, it is known that β sheet structured amyloid β protein exhibits neuron toxicity. The compounds of the invention may specifically bind to β sheet structured amyloid β protein, and therefore they may inhibit its neuron toxicity. Accordingly, the compounds of the invention may be a remedial or preventive agent for conformation disease such as Alzheimer disease, of which the cause or a part of the cause is that protein having a β sheet structure.

Accordingly, the invention provides the following:

A method for treatment and/or prevention of amyloid β protein deposition-related diseases, characterized by administering a compound of formula (I) or a salt or a solvate thereof;
A method for diagnosis of amyloid β protein deposition-related diseases, characterized by using a compound of formula (I) or a salt or a solvate thereof; and
Use of the compound of formula (I) or a salt or a solvate thereof for production of a composition or kit for treatment, prevention or diagnosis of amyloid β protein deposition-related diseases.

Not specifically defined, the form of the medical composition is preferably a liquid preparation, more preferably that for injection. The injection may be directly injected into a brain, or the medical composition may be administered through intravenous injection or infusion since the compound of the invention has high blood-brain barrier permeability as in Example 3. The liquid preparation may be produced in any method known in the art. For producing a solution-type preparation, for example, a compound of the invention may be dissolved in a suitable carrier, injection water, physiological saline water or Ringer solution, then sterilized through a filter, and thereafter it may be filled in suitable containers such as vials or ampoules. As the case may be, the solution preparation may be freeze-dried, and may be restored to its solution with a suitable carrier just before use. A suspension-type preparation may be produced, for example, by sterilizing a compound of the invention through exposure to ethylene oxide, and then suspending it in a sterilized liquid carrier.

The dose of the compound of the invention to a human subject in the above-mentioned therapeutical method, preventive method and use may vary depending on the condition of a patient, the sex, the age and the body weight thereof, but in general, the dose thereof to an adult having a body weight of 70 kg may be from 0.1 mg to 1 g a day, preferably from 1 mg to 100 mg, more preferably from 5 mg to 50 mg. After a patient is treated at the dose for a predetermined period of time, the dose may be increased or decreased depending on the therapeutical result.

The compounds of the invention, or salts or solvates thereof may be used as a diagnostic probe for conformation disease, preferably as an imaging diagnostic probe that is labeled with a radiation emitter. Further, the compounds of the invention are effective for treatment and/or prevention of conformation disease. Accordingly, the invention relates to the following:

A compound of the invention or a salt or a solvate thereof for use as an imaging diagnostic probe for conformation disease;

An imaging diagnostic composition or kit that comprises a compound of the invention or a salt or a solvate thereof;

A medical composition for prevention and/or treatment of conformation disease, comprising the compound of the invention or medically-acceptable salt or solvate thereof, and a medically-acceptable carrier;

A method for diagnosis of conformation disease, characterized by using a compound of the invention or medically-acceptable salt or solvate thereof;

Use of the compound of the invention or medically-acceptable salt or solvate thereof for diagnosis of conformation disease;

A method for prevention and/or treatment of conformation disease, characterized by administering a compound of the invention or medically-acceptable salt or solvate thereof to a subject;

Use of the compound of the invention or medically-acceptable salt or solvate thereof for prevention and/or treatment of conformation disease; and

Use of the compound of the invention in production of a medical composition for prevention and/or treatment of conformation disease.

The dose of the compound of the invention to a human subject in the above-mentioned therapeutical method and preventive method may be as mentioned in the above.

Some of the compounds of the invention may recognize neurofibrillary tangle, and therefore may be used as a detection probe for neurofibrillary tangle or as a staining agent for neurofibrillary tangle. Accordingly, the invention also relates to use of the compounds of the invention or salts or solvates thereof for a diagnostic probe, especially an imaging diagnostic probe for neurofibrillary tangle. The compounds of the invention preferred for a staining agent for neurofibrillary tangle are THK-185, THK-248, THK-254, THK-258, THK-317, THK-386, THK-727.

Accordingly, the invention provides the following:

A composition for detecting or staining neurofibrillary tangle, which comprises a compound of the invention or a salt or a solvate thereof;

A kit for detecting or staining neurofibrillary tangle, which comprises a compound of the invention or a salt or a solvate thereof;

A method for detecting or staining neurofibrillary tangle, which comprises using a compound of the invention or a salt or a solvate thereof; and

Use of the compound of the invention or a salt or a solvate thereof for producing a composition for detecting or staining neurofibrillary tangle.

Preparing the sample for detection and staining neurofibrillary tangle therein and staining it may be attained by any ordinary method known to those skilled in the art.

The invention is described concretely with reference to the following Examples, which, however, do not restrict the invention at all.

Example 1:

Investigation of models with amyloid β protein deposition in their brain:

Investigation of transgenic (Tg) mice with amyloid deposition in their brain:

(1) Tg mice (Tg2576 or APPswe2576/Tau JPL3) were used. A test compound was administered to a Tg mouse via its tail vein. After 1 hour, the chest of the mouse was cut open under deep anesthesia with Na-pentobarbital, and 10 % neutral buffer formalin was transcardially perfused through the mouse to fix it.
(2) The head was cut open, and its brain was taken out, and dipped in 30 % sucrose for 12 hours or more. Next, the taken-out brain was immediately frozen in finely-ground dry ice, and using a cryostat (by Bright, Model-OT), a frozen section of the brain was formed on a poly-L-lysine-coated slide.
(3) Not entrapped, the thus-prepared brain section was microscopically observed with a fluorescence microscope (Nikon Eclipse 80i), and photographed with a digital camera (Nikon Dxm 1200F or Photometrics' Cool SNAP ES). The results are shown in Figs. 1 to 3. THK-097, THK-525 and THK-727 intravenously administered to the animals passed through the blood-brain barrier, and bound to the Tg mouse intracerebral amyloid plaque.

The same section was immunostained as follows:

(1) About 150 μl of 90 % formic acid was dropwise applied to the same section, and statically left as at room temperature for 5 minutes. This was washed with tap water for 5 minutes, and then dipped in cold PBS-Tween 20 for 2 minutes, and thereafter about 150 μl of 0.05 % trypsin solution was dropwise applied to it and reacted at 37°C for 15 minutes.
(2) In an ice bath, this was washed with cold PBS-Tween 20, twice for 5 minutes, and then 2 drops of blocking serum were applied to it and reacted at 37°C for 30 minutes. Excess water was removed, and then about 150 μl of a specific antibody to amyloid β protein, 4G8 (by Chemicon, 1/100 dilution) was dropwise applied to it, and reacted at 37°C for 1 hour.
(3) Further, this was washed with cold PBS-Tween 20, 5 times for 2 minutes, and then 2 drops of an antimouse IgG (H+L), goat, biotin-binding solution were applied to it and reacted at 37°C for 1 hour. Then, this was washed with cold PBS-Tween 20, three times for 2 minutes, and 2 drops of an ABC solution (streptoavidin-biotin-peroxidase composite solution) were applied to it, and left statically as such for 30 minutes. Again this was washed with cold PBS-Tween 20, three times for 2 minutes, and then about 150 μl of a DAB solution (10 mg of DAB was dissolved in 20 ml of 0.05 mol/liter tris-HCl buffer, and just before use, 100 μl of 3 % hydrogen peroxide water was added to it) was dropwise applied to it for sufficiently staining it. Then, this was washed with distilled water for 1 minute to stop the reaction, then entrapped and observed through microscopy.

The results are shown in Fig. 4 and Fig. 5. THK-702 passed through the blood-brain barrier, and bound to the amyloid plaque of the Tg mouse (Fig. 3, upper panel), and the binding site corresponded to the anti-Aβ antibody stained site of the same section (Fig. 3, lower panel).
Fig. 4 shows enlarged images of Fig. 3. A, B and C correspond to A, B and C in Fig. 3, respectively. THK-702 bound to the amyloid plaque of the Tg mouse (Fig. 4, left panels), and the binding site completely corresponded to the anti-Aβ antibody-stained site of the same section (Fig. 4, right panels).
The protocol of a staining test with the compound of the invention on a brain section of an Alzheimer disease patient is described below.

(1) Brain specimens at the temporal lobe and the hippocampus of patients to which a definitive diagnosis of Alzheimer disease had been given, and those of normal senile persons were used. The specimens were given by our coworker, Fukushimura Hospital, Longevity Medicine Institute, and we were given their consent of use of the specimens for our study purpose by the bereaved of the patients (Fukushimura Hospital Ethical Review Board Approval No. 20).
(2) The paraffin-embedded brain tissue was cut into slices having a thickness of 6 μm or 8 μm, and extended and dried on a slide. The paraffin brain section was processed for paraffin removal in xylene twice for 10 minutes, 100 % ethanol twice for 5 minutes, 90 % ethanol for 5 minutes and running water for 10 minutes in that order.
(3) For pretreatment prior to the staining treatment with a compound of the invention, the sections were processed for autofluorescence removal with lipofuscin. First, the paraffin-removed sections were dipped in 0.25 % $KMnO_4$ solution for 20 minutes. These were washed with PBS, twice for 2 minutes, then dipped in 0.1 % $K_2S_2O_5$/oxalic acid solution for about 5 seconds, and then further washed with PBS, three times for 2 minutes.
(4) About 150 μl of a 100 μM solution of a compound of the invention dissolved in 50 % ethanol was dropwise applied to the section and reacted for 10 minutes. After dipped five times in tap water, the section was entrapped

with Fluor Save Reagent (Calbiochem), and analyzed through microscopy with a fluorescence microscope (Nikon, Eclipse 80i). The images were taken with a digital camera (Nikon Dxm 1200F or Photometrics' Cool SNAP ES).

Immunostaining was attained as follows:

(a) Method of immunostaining of amyloid β protein:

(1) After paraffin removal, the sections were washed in distilled water, twice for 2 minutes. Then, using ImmunoPen, the tissue was marked with a surrounding line; about 150 μl of formic acid was dropwise applied to the section and statically left at room temperature for 5 minutes. The section was washed with tap water for 5 minutes, then dipped in cold PBS-Tween 20 for 2 minutes, and thereafter about 150 μl of 0.05 % trypsin solution was dropwise applied to it and reacted at-37˚C for 15 minutes.
(2) In an ice bath, this was washed with cold PBS-Tween 20, twice for 5 minutes, and then 2 drops of blocking serum were applied to it and reacted at 37˚C for 30 minutes. Excess water was removed, and then about 150 μl of a specific antibody to amyloid β protein, 6F/3D (by DAKO, 1/50 dilution) was dropwise applied to it, and reacted at 37˚C for 1 hour.
(3) Further, this was washed with cold PBS-Tween 20, 5 times for 2 minutes, and then 2 drops of an antimouse IgG (H+L), goat, biotin-binding solution were applied to it and reacted at 37˚C for 1 hour. Then, this was washed with cold PBS-Tween 20, three times for 2 minutes, and 2 drops of an ABC solution (streptoavidin-biotin-peroxidase composite solution) were applied to it, and left statically as such for 30 minutes. Again this was washed with cold PBS-Tween 20, three times for 2 minutes, and then about 150 μl of a DAB solution (10 mg of DAB was dissolved in 20 ml of 0.05 mol/liter tris-HCl buffer, and just before use, 100 μl of 3 % hydrogen peroxide water was added to it) was dropwise applied to it for sufficiently staining it. Then, this was washed with distilled water for 1 minute to stop the reaction, then entrapped and observed through microscopy.

(b) Method of immunostaining of neurofibrillary tangle:

(1) After paraffin removal treatment, the section was washed with cold PBS-Tween 20, twice for 5 minutes, and then 2 drops of blocking serum were applied to it and reacted at 37˚C for 30 minutes. Excess water was removed, and then 2 drops of an antibody specific to tau, AT-8 (by Mia Nobels, 1/10.0 dilution) were applied to it, and reacted overnight at 4˚C.
(2) On the next day, this was washed with cold PBS-Tween 20, five times for 2 minutes, and then 2 drops of an anti-rabbit IgG, goat, biotin-binding solution were applied to it and reacted at 37˚C for 1 hour. Then, this was washed with cold PBS-Tween 20, three times for 2 minutes, and 2 drops of an ABC solution (streptoavidin-biotin-peroxidase composite solution) were applied to it, and left statically as such for 30 minutes.
(3) Again this was washed with cold PBS-Tween 20, three times for 2 minutes, and then about 150 μl of a DAB solution (10 mg of DAB was dissolved in 20 ml of 0.05 mol/liter tris-HCl buffer, and just before use, 100 μl of 3 % hydrogen peroxide water was added to it) was dropwise applied to it for sufficiently staining it. Then, this was washed with distilled water for 1 minute to stop the reaction, then entrapped and observed through microscopy. The blocking serum, the anti-rabbit IgG, goat, biotin-binding solution and the ABC solution used herein were those in a phosphorylated tau immunohistostain kit (Wako 299-57301).

The results of the above-mentioned staining tests with the compounds of the invention are shown in Fig. 2 to Fig. 26. THK-097 bound to the amyloid βprotein in the brain section of an Alzheimer disease patient (Fig. 2). THK-184 bound to the amyloid protein in the brain section of an Alzheimer disease patient (Fig. 3). THK-185 bound to the amyloid β protein and the neurofibrillary tangle in the brain section of an Alzheimer disease patient (Fig. 4). THK-203 bound to the amyloid β protein in the brain section of an Alzheimer disease patient (Fig. 5). THK-207 bound to the amyloid β protein in the brain section of an Alzheimer disease patient (Fig. 6). THK-248 bound to the amyloid β protein in the brain section of an Alzheimer disease patient (Fig. 7). THK-254 bound to the neurofibrillary tangle in the brain section of an Alzheimer disease patient (Fig. 8). THK-258 bound to the amyloid β protein and the neurofibrillary tangle in the brain section of an Alzheimer disease patient (Fig. 9). THK-262 bound to the amyloid β protein in the brain section of an Alzheimer disease patient (Fig. 10). THK-276 bound to the amyloid β protein in the brain section of an Alzheimer disease patient (Fig. 11). THK-281 bound to the amyloid β protein in the brain section of an Alzheimer disease patient (Fig. 12). THK-308 bound to the amyloid β protein in the brain section of an Alzheimer disease patient (Fig. 13). THK-317 bound

to the amyloid β protein and the neurofibrillary tangle in the brain section of an Alzheimer disease patient (Fig. 14). THK-383 bound to the amyloid βprotein in the brain section of an Alzheimer disease patient (Fig. 15). THK-385 bound to the amyloid β protein in the brain section of an Alzheimer disease patient (Fig. 16). THK-386 bound to the amyloid β protein and the neurofibrillary tangle in the brain section of an Alzheimer disease patient (Fig. 17). THK-525 bound to the amyloid β protein in the brain section of an Alzheimer disease patient (Fig. 18). THK-556 bound to the amyloid β protein in the brain section of an Alzheimer disease patient (Fig. 19). THK-558 bound to the amyloid β protein in the brain section of an Alzheimer disease patient (Fig. 20). THK-559 bound to the amyloid protein in the brain section of an Alzheimer disease patient (Fig. 21). THK-561 bound to the amyloid β protein in the brain section of an Alzheimer disease patient (Fig. 22). THK-562 bound to the amyloid β protein in the brain section of an Alzheimer disease patient (Fig. 23). THK-563 bound to the amyloid β protein in the brain section of an Alzheimer disease patient (Fig. 24). THK-565 bound to the amyloid β protein in the brain section of an Alzheimer disease patient (Fig. 25). THK-585 bound to the amyloid protein in the brain section of an Alzheimer disease patient (Fig. 26). In that manner, it has been found that the compounds of the invention can specifically recognize the amyloid β protein and the neurofibrillary tangle in the brain sections of Alzheimer disease patients. In addition, it has also been found that the other compounds of the invention than those mentioned above can also specifically bind to amyloid β protein.

Test methods for the properties of the compounds of the invention are described below.

(A) Acute Toxicity Test:

The compounds of the invention were tested for their acute toxicity through intravenous administration thereof to mice. Four Crj:CD1 male mice formed one test group, and some test groups of those mice were used (the mean body weight of the mice of each group was from 31 to 32 g). A test compound was dissolved in a mixture of 1 N HCl, polyethylene glycol-400 and distilled water, or dissolved in DMSO, and then diluted with distilled water. This was administered to each mouse via its tail vein. After that, the mice were observed for 7 days. The results are shown in Table 2.

[Table 2]

Table 2 - Acute Toxicity Test of Compounds of the Invention

| | Maximum Permissible Dose (mg/kg, intravenous administration) |
|---|---|
| THK-525 | ≥10 |
| THK-702 | ≥10 |
| THK-707 | ≥10 |
| THK-708 | ≥10 |
| THK-713 | ≥10 |
| THK-727 | ≥10 |
| THK-752 | ≥10 |
| THK-757 | ≥10 |
| THK-761 | ≥10 |
| THK-763 | ≥10 |
| THK-765 | ≥10 |
| THK-766 | ≥10 |

(B) Brain Permeability Test:

I. Brain Permeability Test through HPLC:

A compound of the invention was intravenously administered to mice, and tested for its in-vivo brain permeability in the mice.

(1) Slc:ICR mice (by Nippon SLC) (body weight, 30 to 40 g; n = 3) were used.
(2) A test compound was dissolved in a mixture of 5% Tween 80-5 % ethyl alcohol-5 % 1 N HCl-physiological saline water, and injected into the test mouse via its tail vein. Two minutes after the administration, the blood was collected

from the test mouse under ether anesthesia via its abdominal aorta using a heparin-processed syringe, and the brain material was collected from it.

(3) After collected, the blood was centrifuged at 4˚C and 14,000 rpm for 10 minutes, and the supernatant was stored at -80˚C as plasma. After collected, the brain material (including the cerebellum) was stored at -80˚C.

(4) 0.3 ml of acetonitrile was added to 0.1 ml of the plasma, then vortexed, and centrifuged at 4˚C and under 10,000 G for 5 minutes. After centrifuged, 0.2 ml of the resulting supernatant was transferred into Mini-Uniprep (Whatman), then 0.2 ml of 20 mM phosphate buffer was added to it and filtered. 0.2 ml of the resulting filtrate was analyzed through HPLC (Shiseido Nanospace SI-2; pump, 3001; UV-VIS detector, 3002; column thermostat, 3004; fluorescence detector, 3013).

(5) 2 ml of methanol was added to the brain, homogenized, and centrifuged at 4˚C and 3000 or 4000 rpm for 10 minutes. After centrifuged, 500 μl of the resulting supernatant was diluted 10-fold with 20 mM phosphate buffer. (i) 2 to 3 ml of acetonitrile, (ii) 2 to 5 m 1 of methanol and (iii) 4 to 6 ml of ultra-pure water were led through a cartridge for solid-phase extraction (J. T. Baker Speedisk) in that order, and the 10-fold diluted supernatant solution was led through it. Via an empty syringe, air was applied to the cartridge for solid-phase extraction, two or three times to remove water, and then this was eluted with about 500 μl of acetonitrile or methanol, and the eluate was diluted 2-fold with 20 mM phosphate buffer. 0.2 ml of the resulting solution was analyzed through HPLC.

(6) The test compound content in the plasma and in the brain was determined relative to the dose of the compound (% ID (injected dose)/g or ml).

Table 3 shows the brain permeability of the test compound in mice in 2 minutes after intravenous administration of the compound. It is considered that the brain permeability of a compound necessary for PET or SPECT directed to a central nervous system will be at least 0.5 % ID/g. To that effect, the compounds tested herein have an extremely high brain permeability.

[Table 3]

| Table 3 - Brain Permeability of Compound of the Invention in 2 minutes after intravenous administration (mice) | | |
|---|---|---|
| | % ID/g or ml | |
| | Brain | Plasma |
| THK-525 | 7.9 | 3.1 |
| THK-702 | 5.1 | 2.7 |
| THK-707 | 4.4 | 0.58 |
| THK-713 | 5.9 | 3.29 |
| THK-752 | 6.7 | 1.9 |

II. Investigation with [$^{18}$F]-labeled Compounds:

Labeling Production of [$^{18}$F]THK525:

A positron beam of 12 MeV, accelerated with a cyclotron HM12 (by Sumitomo Heavy Industries), was applied to [18O] $H_2O$ having an isotope purity of at least 95%, for 30 minutes, thereby producing 18$F^-$. Next, the solution was led through an anion exchange resin (AG1-X8) whereby $^{18}F^-$ was trapped by the resin, and then this was eluted with 33 mM $K_2CO_3$ solution. 300 μL (3.28 GBq) of the aqueous $^{18}F^-$- containing $K_2CO_3$ solution was put into a brown vial (capacity 10 mL), and Kryptofix 222 (16 mg) and acetonitrile (2 mL) were added to it. Then, with heating in an oil bath (110˚C), He gas was jetted to it, and acetonitrile was completely removed while azeotroped with water. Further, acetonitrile (3 mL) was added to it, and acetonitrile was removed under the same heating condition. This operation was repeated three times, whereby the vial was made to contain no water. A DMSO solution (0.8 mL) with a labeling precursor THK575 (1.9 mg) dissolved therein was added to it, and stirred under heat in an oil bath (110˚C) for 10 minutes. Next, the DMSO solution was led through a Sep-Pak™ alumina cartridge (by Waters) and a filter (0.5 μm), and the resulting filtrate was processed through semi-partitioning HPLC (column, Inertsil™ ODS-3 (10 x 250 mm); mobile phase, EtOH/MeCN/20 mM $NaH_2PO_4$ = 15/45/40; flow rate, 5.0 mL/min), in which the [$^{18}$F]THK525- derived radioactive peak that was eluted in about 11 to 12 minutes was collected. The radiochemical yield after attenuation correction, obtained from the radioactivity of the fraction, was 42 %; and the radiochemical purity was 99% or more.

Labeling Production of [$^{18}$F] THK702:

A positron beam of 12 MeV, accelerated with a cyclotron HM12 (by Sumitomo Heavy Industries), was applied to [$^{18}$O]$H_2O$ having an isotope purity of at least 95 %, for 30 minutes, thereby producing $^{18}F^-$. Next, the solution was led through an anion exchange resin (AG1-X8) whereby $^{18}$F-was trapped by the resin, and then this was eluted with 33 mM $K_2CO_3$ solution. 200 μL (3.24 GBq) of the aqueous $^{18}F^-$- containing $K_2CO_3$ solution was put into a brown vial (capacity 10 mL), and Kryptofix 222 (16 mg) and acetonitrile (3 mL) were added to it. Then, with heating in an oil bath (110˚C), He gas was jetted to it, and acetonitrile was completely removed while azeotroped with water. Further, acetonitrile (3 mL) was added to it, and acetonitrile was removed under the same heating condition. This operation was repeated three times, whereby the vial was made to contain no water. A DMSO solution (0.8 mL) with a labeling precursor THK703 (2.2 mg) dissolved therein was added to it, and stirred under heat in an oil bath (110˚C) for 10 minutes. Next, the reaction solution was diluted with distilled water (8 mL), and loaded in Sep-Pak tC18 cartridge (by Waters). Then, the cartridge was washed with distilled water, and eluted with EtOH, and the resulting eluate was processed through semi-partitioning HPLC (column, Inertsil™ ODS-3 (10 × 250 mm); mobile phase, MeCN/20 mM $NaH_2PO_4$ = 40/60; flow rate, 7.0 mL/min), in which the [$^{18}$F]THK702-derived radioactive peak that was eluted in about 11 minutes was collected. The radiochemical yield after attenuation correction, obtained from the radioactivity of the fraction, was 21 %; and the radiochemical purity was 99 % or more.

Labeling Production of [$^{18}$F]THK727:

A positron beam of 12 MeV, accelerated with a cyclotron HM12 (by Sumitomo Heavy Industries), was applied to [$^{18}$O] $H_2O$ having an isotope purity of at least 95 %, for 30 minutes, thereby producing $^{18}F^-$. Next, the solution was led through an anion exchange resin (AG1-X8) whereby $^{18}F^-$ was trapped by the resin, and then this was eluted with 33 mM $K_2CO_3$ solution. 100 μL (1.27 GBq) of the aqueous $^{18}F^-$- containing $K_2CO_3$ solution was put into a brown vial (capacity 10 mL), and Kryptofix 222 (10 mg) and acetonitrile (3 mL) were added to it. Then, with heating in an oil bath (110˚C), He gas was jetted to it, and acetonitrile was completely removed while azeotroped with water. Further, acetonitrile (3 mL) was added to it, and acetonitrile was removed under the same heating condition. This operation was repeated three times, whereby the vial was made to contain no water. A DMSO solution (0.4 mL) with a labeling precursor THK726 (1.9 mg) dissolved therein was added to it, and stirred under heat in an oil bath (110˚C) for 10 minutes. Next, the DMSO reaction solution was led through a Sep-Pak™ alumina cartridge (by Waters) and a filter (0.5 μm), then DMSO (0.2 mL) was additionally given to it. The resulting filtrate was processed through semi-partitioning HPLC (column, Inertsil™ ODS-3 (10 × 250 mm); mobile phase, MeCN/20 mM $NaH_2PO_4$ = 40/60; flow rate, 5.0 mL/min), in which the [$^{18}$F]THK727-derived radioactive peak that was eluted in about 23 minutes was collected. The radiochemical yield after attenuation correction, obtained from the radioactivity of the fraction, was 44 %; and the radiochemical purity was 99 % or more.

Labeling Production of [$^{18}$F]THK763:

A positron beam of 12 MeV, accelerated with a cyclotron HM12 (by Sumitomo Heavy Industries), was applied to [$^{18}$O]$H_2O$ having an isotope purity of at least 95%, for 30 minutes, thereby producing $^{18}F^-$. Next, the solution was led through an anion exchange resin (AG1-X8) whereby $^{18}F^-$ was trapped by the resin, and then this was eluted with 33 mM $K_2CO_3$ solution. 200 μL (3.02 GBq) of the aqueous $^{18}F^-$- containing $K_2CO_3$ solution was put into a brown vial (capacity 10 mL), and Kryptofix 222 (16 mg) and acetonitrile (3 mL) were added to it. Then, with heating in an oil bath (110˚C), He gas was jetted to it, and acetonitrile was completely removed while azeotroped with water. Further, acetonitrile (3 mL) was added to it, and acetonitrile was removed under the same heating condition. This operation was repeated three times, whereby the vial was made to contain no water. A DMSO solution (0.7 mL) with a labeling precursor THK762 (3.1 mg) dissolved therein was added to it, and stirred under heat in an oil bath (110˚C) for 10 minutes. Next, the reaction solution was diluted with distilled water (7 mL), and loaded in Sep-Pak tC18 cartridge (by Waters). Then, the cartridge was washed with distilled water and MeCN/20 mM $NaH_2PO_4$ (v/v = 3/7, 5 mL) in that order, and eluted with EtOH, and the resulting eluate was processed through semi-partitioning HPLC (column, Inertsil™ ODS-3 (10 × 250 mm); mobile phase, MeCN/20 mM $NaH_2PO_4$ = 35/65; flow rate, 6.0 mL/min), in which the [$^{18}$F]THK763-derived radioactive peak that was eluted in about 24 to 25 minutes was collected. The radiochemical yield after attenuation correction, obtained from the radioactivity of the fraction, was 38 %; and the radiochemical purity was 99 % or more.

Preparation of Labeled Compound-Containing Physiological Saline:

The partitioning HPLC fraction containing [$^{18}$F] THK525, [$^{18}$F] THK702, [$^{18}$F] THK727 or [$^{18}$F] THK763, obtained through labeling production, was diluted with distilled water (about 20 mL), then loaded in Sep-Pak tC18 cartridge (by waters). The cartridge was washed with distilled water (5 to 10 mL), and then the labeled compound was eluted with

EtOH (3 to 5 mL). A suitable amount of 5 % Polysorbate 80/ethanol solution was added to the EtOH eluate, and the solvent was evaporated away with heating at 80˚C with a rotary evaporator. Thus obtained, a mixture of the labeled compound and Polysorbate 80 was dissolved in physiological saline, thereby preparing a labeled compound-containing physiological saline. Thus prepared, the radiochemical purity of the chemical solution was 95 % or more.

Assessment of Brain Permeability of Labeled Compound in Mice:

[$18^F$] THK525, [$^{18}$F] THK702, [$^{18}$F]THK727 or [$^{18}$F]THK763-containing physiological saline was administered to male ICR mice (6 to 7 weeks-age) via their tail vein. From the radioactivity deposition in the brain after 2 and 30 minutes, the brain permeability of the labeled compound was assessed. The radiochemical purity of the labeled compound-containing physiological saline used was at least 95 %, and the relative radioactivity thereof was from 18.5 to 148 GBq/μmol; and from 1.11 to 2.22 MBq of the labeled compound was administered to one mouse. Regarding the assessment of the radioactivity deposition, the proportion of the radioactivity per the unit weight of the sample tissue to the overall radioactivity given to the test animal (% injected dose/g of tissue; % ID/g) was taken as the index. For measuring the radioactivity, used was a gamma counter (1480 WIZARD, by Perkin Elmer). The experiment protocol was as follows: A labeled compound was administered to mice via their tail vein. After 2 and 30 minutes, the cervical spine of the mouse was dislocated under ether anesthesia. Immediately, the blood was collected from its heart, and the whole brain (including cerebellum and brainstem) was taken out. The radioactivity and the tissue weight of each sample were measured, and from the data, % ID/g was computed.

Table 4 shows the brain permeation of the [$^{18}$F]-labeled test compound in 2 minutes after the intravenous administration of the compound in mice. It is considered that the brain permeability of a compound necessary for PET or SPECT directed to a central nervous system will be at least 0.5% ID/g. To that effect, the following [$^{18}$F]-labeled test compounds have an extremely high brain permeability.
[Table 4]

Table 4 - Brain Permeability of [$^{18}$F]-labeled Compound of the Invention in 2 minutes after intravenous administration (mice)

| | % ID/g or ml | |
|---|---|---|
| | Brain | Plasma |
| [$^{18}$F] THK-525 | 4.7 | 2.8 |
| [$^{18}$F]THK-702 | 4.2 | 2.3 |
| [$^{18}$F] THK-727 | 4.1 | 2.9 |
| [$^{18}$F] THK-763 | 4.6 | 3.7 |

Mutagenicity Test:

In view of their use, it is desirable that the compounds of the invention have no mutagenicity or have little mutagenicity to a level causing no problem. For investigating the gene mutagenesis of the compounds of the invention, herein carried out was a reverse mutation test with histidine-requiring *Salmonella typhimurium* TA100 and TA98 strains. Two tests were carried out. One is a test for dose determination test; and the other is for mutagenicity.
The test methods and the mutagenicity of the compounds of the invention are mentioned below. In the dose determination test, six doses of 0.160, 0.800, 4.00, 20.0, 100 and 500 μg/plate (common ratio 5) were tried.
As a result of the dose determination test, when the mutagenicity of the test compound was admitted, then the actual test for mutagenicity was carried out, using the dose capable of giving an accurate dose-reaction curve. When no mutagenicity of the test compound was admitted in the dose determination test and when the growth inhibition of the test cells was admitted therein, then the dose to present the growth inhibition was taken as the maximum dose; and when the growth inhibition was not admitted, then a dose of 5000 μg/plate was taken as the maximum dose. With that, the actual test for mutagenicity was carried out on the level of 6 doses (common ratio 2).

First, a test compound was dissolved or suspended in DMSO, then diluted in order to prepare test compound liquids having a varying concentration.
100 μl of a test compound liquid or a negative control (DMSO) solution was put into a sterilized test tube. Then, for the case in the absence of a metabolic activation system (-S9mix), 500 μl of 0.1 mol/liter sodium-phosphate buffer (pH 7.4) was added to it; and for the case in the presence of a metabolic activation system (+S9mix), 500 μl of S9mix was added to it. Next, 100 μl of the test strain suspension that had been cultivated with shaking at 37˚C for 8 hours was added to it, and

preincubated in a shaking thermostat at 37˚C for 20 minutes. After shaken, 2 ml of top agar was added to it, and the contents were mixed.
Next, the mixture was poured onto a minimal glucose agar plate medium (plate) and spread uniformly thereon, then the top agar was solidified, and the plate was transferred into a thermostat and incubated at 37˚C for 48 hours.
After the incubation, the growing condition of the test cells on the plate was observed with a stereoscopic microscope, and the deposition condition of the test substance was observed with the naked eye. Then, the number of the colonies grown through reverse mutation was counted.
For counting it, used as a colony analyzer. After area correction and miss-counting correction, the number of the colonies was computed. When the colony analyzer could not be used owing to the deposition of the test compound or the cell growth inhibition, then the number of the colonies was counted with the naked eye.
In case where the number of the colonies through reverse mutation increased two times or more that of the negative control and where the dose dependency or the reproducibility of the increase was admitted, then the tested sample was decided as positive. When the sample was decided as positive, then the relative activity that indicates the relative comparative value of the intensity of the mutagenicity of the test compound was obtained according to the following formula:

Relative Activity
= {(number of colonies per plate having the concentration)
- (number of colonies per negative control plate)}/the
value of concentration (mg/plate).

According to the process mentioned above, the reverse mutation test was carried out. The following Table 5 shows the relative activity value that indicates the relative comparative value of the intensity of the mutagenicity of the test compound.
Irrespective of the absence or presence of S9mix, THK-097, THK-336, THK-525, THK-683, THK-702, THK-708, THK-711, THK-713, THK-727 and THK-752 did not increase the number of the colonies after reverse mutation, at least two times that of the negative control, and their relative activity was minus. In the presence of S9mix, the relative activity of THK-707, THK-761 and THK-763 was weak; but on the other hand, in the presence of S9mix, the relative activity of FDDNP (Agdeppa et al., Journal of Neuroscience, Vol. 21, page RC189, 2001) and IMPY (Kung et al., Brain Research, Vol. 956, page 202, 2002) was extremely high. The results in Table 5 confirm that the compounds of the invention tested in this experiment did not have mutagenicity or, even though they had it, their relative activity was extremely weak as compared with that of FDDNP and IMPY.
[Table 5]

Table 5 - Mutagenicity Test of Compounds of the Invention

| | Relative Activity | |
|---|---|---|
| | in the presence of S9Mix | in the absence of S9Mix |
| THK-097 | minus[1)] | minus |
| THK-336 | minus | minus |
| THK-525 | minus | minus |
| THK-683 | minus | minus |
| THK-702 | minus | minus |
| THK-707 | minus | 264 |
| THK-708 | minus | minus |
| THK-711 | minus | minus |
| THK-713 | minus | minus |

(continued)

| | Relative Activity | |
|---|---|---|
| | in the presence of S9Mix | in the absence of S9Mix |
| THK-727 | minus | minus |
| THK-752 | minus | minus |
| THK-761 | minus | 11995 |
| THK-763 | minus | 3058 |
| FDDNP2) | minus | 3,564,960 |
| IMPY2) | minus | 3,326,100 |
| 1) The number of colonies after reverse mutation did not increase at least two times that of the negative control, 2) The data of FDDNP and IMPY were from PCT/JP03/07183 (WO03/106439). | | |

Fluorescence Congo Red Method:

Next described is a screening method for the compounds of the invention. Some of the compounds of the invention could not be screened according to a Thioflavin T method, since their fluorescence wavelength overlaps with that of Thioflavin T. For such compounds, the following novel screening method was introduced.

(1) Amyloid β protein 1-40 (bought from Peptide Laboratory) was dissolved in a phosphate buffer (pH 7.4) and left at 37˚C for 4 days.
(2) 50 μl of Congo red dissolved in the buffer was applied to a 96-well microplate (final concentration, 0.1, 0.3, 1 μM).
(3) 100 μl of amyloid β protein was added to it (final concentration 5 μM), and left as such for 30 minutes.
(4) 100 μl of a test compound dissolved in the buffer was added to it (final concentration, 10 μM, and left as such for 60 minutes.
(5) Using a fluorescence microplate reader (Spectra Max 190 by Molecular Device), the sample was analyzed at the optimum exciting wavelength and the test wavelength that had been previously determined.
(6) The fluorescence intensity in the presence of the test compound, amyloid β protein and Congo red is represented by A; that in the presence of the test compound and Congo red is by B; that in the presence of the test compound and amyloid P protein is by C; and that in the presence of only the test compound is by D. The β structure recognition of the test compound is computed according to the following formula:

$$\beta \text{ structure recognition (\%) of test compound} = \{(A-B)/(C-D)\} \times 100.$$

(7) It may be said that the test compound having a higher β structure recognition percentage may have higher binding specificity to amyloid β protein.

Table 6 shows the results. The binding of the test compound to Aβ was concentration-dependently inhibited by Congo red that specifically binds to Aβ. The above clarifies that the test compounds recognized the β structure of Aβ. [Table 6]

Table 6 - Binding of Compound of the Invention to Aβ (fluorescence Congo red method)

| Compound (10 μM) | Binding Percentage of Compound in the presence of Congo red (CR) | | |
|---|---|---|---|
| | in the presence of 0.1 μM CR | in the presence of 0.3 μM CR | in the presence of 1 μM CR |
| THK-525 | 61.4 | 24.1 | -6.4 |
| THK-702 | 74.9 | 37.3 | 0.8 |

(continued)

| Compound (10 μM) | Binding Percentage of Compound in the presence of Congo red (CR) | | |
|---|---|---|---|
| | in the presence of 0.1 μM CR | in the presence of 0.3 μM CR | in the presence of 1 μM CR |
| THK-707 | 66.8 | 36.4 | 29.7 |
| THK-708 | 66.5 | 29.1 | 11.0 |
| THK-711 | 60.4 | 29.1 | 10.3 |
| THK-713 | 58.2 | 21.2 | 3.0 |
| THK-727 | 73.6 | 35.0 | 3.1 |
| THK-752 | 50.4 | 20.6 | 11.3 |
| THK-757 | 52.4 | 24.8 | 10.3 |
| THK-761 | 55.2 | 21.6 | 7.0 |
| THK-763 | 54.7 | 22.9 | 7.5 |
| THK-765 | 71.2 | 41.3 | 23.0 |
| THK-766 | 61.0 | 21.5 | 11.2 |
| THK-767 | 60.0 | 29.9 | 15.9 |
| Thioflavin T | 49.4 | 22.3 | 9.3 |

INDUSTRIAL APPLICABILITY

The compounds of the invention for diagnostic probe for conformation disease, especially those for imaging diagnostic probe, as well as the medical composition for treatment and/or prevention of conformation disease comprising the compound are extremely useful for early detection, treatment and prevention of conformation disease such as Alzheimer disease that is at present considered as a most intractable disease; and they are applicable to the field of production of diagnostic drugs and kits for conformation disease, to the field of production of remedial medicines and preventive medicines for conformation disease, and to studies of conformation disease.

## Claims

**1.** A compound of a formula (I), or a salt or a solvate thereof:

[Formula 1]

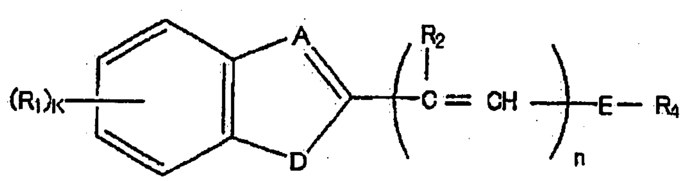

(I)

[wherein A represents CH or N;
D represents S, NH, N-$C_{1-3}$ alkyl, O or $CH_2$;
$R_1$ each independently represents hydrogen, halogen, OH, COOH, $SO_3H$, $NO_2$, SH, $NR^aR^b$, $C_{1-6}$ alkyl, O-$C_{1-6}$ alkyl, CN, C=O, O-$(CH_2)_1$-OTs , or O-$(CH_2)_m$-$CHR^cR^d$, or

[Formula 2]

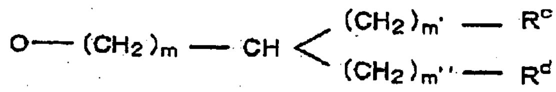

;

adjacent $R_1$'$_S$, taken together, may form a phenyl ring;

$R_2$ represents hydrogen, $C_{1-4}$ alkyl, $C_{1-4}$ alkyl-halogen, OH or CN;

$R^4$ represents hydrogen, halogen, OH, COOH, $SO_3H$, $NO_2$, SH, $NR^5R^6$, $C_{1-6}$ alkyl, O-$C_{1-6}$ alkyl, CN, C=O, pyrrolidine ring, pyrrole, pyrazole, imidazole, triazole, or

[Formula 3]

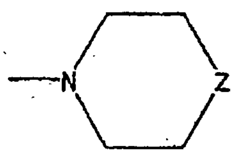 , 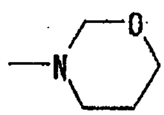 , 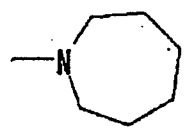 or 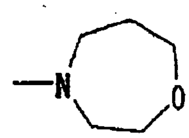 ;

$R^a$ and $R^b$ each independently represent hydrogen or $C_{1-4}$ alkyl;

$R^c$ and $R^d$ each independently represent hydrogen, halogen, OH, COOH, $SO_3H$, $NO_2$, SH, $NR^aR^b$, $C_{1-6}$ alkyl, O-$C_{1-6}$ alkyl, CN, C=O or OTs;

$R^5$ and $R^6$ each independently represent hydrogen or $C_{1-4}$ alkyl;

k indicates an integer of from 1 to 4;

l indicates an integer of from 1 to 4;

m indicates an integer of from 0 to 3;

m' indicates an integer of from 0 to 3;

m" indicates an integer of from 0 to 3;

n indicates an integer of from 1 to 4;

E represents a benzene ring or

[Formula 4]

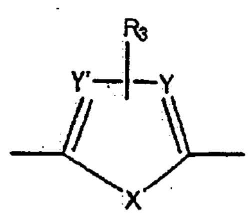

;

X represents CH, S, NH, N-$C_{1-3}$ alkyl or O;

Y represents CH or N;

Y' represents CH or N;

Z represents O, S, $CH_2$ or N-$R^e$;

$R_3$ is present when both or one of Y and Y' is CH, representing hydrogen, $C_{1-4}$ alkyl, OH or halogen;

$R^e$ represents hydrogen or $C_{1-4}$ alkyl;

the above alkyl may be substituted with halogen;

the configuration around the double bond that bonds two ring parts may be any of cis-form or trans-form].

**2.** The compound as claimed in claim 1, or the salt or the solvate thereof, which is selected from a group consisting of THK-097, THK-203, THK-207, THK-281, THK-525, THK-702, THK-708, THK-727, THK-752, THK-761, THK-763 and THK-766.

**3.** The compound as claimed in claim 1, or the salt or the solvate thereof, wherein $R^4$ is a morpholine ring.

**4.** The compound as claimed in claim 3, or the salt or the solvate thereof, which is selected from a group consisting of THK-097, THK-203, THK-525, THK-575, THK-702, THK-703, THK-726 and THK-727.

**5.** The compound as claimed in claim 1, or the salt or the solvate thereof, which is selected from a group consisting of THK-683, THK-707, THK-708, THK-711, THK-713, THK-752, THK-761 and THK-763.

**6.** The compound as claimed in any one of claims 1 to 5, or the salt or the solvate thereof, which is labeled.

**7.** The compound as claimed in claim 6, or the salt or the solvate thereof, which is radioactive-labeled.

**8.** The compound as claimed in claim 7, or the salt or the solvate thereof, which is labeled with a positron emitter.

**9.** A diagnostic composition for conformation disease, which comprises a compound of any one of claims 1 to 8, or the salt or the solvate thereof.

**10.** A medical composition for treatment and/or prevention of conformation disease, which comprises a compound of any one of claims 1 to 8, or the salt or the solvate thereof.

**11.** A diagnostic kit for conformation disease, which comprises, as the indispensable constitutive component thereof, a compound of any one of claims 1 to 8, or the salt or the solvate thereof.

**12.** A composition or kit for detecting or staining a β sheet structured protein or neurofibrillary tangle, which comprises, as the indispensable constitutive component thereof, a compound of any one of claims 1 to 8, or the salt or the solvate thereof.

**13.** The composition as claimed in claim 9 or the kit as claimed in claim 11 or 12, which is for imaging diagnosis.

**14.** A method for treatment and/or prevention of conformation disease of a subject, which comprises administering a compound of any one of claims 1 to 8, or the salt or the solvate thereof to the subject.

**15.** A method for diagnosis of conformation disease of a subject, which comprises administering a compound of any one of claims 1 to 8, or the salt or the solvate thereof to the subject.

**16.** Use of the compound of any one of claims 1 to 8, or the salt or the solvate thereof, for producing a composition or kit for diagnosis of conformation disease of a subject.

**17.** Use of the compound of any one of claims 1 to 8, or the salt or the solvate thereof, for producing a medical composition for treatment and/or prevention of conformation disease of a subject.

**18.** A method for detecting or staining a P sheet structured protein or neurofibrillary tangle in a sample, which comprises staining the sample with a compound of any one of claims 1 to 8, or the salt or the solvate thereof.

**19.** Use of the compound of any one'of claims 1 to 8, or the salt or the solvate thereof, for producing a composition or kit for detecting or staining a β sheet structured protein or neurofibrillary tangle in a sample.

**20.** The composition or kit as claimed in claim 12, the method as claimed in claim 18 or the use as claimed in claim 19, wherein the compound is THK-727.

Fig. 1

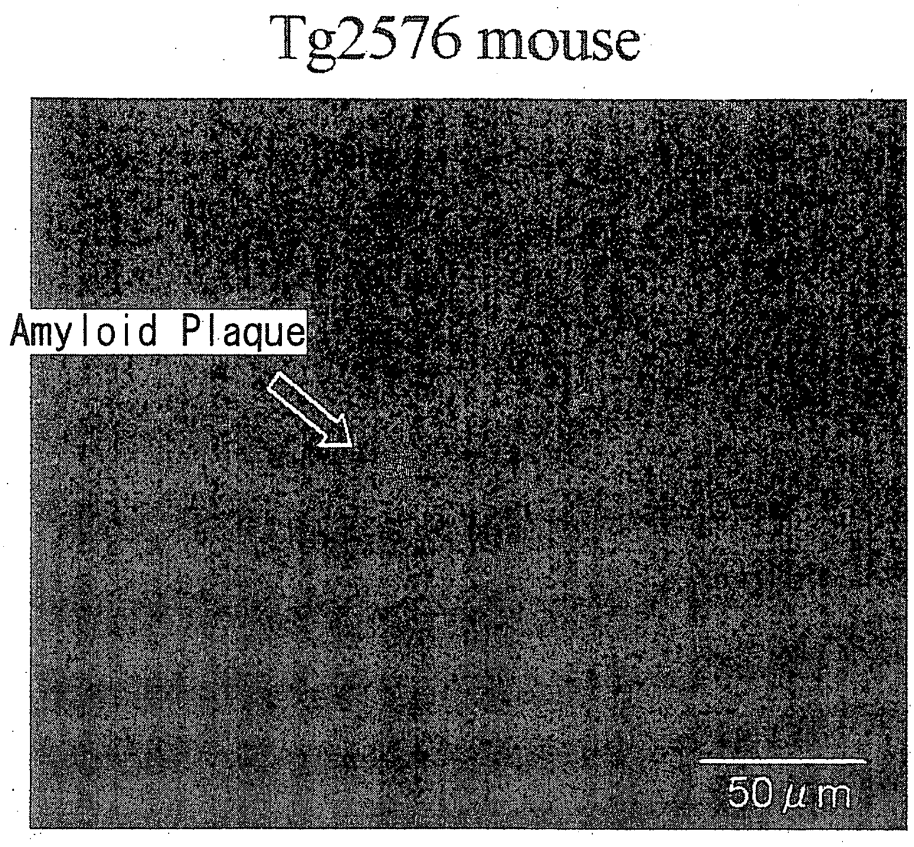
Tg2576 mouse
Amyloid Plaque
50 μm

Fig. 2

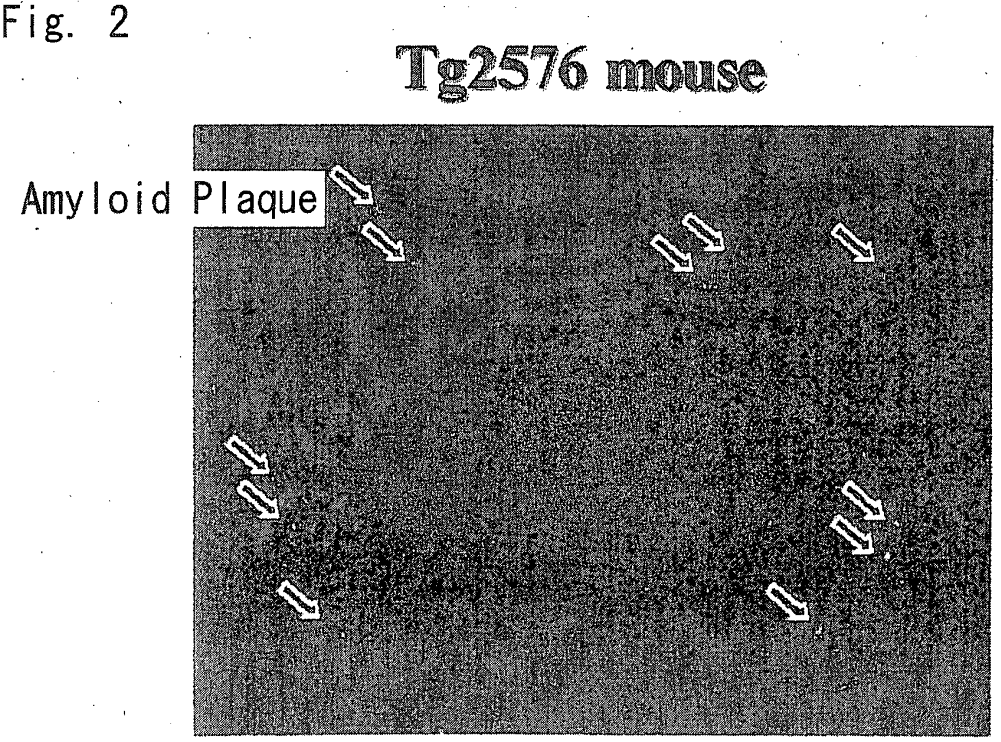
Tg2576 mouse
Amyloid Plaque

Fig. 3

APPswe2576 /Tau JPL3 mouse

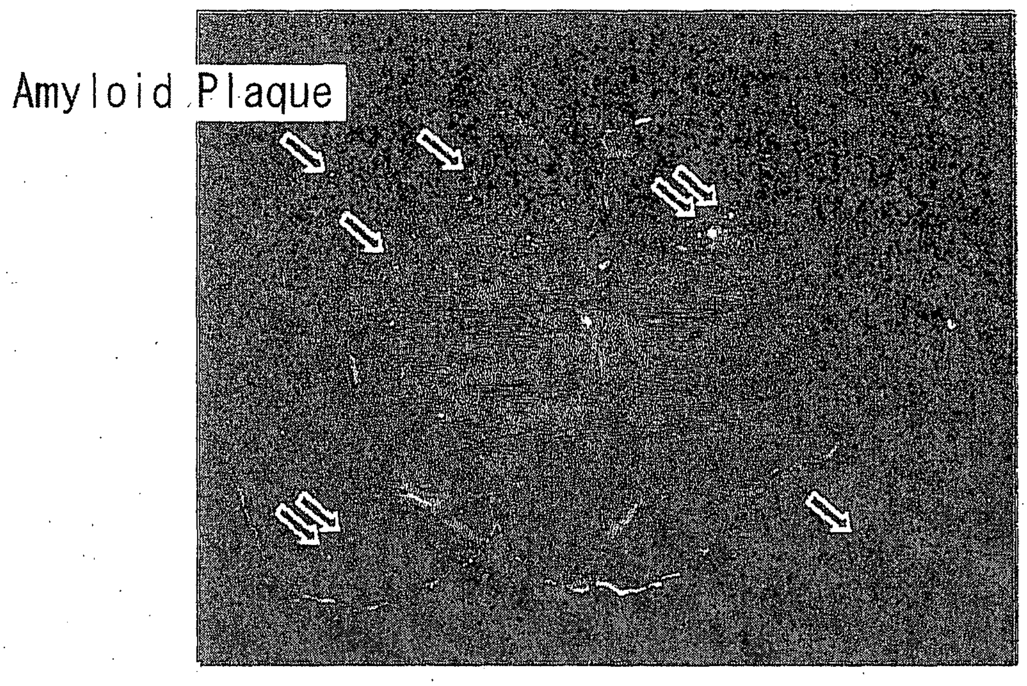

Fig. 4

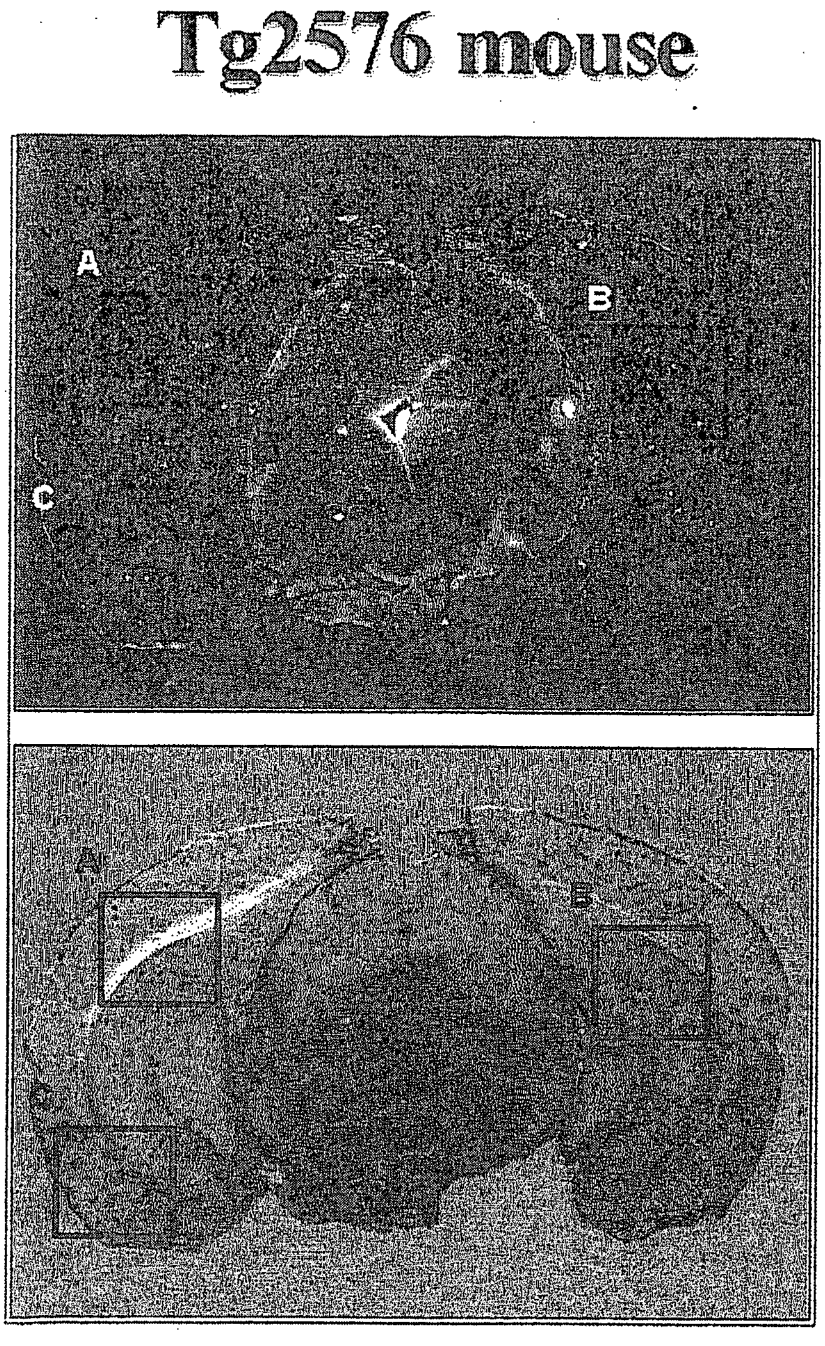

Fig. 5

THK-702 Anti-A$\beta$ Antibody Staining

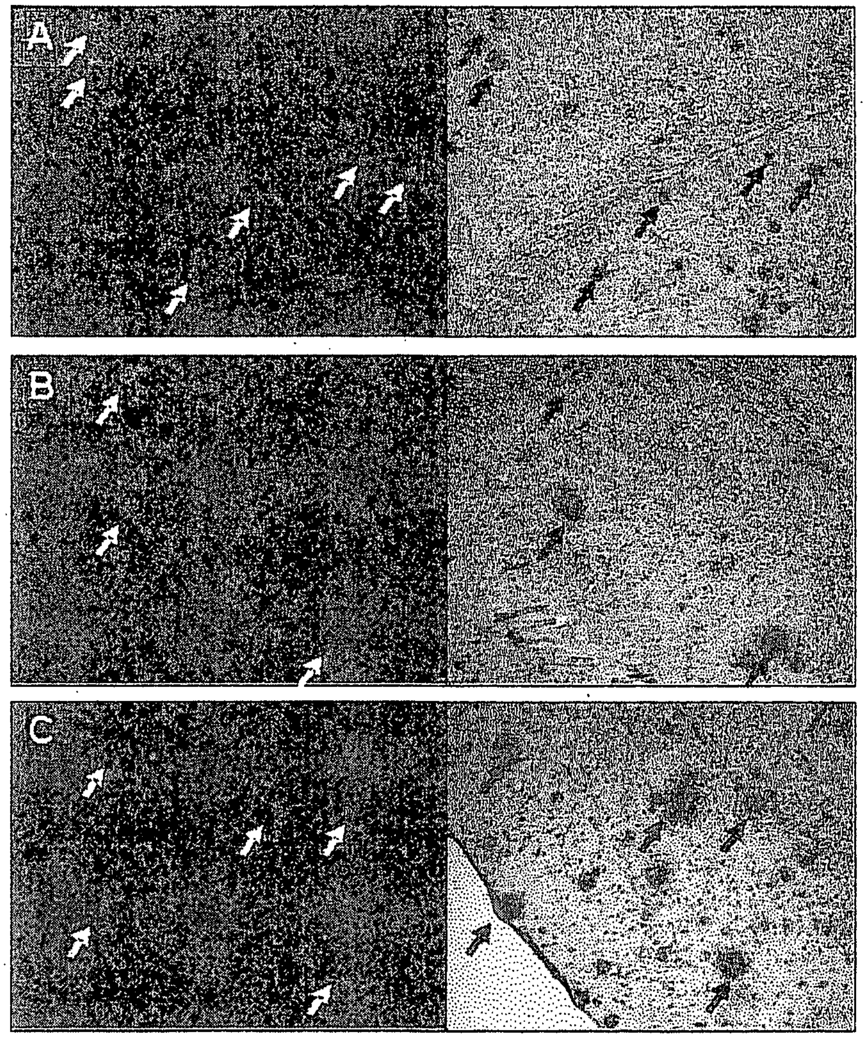

Fig. 6

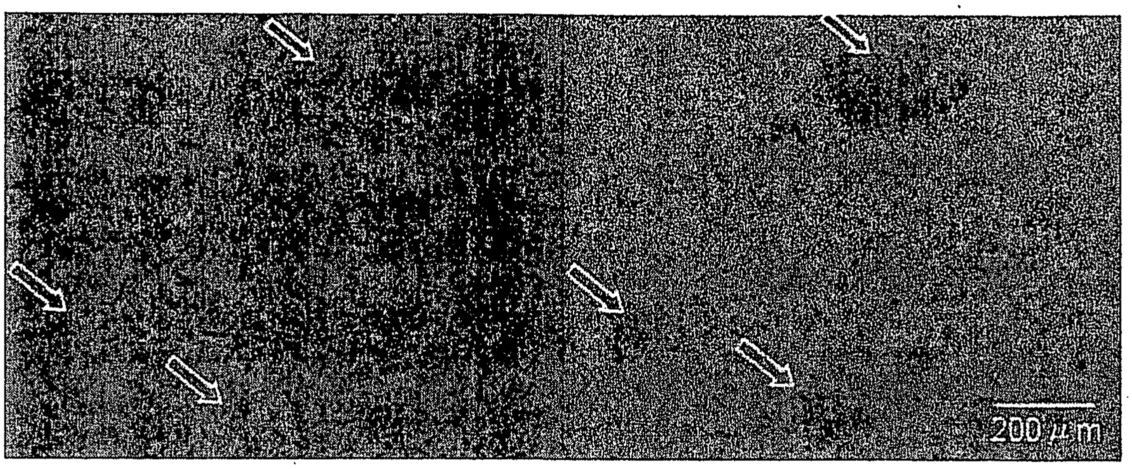


THK-097 Staining　Anti-Aβ Antibody Staining

Fig. 7

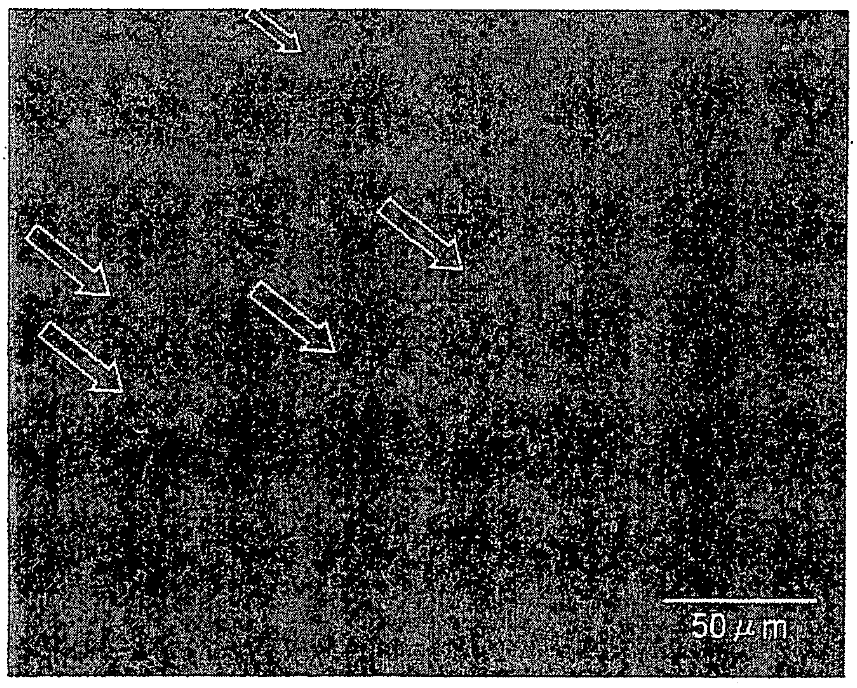


THK-184 Staining

Fig. 8

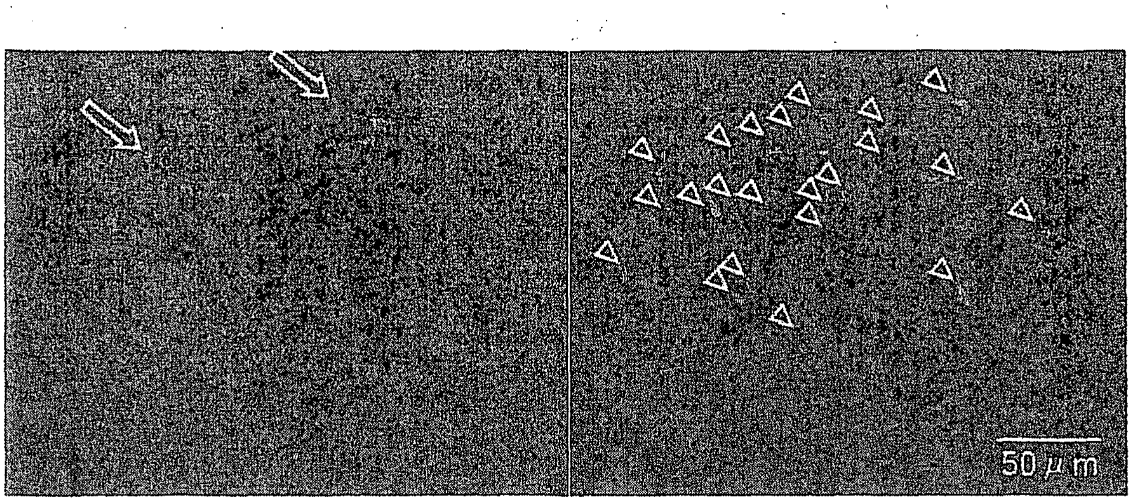


THK-185 Staining

Fig. 9

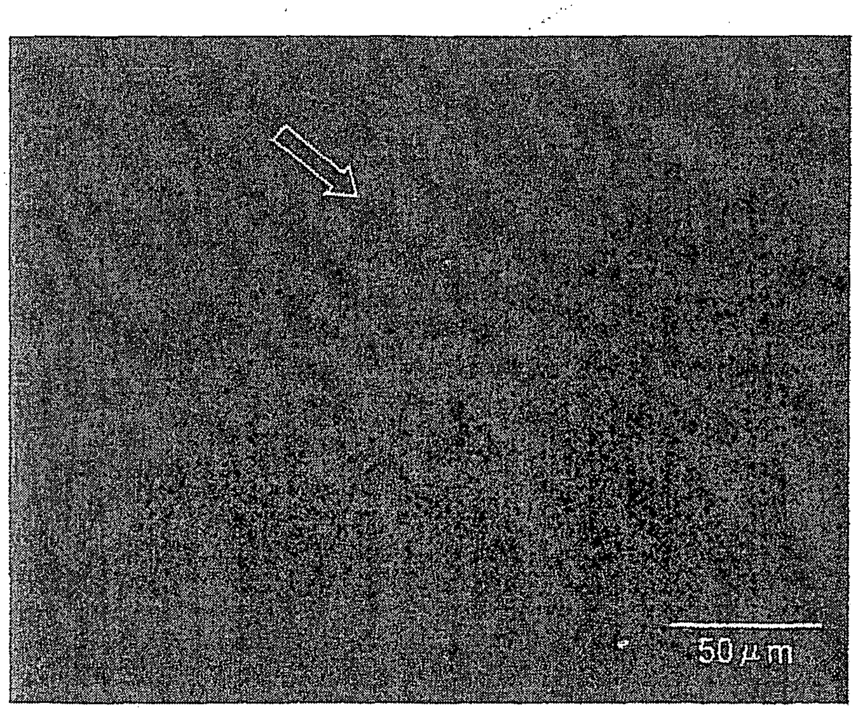


THK-203 Staining

Fig. 10

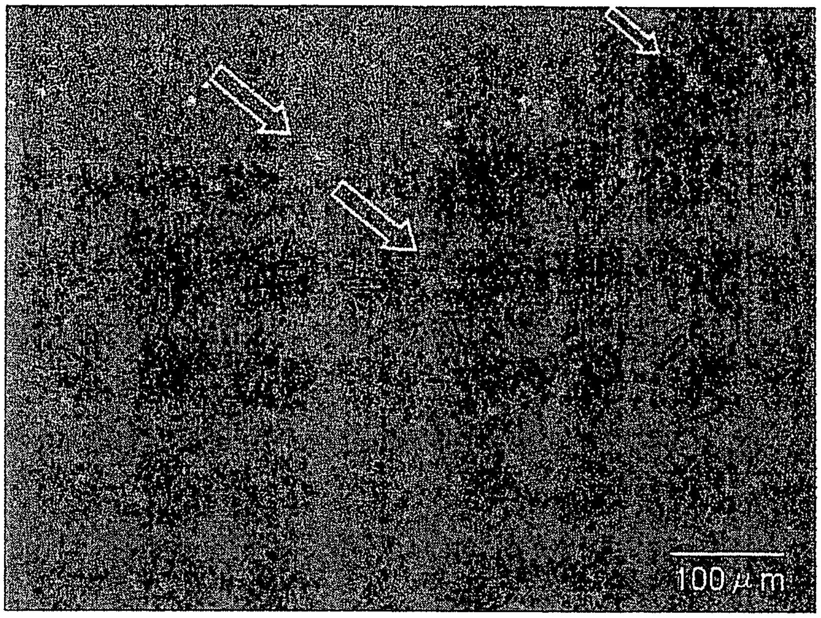


THK-207 Staining

Fig. 11

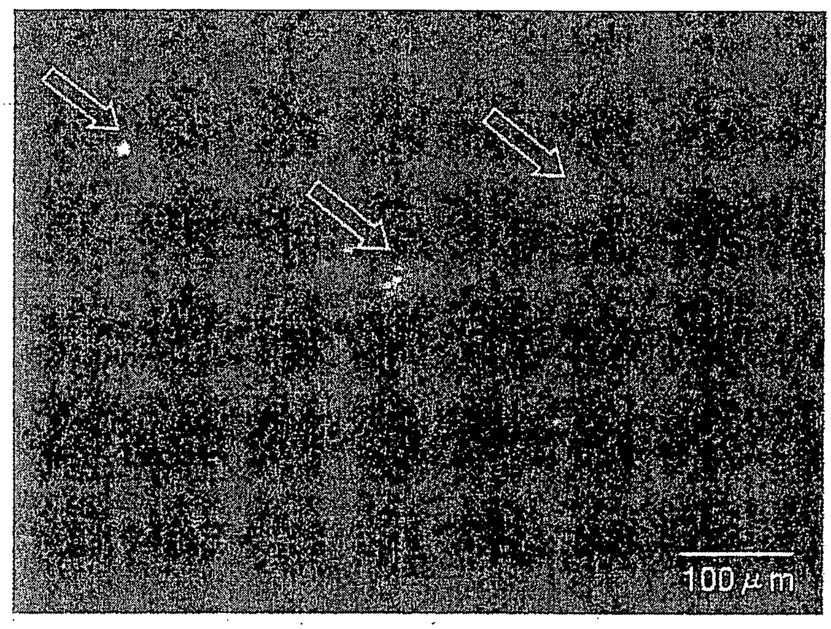


THK-248 Staining

Fig. 12

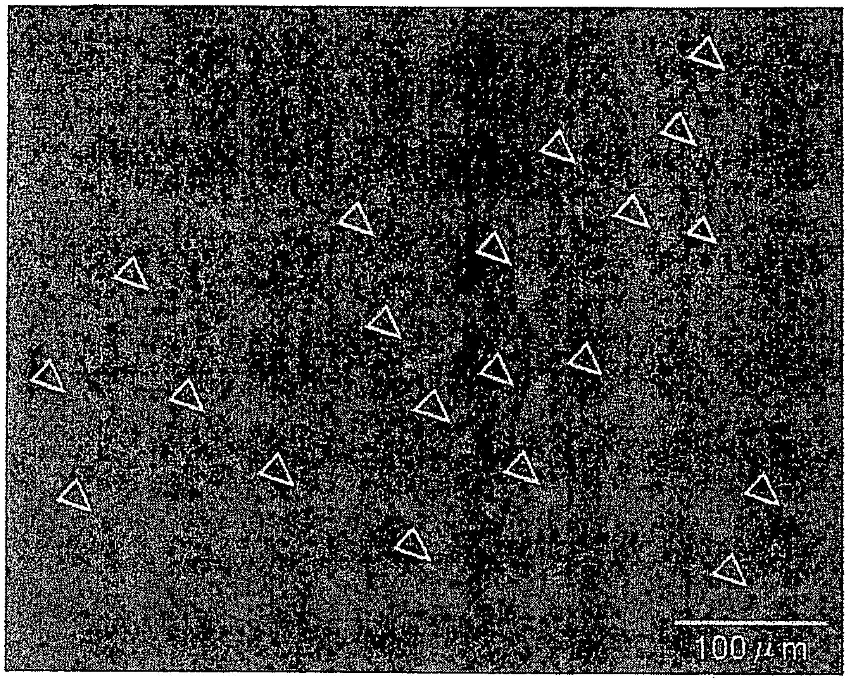


THK-254 Staining

Fig. 13

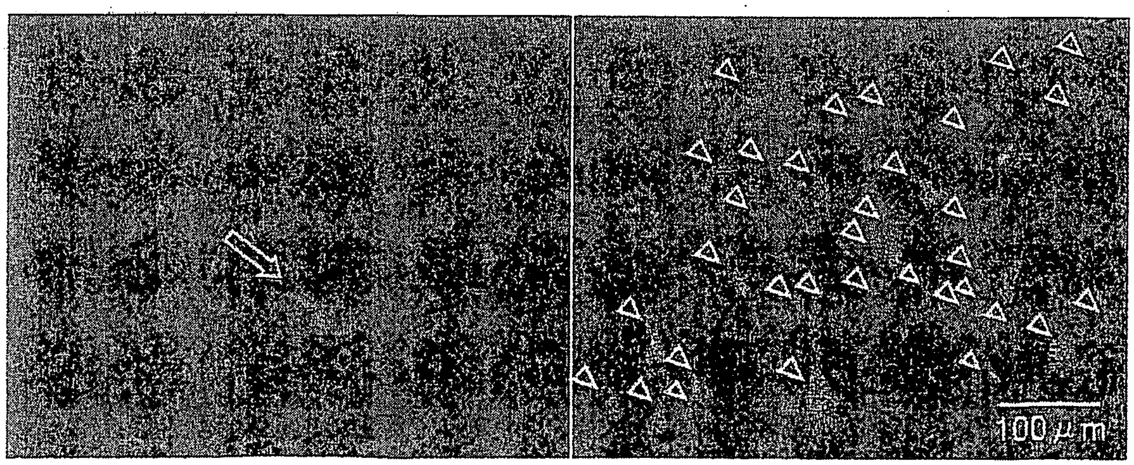


THK-258 Staining

Fig. 14

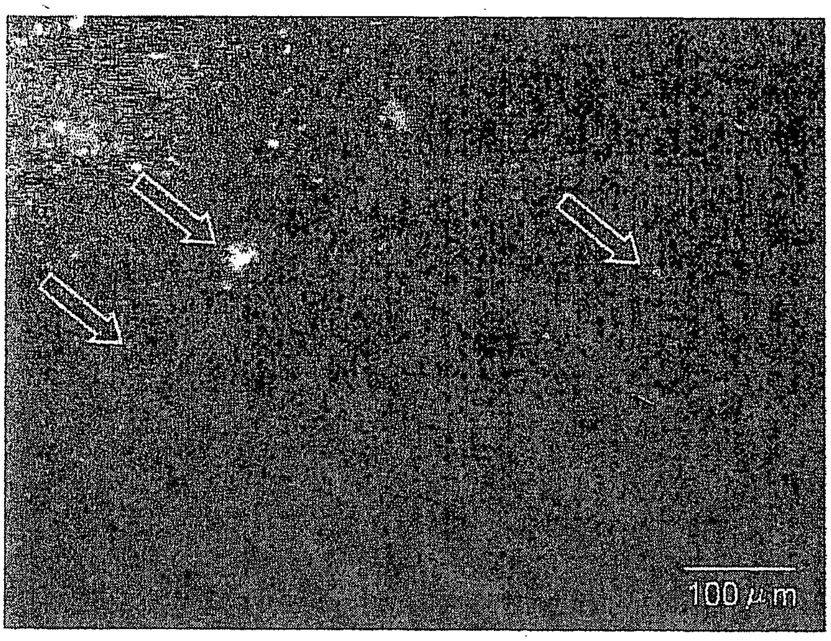


THK-262 Staining

Fig. 15

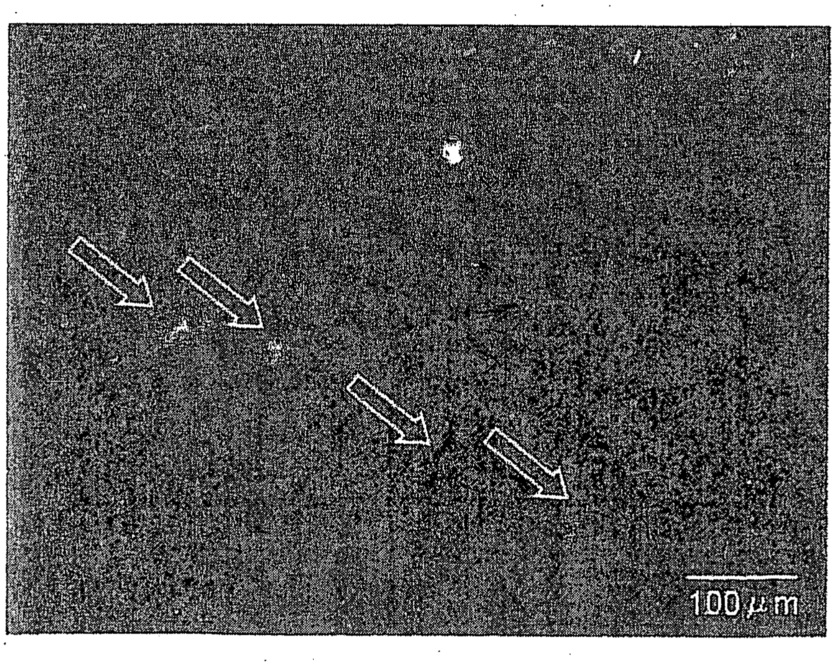


THK-276 Staining

Fig. 16

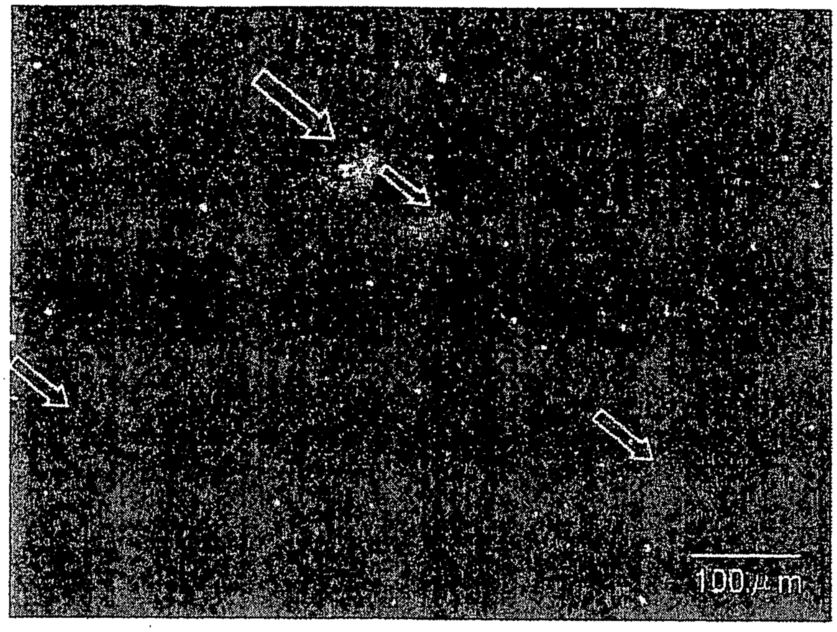


THK-281 Staining

Fig. 17

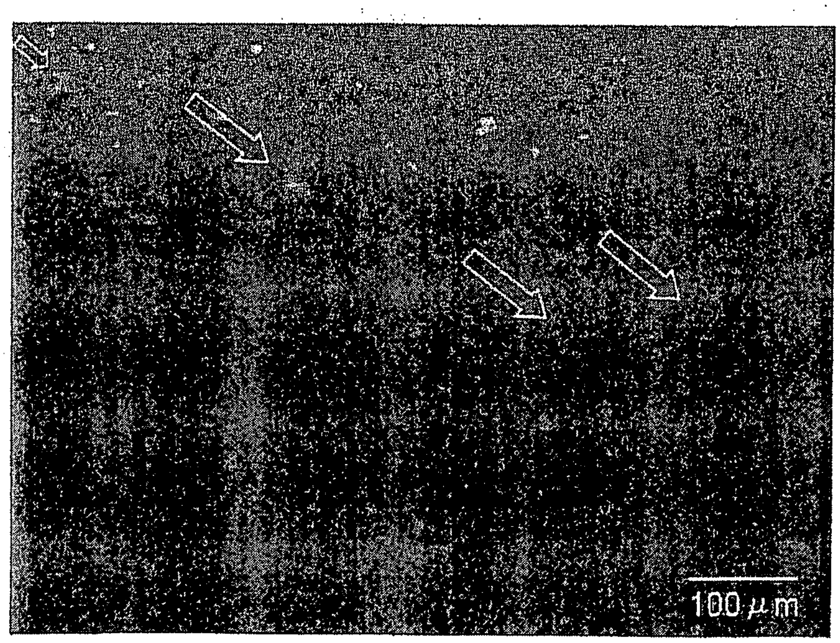


THK-308 Staining

Fig. 18

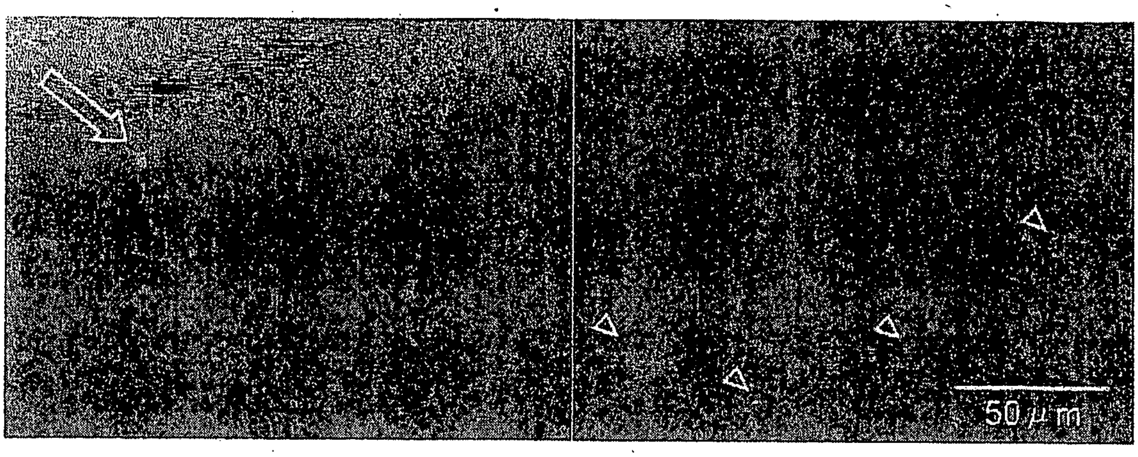


THK-317 Staining

Fig. 19

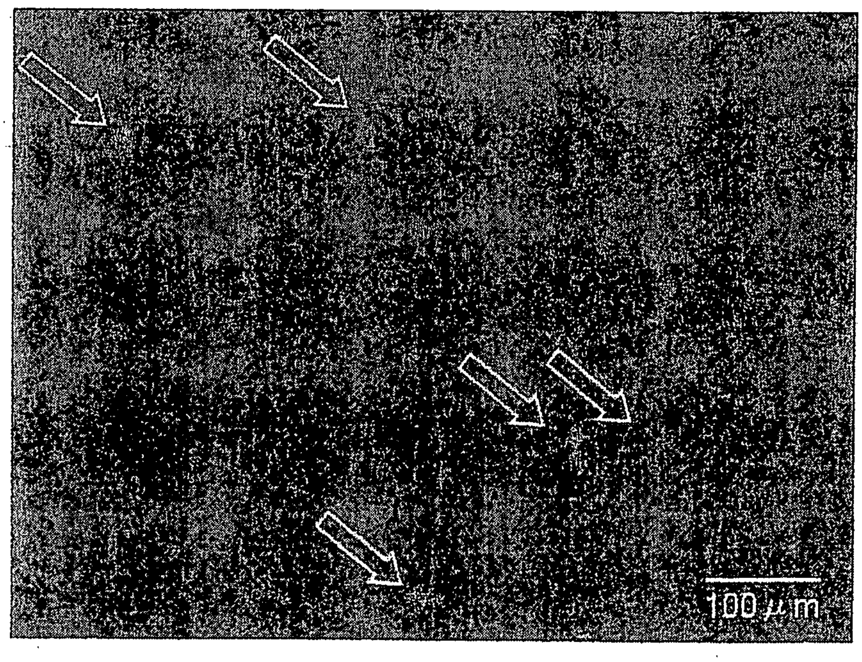


THK-383 Staining

Fig. 20

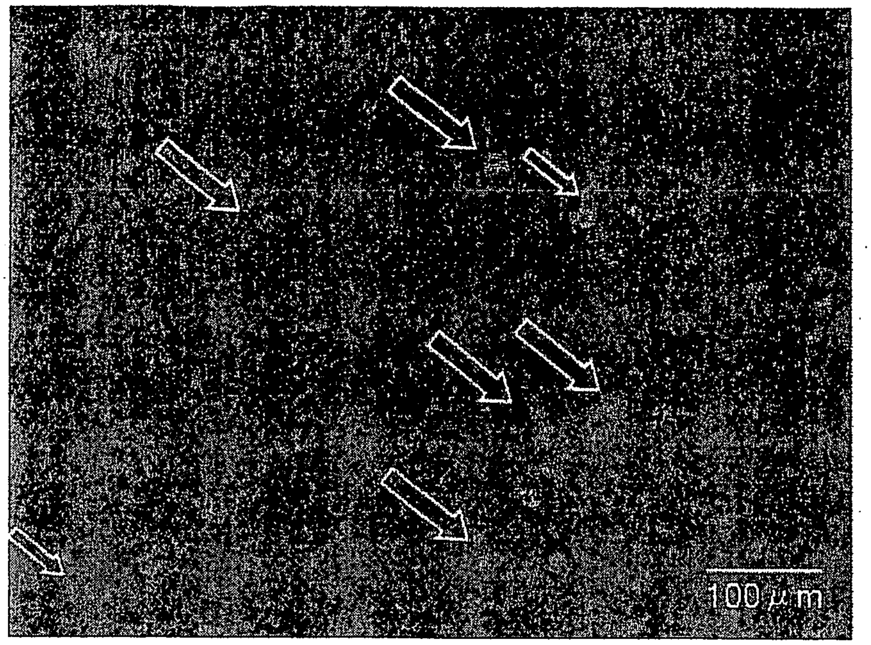


THK-385 Staining

Fig. 21

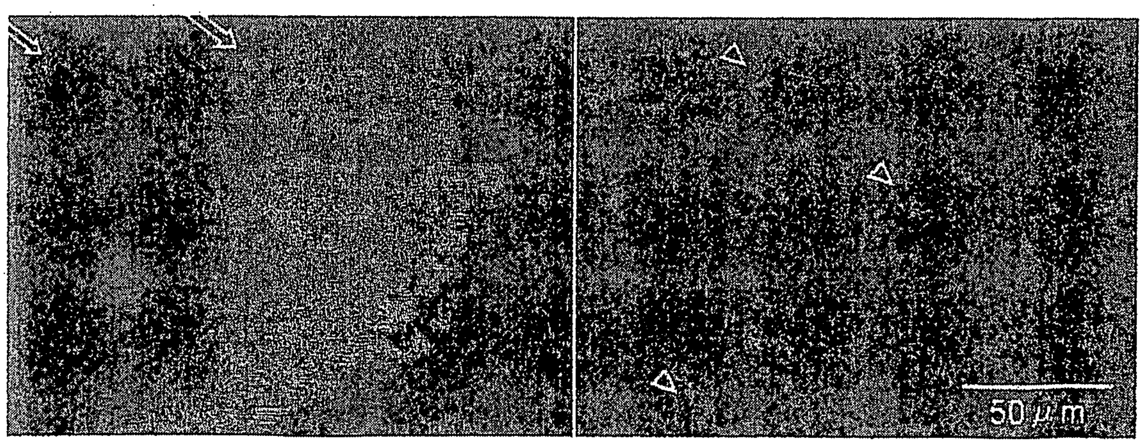


THK-386 Staining

Fig. 22

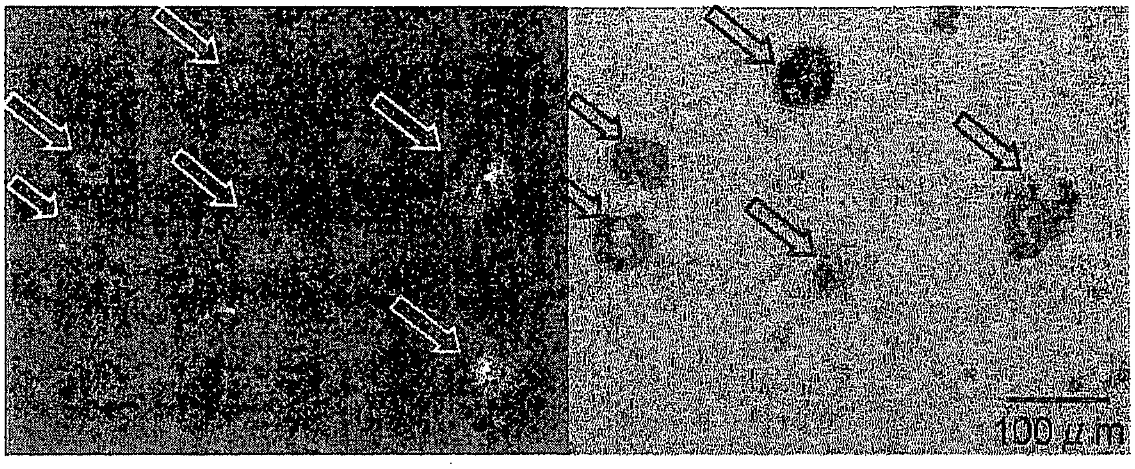


THK-525 Staining　　Anti-Aβ Antibody Staining

Fig. 23

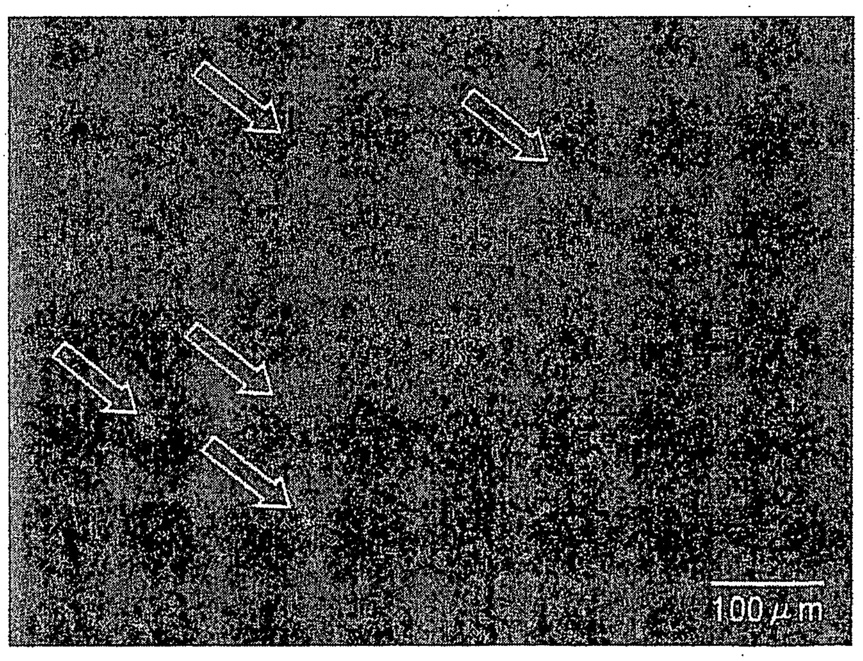


THK-556 Staining

Fig. 24

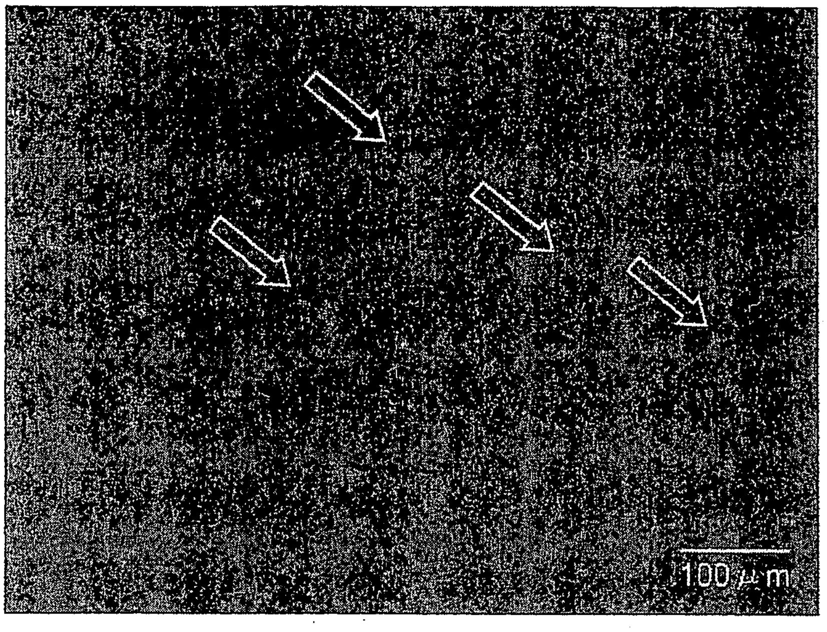


THK-558 Staining

Fig. 25

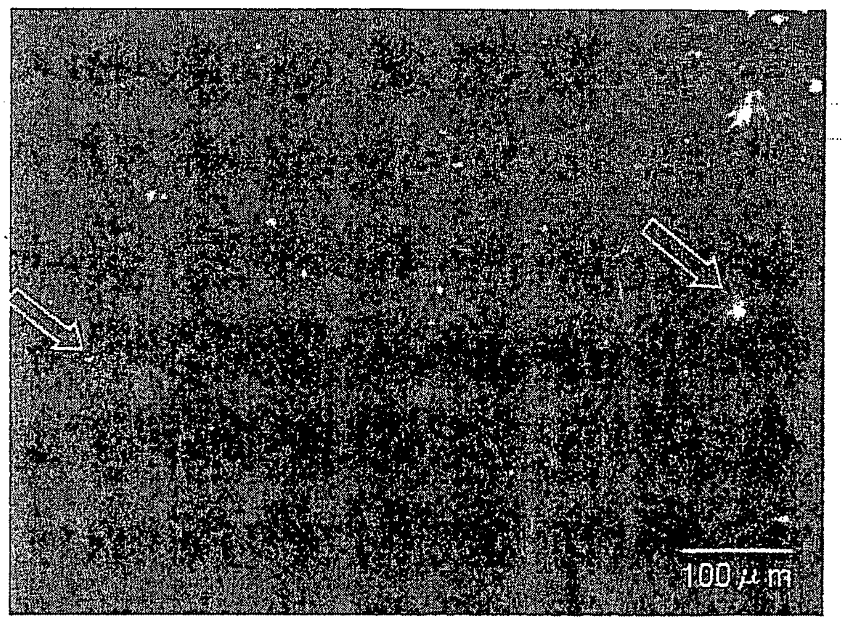


THK-559 Staining

Fig. 26

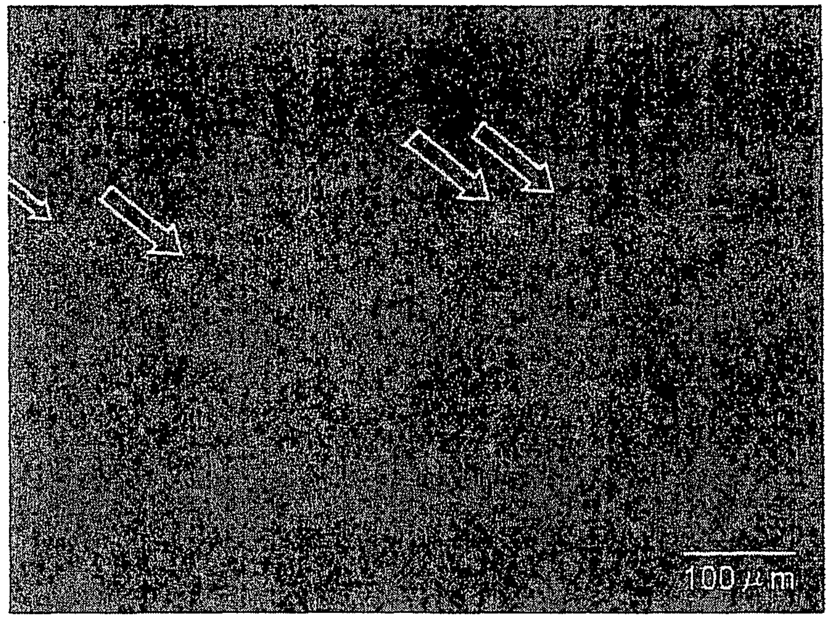


THK-561 Staining

Fig. 27

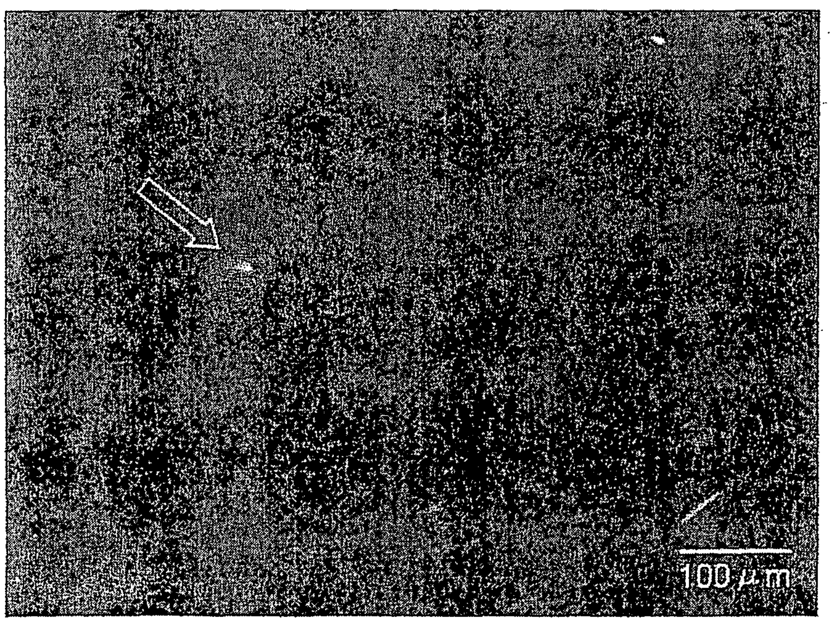


THK-562 Staining

Fig. 28

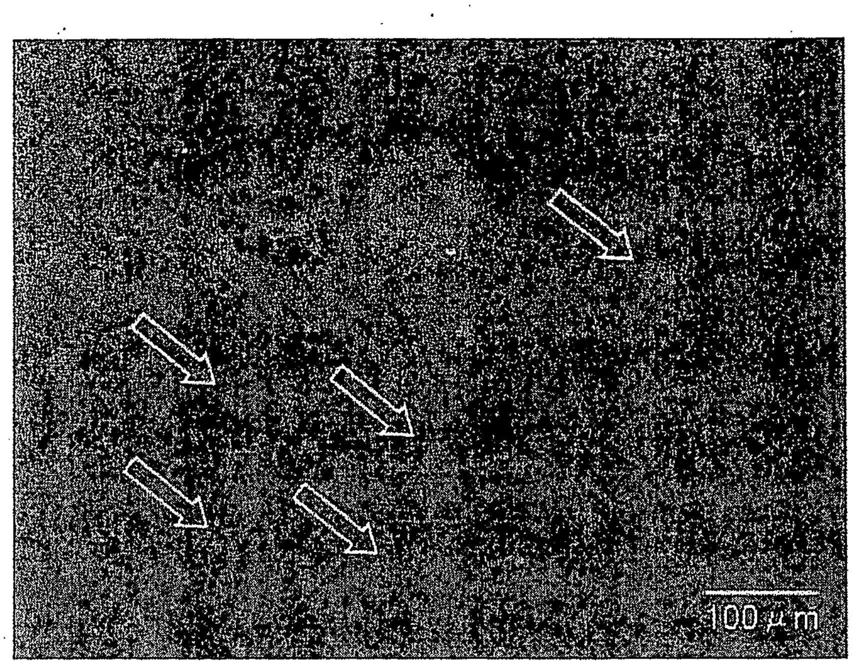


THK-563 Staining

Fig. 29

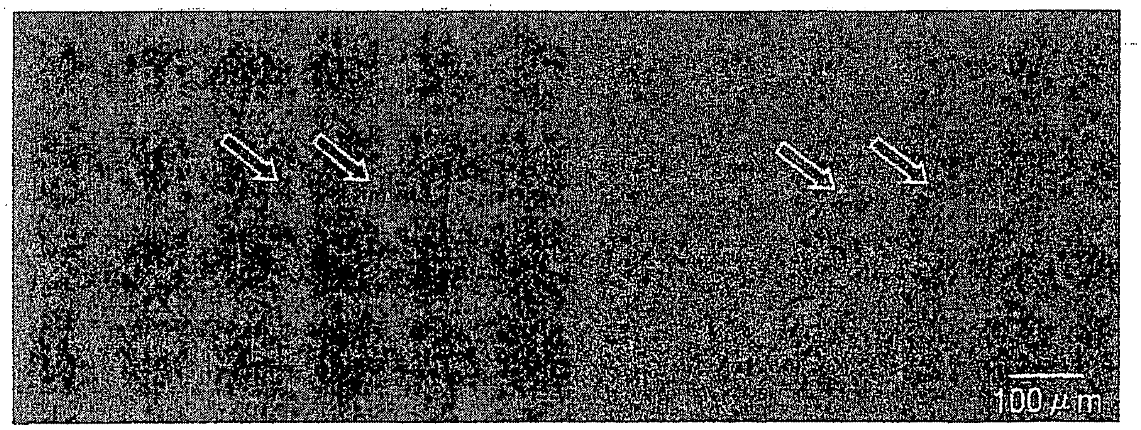


THK-565 Staining　　Anti-Aβ Antibody Staining

Fig. 30

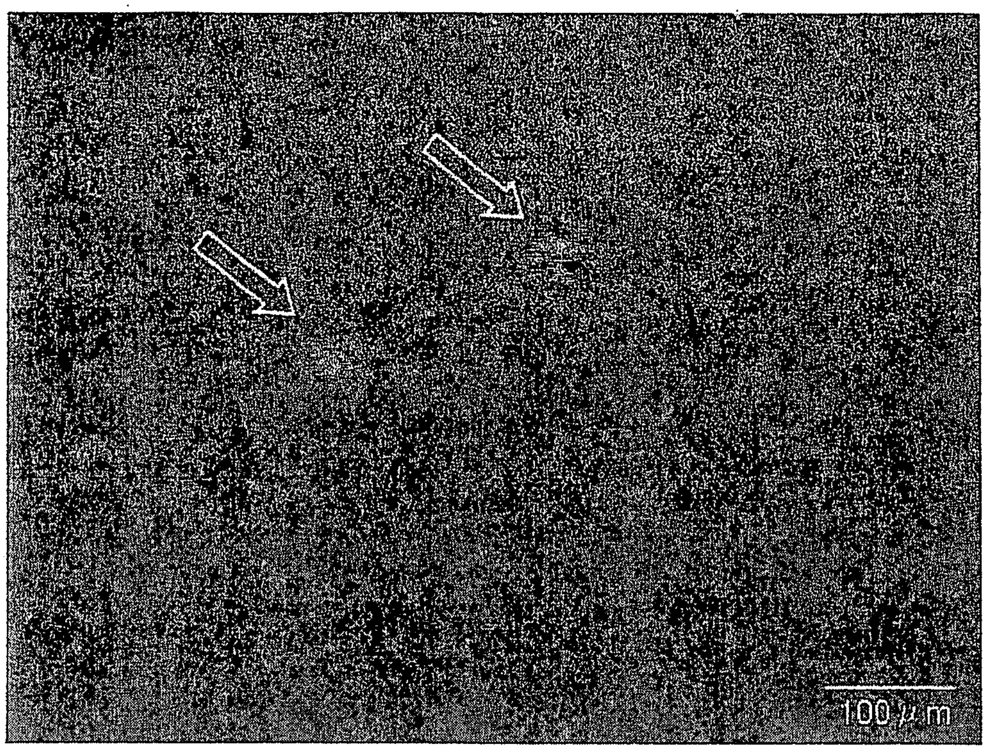


THK-585 Staining

Fig. 31

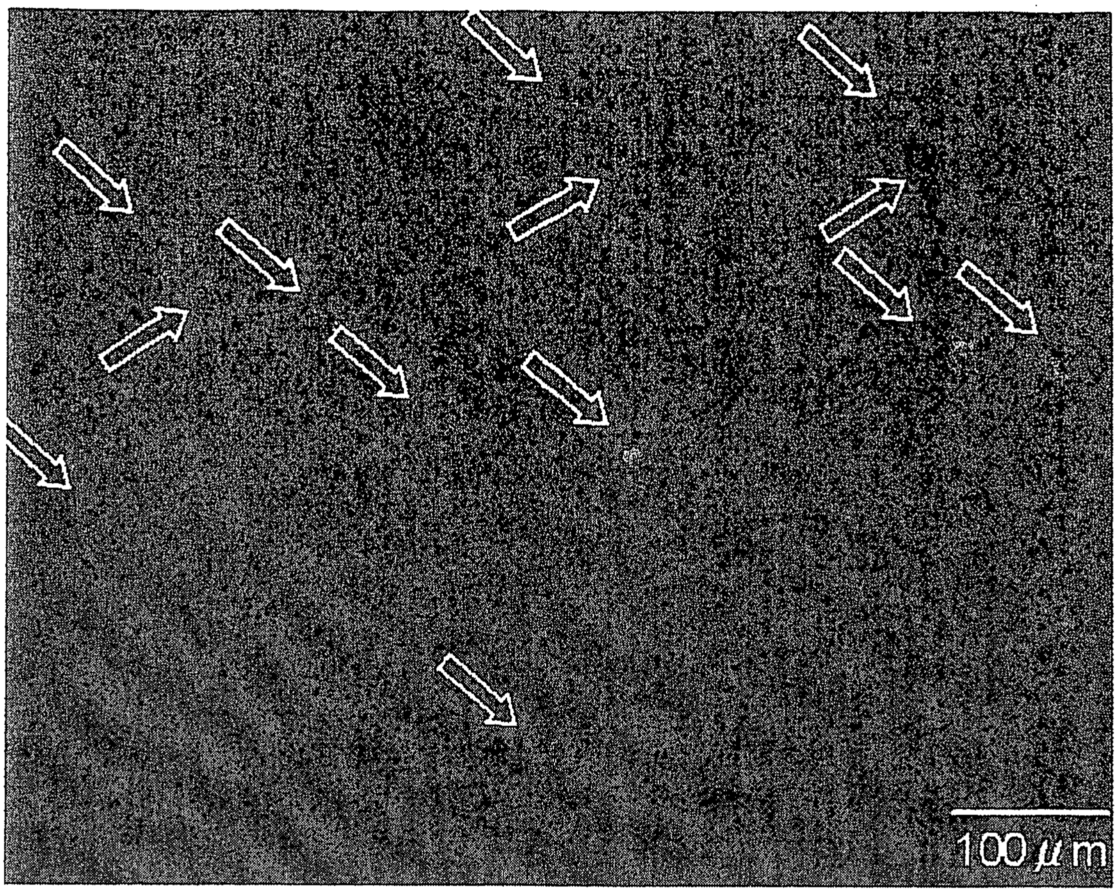


THK-702 Staining

Fig. 32

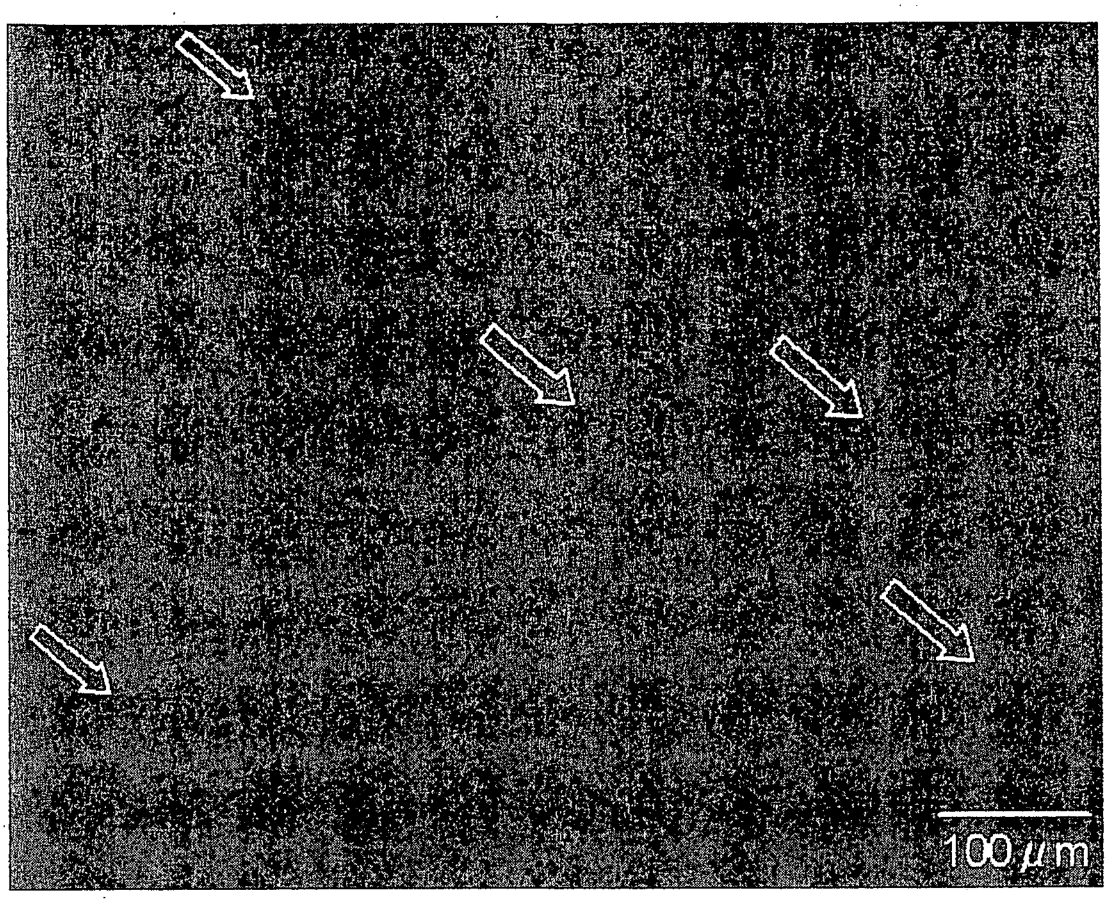


THK-708 Staining

Fig. 33

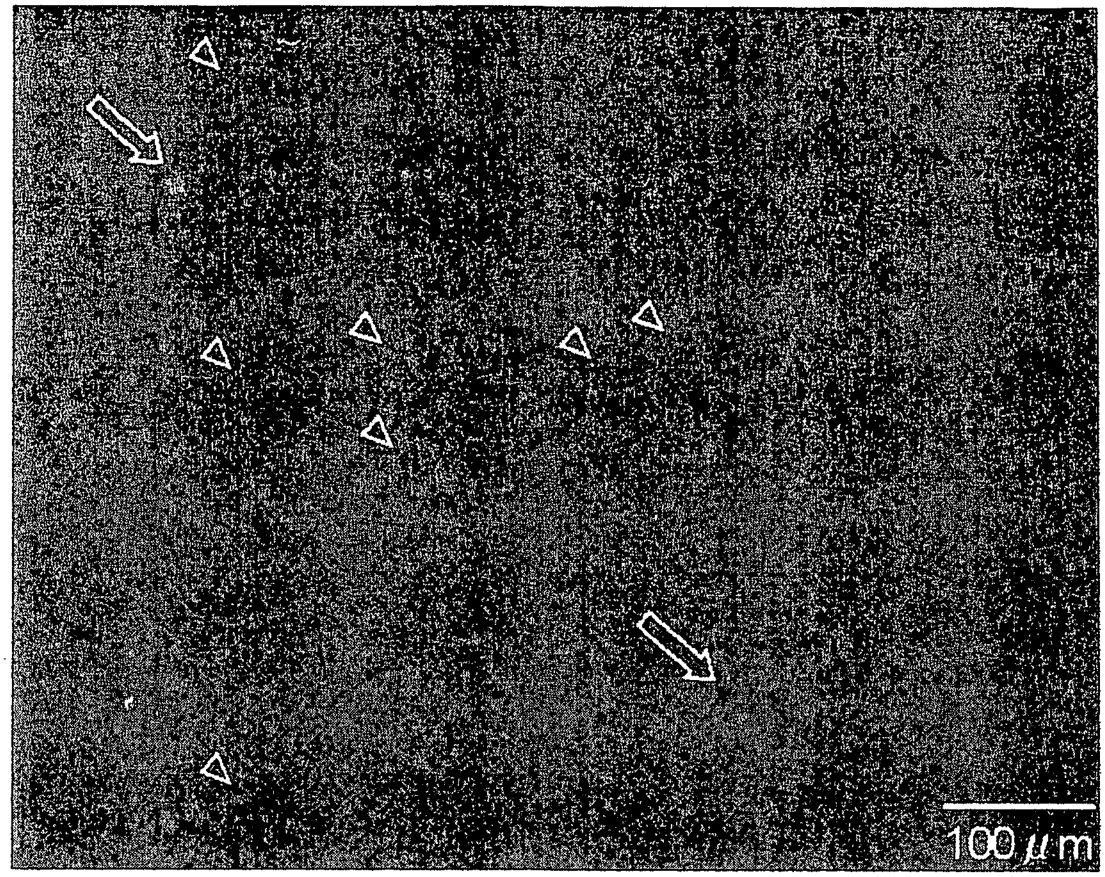


THK-727 Staining

Fig. 34

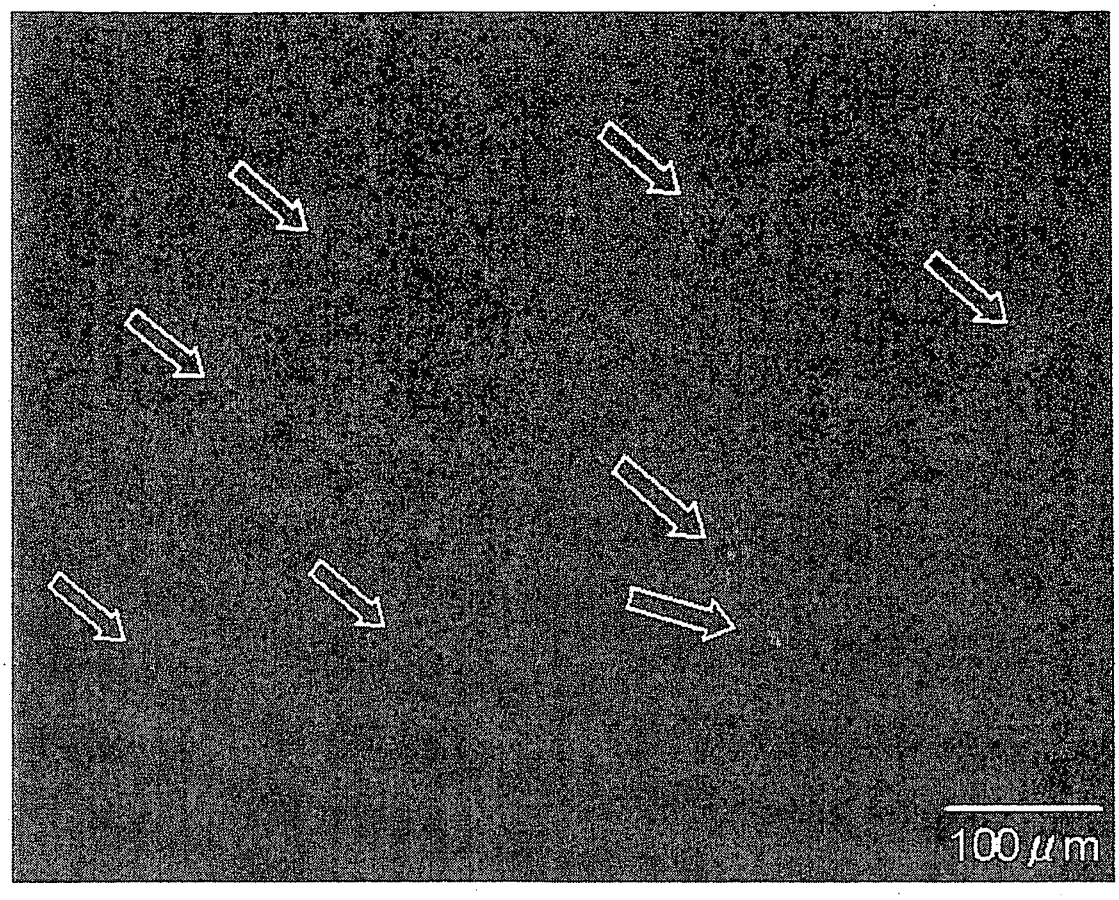


THK-752 Staining

Fig. 35

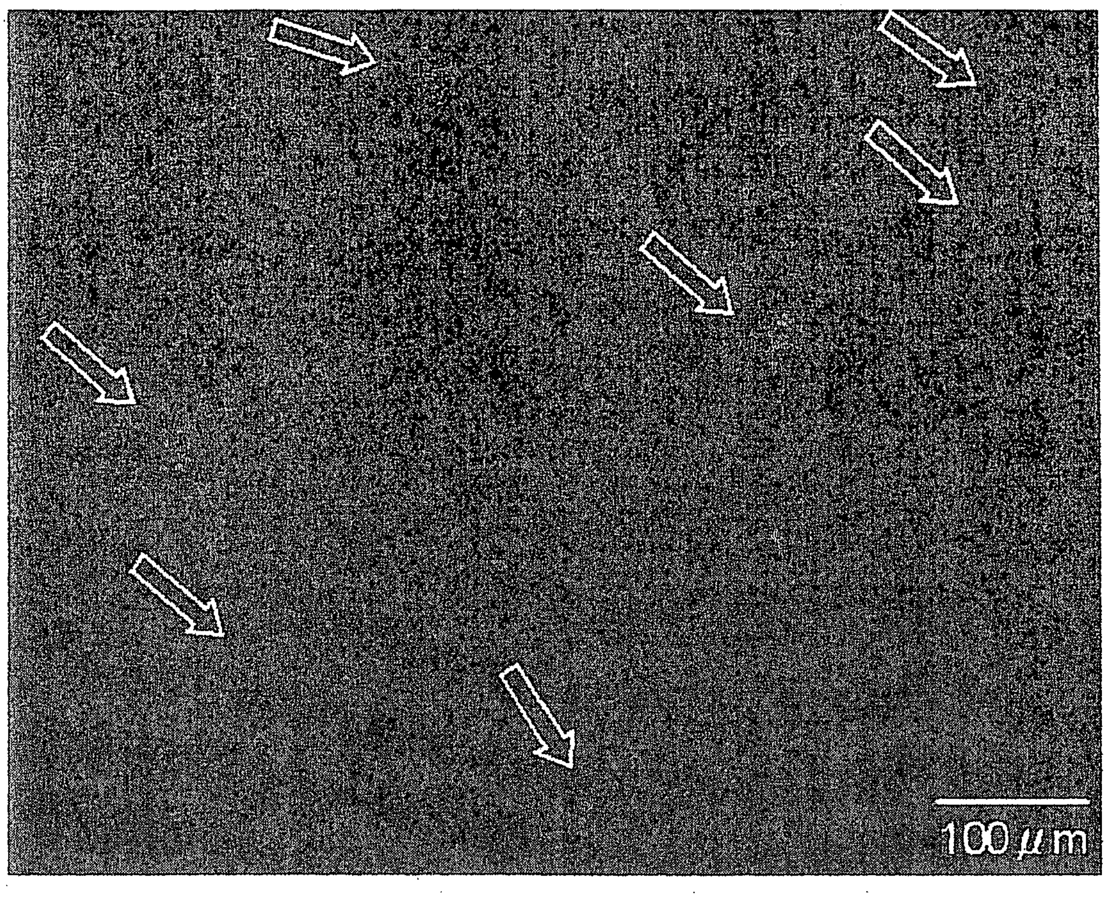


THK-761 Staining

Fig. 36

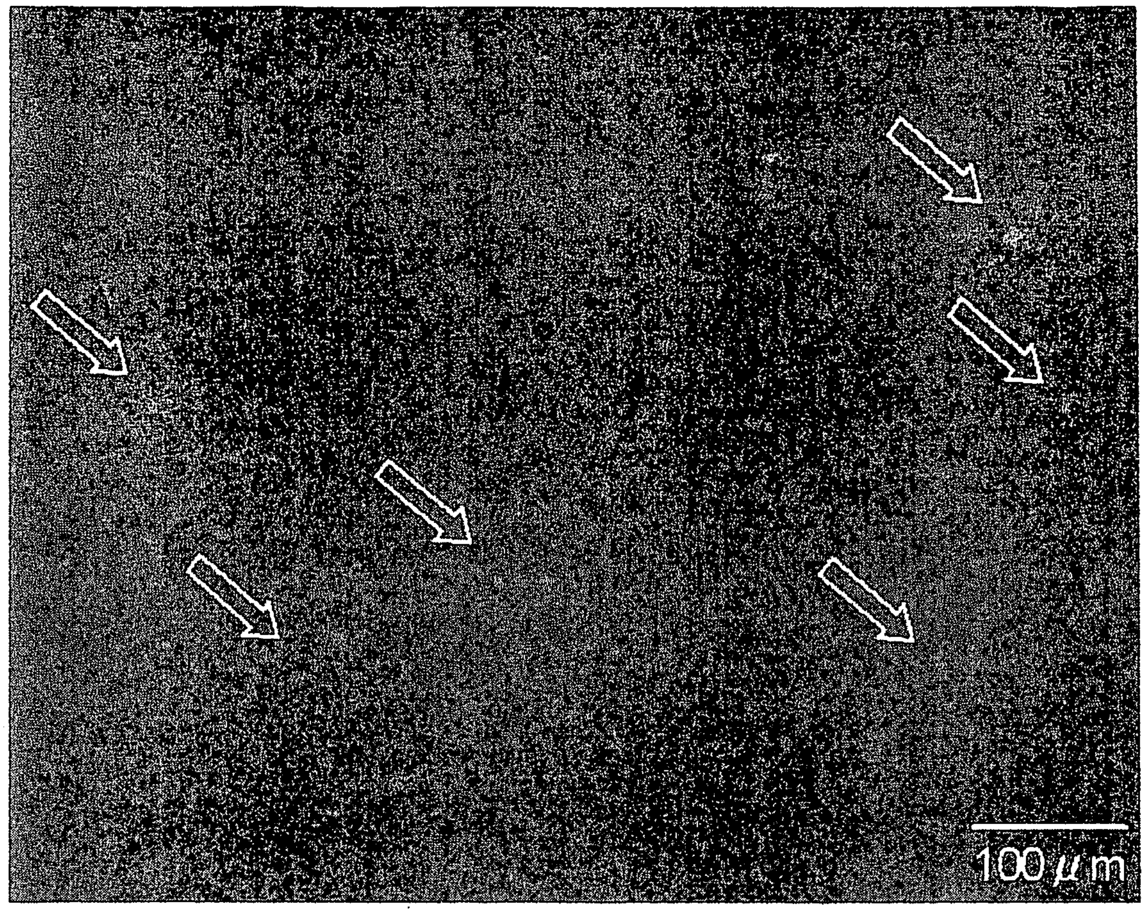


THK-763 Staining

Fig. 37

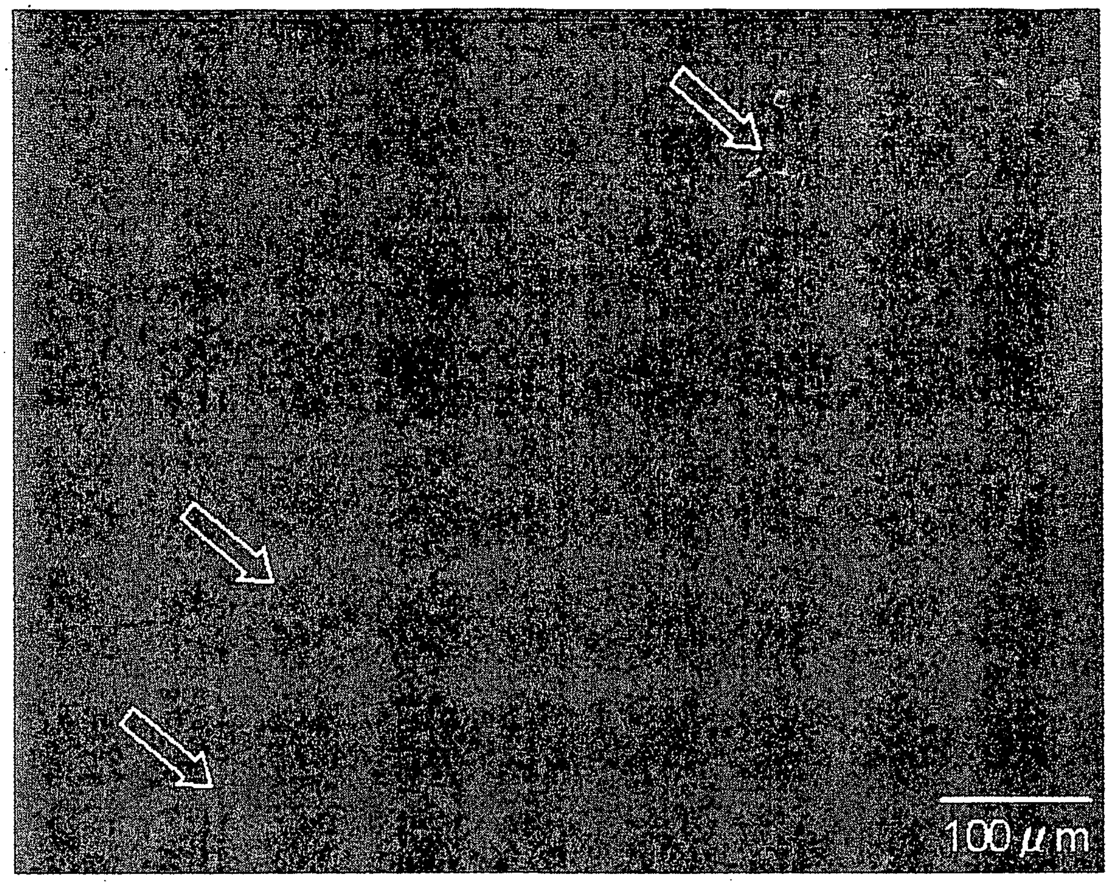


THK-766 Staining

**INTERNATIONAL SEARCH REPORT**

International application No.
PCT/JP2006/325804

A. CLASSIFICATION OF SUBJECT MATTER
*C07D277/84*(2006.01)i, *A61K31/4245*(2006.01)i, *A61K31/4439*(2006.01)i, *A61K31/5377*(2006.01)i, *A61K51/00*(2006.01)i, *A61P25/14*(2006.01)i, *A61P25/16*(2006.01)i, *A61P25/28*(2006.01)i, *C07D401/06*(2006.01)i,
According to International Patent Classification (IPC) or to both national classification and IPC

B. FIELDS SEARCHED
Minimum documentation searched (classification system followed by classification symbols)
C07D277/84, A61K31/4245, A61K31/4439, A61K31/5377, A61K51/00, A61P25/14, A61P25/16, A61P25/28, C07D401/06, C07D405/06, C07D413/04, C07D417/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAplus(STN), REGISTRY(STN), WPI(DIALOG)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2004-67659 A (BF Research Institute, Inc.), 04 March, 2004 (04.03.04), Full text; Claims 1 to 22; Par. No. [0005] (Family: none) | 1,6-13,16-19<br>10,17<br>2-5,20 |
| X<br>Y<br>A | WO 2004/035522 A1 (BF Research Institute, Inc.), 29 April, 2004 (29.04.04), Full text; Claims 1, 4, 5, 10 to 39; page 9, lines 38 to 39 & EP 1547996 A1 & US 2005/0260126 A1 & JP 2004-544906 A | 1,6-13,16-19<br>10,17<br>2-5,20 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 March, 2007 (08.03.07) | 20 March, 2007 (20.03.07) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2006/325804

C (Continuation). DOCUMENTS CONSIDERED TO BE RELEVANT

| Category* | Citation of document, with indication, where appropriate, of the relevant passages | Relevant to claim No. |
|---|---|---|
| X<br><br>A | WO 97/26919 A2 (WARNER-LAMBERT CO.), 31 July, 1997 (31.07.97), Example 6; page 20, line 10 to page 23, line 22; page 24, lines 4 to 13; page 25, lines 6 to 15; table 1<br>& US 6001331 A | 1,6-13,16, 17,19<br>2-5,18,20 |
| X<br><br>Y<br>A | SHIMADZU, H., et al., Novel probes for imaging amyloid-β: F-18 and C-11 labeling of 2-(4-aminostyryl)benzoxazole derivatives, J. Label Compd Radiopharm, 2004, 47, pp. 181-190 | 1,6-9,11-13, 16,18,19<br>10,17<br>2-5,20 |
| X<br><br>Y<br>A | OKAMURA, N., et al., Styrylbenzoxazole Derivatives for In Vivo Imaging of Amyloid Plaques in the Brain, The Journal of Neuroscience, March 10, 2004, 24(10), pp. 2535-2541 | 1,6-9,11-13, 16,18,19<br>10,17<br>2-5,20 |
| X<br><br>Y<br>A | OKAMURA, N., et al., Quinoline and Bezimidazole Derivatives:Candidate Probes for In Vivo Imaging of Tau Pathology in Alzheimer's Disease, The Journal of Neuroscience, November 23, 2005, 25(47), pp. 10857-10862 | 1,6-9,11-13, 16,18,19<br>10,17<br>2-5,20 |
| X<br>A | JP 6-194780 A (Konica Corp.), 15 July, 1994 (15.07.94), Compound I-32<br>& EP 608548 A2 & US 5374512 A | 1-3<br>4-13,16-20 |
| X<br>A | STETINOVA, J., et al., 2-(Benzoxazole-2-yl)-3-heteroarylprop-2-enenitrile, Chem. Papers, 1997, 51(6b), pp. 390-394, Compound IIc, IId | 1<br>2-13,16-20 |
| X<br>A | JP 2004-525800 A (Bayer AG.), 26 August, 2004 (26.08.04), Example 28<br>& WO 2002/080161 A2 & EP 1377976 A2 | 1<br>2-13,16-20 |
| X<br>A | JP 2004-534344 A (Bayer AG.), 11 November, 2004 (11.11.04), Example 52<br>& WO 2002/080150 A2 & EP 1377975 A2 | 1<br>2-13,16-20 |
| P,X<br>P,A | ISHIKAWA, K., et al., Styrylbenzoazole derivatives for imaging of prion plaques and treatment of transmissible spongiform encephalopathies, Journal of Neurochemistry, 2006, 99, pp. 198-205 | 1,6-13,16-19<br>2-5,20 |

Form PCT/ISA/210 (continuation of second sheet) (April 2005)

**INTERNATIONAL SEARCH REPORT**

International application No.
PCT/JP2006/325804

Continuation of A. CLASSIFICATION OF SUBJECT MATTER
(International Patent Classification (IPC))

*C07D405/06*(2006.01)i, *C07D413/04*(2006.01)i, *C07D417/06*(2006.01)i

(According to International Patent Classification (IPC) or to both national classification and IPC)

Continuation of Box No.II-1 of continuation of first sheet(2)

to search (PCT Article 17(2)(a)(i) and PCT Rule 39.1(iv)).

Form PCT/ISA/210 (extra sheet) (April 2005)

**INTERNATIONAL SEARCH REPORT**

International application No.
PCT/JP2006/325804

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 14, 15
because they relate to subject matter not required to be searched by this Authority, namely:
The inventions as set forth in claims 14 and 15 pertain to methods for treatment of the human body by therapy or diagnostic methods to be practiced on the human body and thus relate to a subject matter which this International Searching Authority is not required (continued to extra sheet)

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

INTERNATIONAL SEARCH REPORT

International application No.
PCT/JP2006/325804

<Subject of search>

Claims 1, 3, 6-13 and 16-19:

The inventions of claims 1, 3, 6-13 and 16-19 relate to a compound of the formula (I) below, a composition comprising the compound and the like.

[Chemical formula]

The compound defined by the formula (I) includes within its scope an extremely wide variety of compounds depending on the combinations of the substituents A, D, $R_1$ and $R_4$.

However, when the contents of the description are examined, it is only disclosed that, among the compounds of the formula (I), a compound which has a benzothiazole, benzimidazole, benzoxazole, or naphthol[1,2-d]thiazole ring having a specific substituent and wherein $R_1$'s independently represent a hydrogen, a methyl group, an alkoxy group having a substituent or together form a phenyl ring, $R^4$ represents an amino group or a nitrogenated heterocyclic ring, and n is 1 can bind to an amyloid-β protein.

Therefore, in the present state of the description, it cannot be considered that all of the inventions of claims 1, 3, 6-13 and 16-19 are disclosed in a manner sufficiently clear and complete for the inventions to be carried out by a person skilled in the art, and the inventions of these claims cannot be considered to be sufficiently supported (PCT Articles 5 and 6).

A prior art search was made mainly on the inventions of the claims stated above and the compounds supported by the description, i.e., compounds having the following substituents, within a reasonable scope of burden.

Regarding the inventions of claims 2, 4, 5 and 20, a complete search was made.

·A is N;
·D is S, N, O or $CH_2$;
·$R_1$'s independently represent a hydrogen, OH, an alkyl group which may have a substituent, an alkoxy group which may have a substituent, or may together form a carbon ring;
·$R_4$ represent $NR_5R_6$, wherein $R_5$ and $R_6$ independently represent a hydrogen or alkyl group or may together form a heterocyclic ring; and
·n is 1.

As a result of a search, it is found that the present invention includes within its scope a various known compounds and that the inventions of claims 1, 3, 6-13 and 16-19 are obviously unnovel as stated in page 2, Box C. Therefore, in the presentation of reference documents, mainly those documents which disclose the compounds specifically mentioned in the description are listed.

(continued to next sheet)

Form PCT/ISA/210 (extra sheet) (April 2005)

**INTERNATIONAL SEARCH REPORT**

International application No.
PCT/JP2006/325804

Claims 2, 4, 5-13 and 16-20

In claims 2, 4, 5 and 20, there is found symbols such as "THK-097". However, it is unclear what compounds are shown by the symbols, and therefore the inventions of these claims are unclear.

Similar comments apply to claims which depend on these claims.

In this international search report, a prior art search was made on the understanding that a symbol such as THK-097 means a compound having a structural formula shown in the right side of the symbol in each of Tables 1-1, 1-2, 1-3 and 1-4, with taking the contents of the description of the present application into consideration.

Claims 5-13 and 16-19

When a symbol "THK-683" in claim 5 is considered to mean a structural formula shown in the right side of the symbol in Table 1-1 as stated above, the compound is not included within the scope of the compound of the formula (I) in claim 1 of the present application. Therefore, claim 5, which depends on claim 1, lacks in consistency.

Similar comments apply to claims which depend on claim 5.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9605918 W
- US 9807889 W
- JP 2000080082 A
- JP 2000080083 A
- JP 2001076075 A
- JP 1002204 W
- JP 1002205 W
- JP 3007183 W
- JP 3015269 W
- JP 3015229 W
- JP 2004001546 W
- WO 03106439 A

### Non-patent literature cited in the description


- **PUCHTLER et al.** *Journal of Histochemistry and Cytochemistry,* 1962, vol. 10, 35
- **PUCHTLER et al.** *Journal of Histochemistry and Cytochemistry,* 1983, vol. 77, 431
- **LE VINE.** *Protein Science,* 1993, vol. 2, 404-410
- **YANKNER et al.** *Science,* 1989, vol. 245, 417-420
- **BRAAK H. ; BRAAK E.** *Acta Neuropathol.,* 1991, vol. 82, 239-259
- **WISCHIK et al.** Neurobiology of Alzheimer's Disease. Oxford University Press, 2001, 103-206
- **ISHIGURO et al.** *Neurosci. Lett.,* 1999, vol. 270, 81-84
- **ITOH et al.** *Ann. Neurol.,* 2001, vol. 50, 150-156
- **AGDEPPA et al.** *Journal of Neuroscience,* 2001, vol. 21, RC189
- **KUNG et al.** *Brain Research,* 2002, vol. 956, 202